# EUROPEAN PATENT APPLICATION

(11) **EP 4 471 144 A1**
(43) Date of publication of application: **04.12.2024**
(21) Application number: 23743004.6
(22) Date of filing: 23.01.2023
(51) Int. Cl.: C12N 15/113, A61K 31/713, A61K 48/00, A61P 3/06

(54) **COMPOSITION AND METHOD FOR INHIBITING EXPRESSION OF PROTEIN LPA(APO(A))**

(30) Priority: 24.01.2022 WO PCT/CN2022/073415
(71) Applicant: Shanghai Argo Biopharmaceutical Co., Ltd., Shanghai 201803 (CN)
(72) Inventor: SHU, Dongxu, Ningbo, Zhejiang 315100 (CN); SHAO, Pengcheng Patrick, Warrington Township Pennsylvania 018976 (US); XIA, Shiwei, Hangzhou, Zhejiang 311400 (CN)
(74) Representative: Elkington and Fife LLP
(86) International application number: PCT/CN2023/073456
(87) International publication number: WO 2023/138689

(57) **Abstract**

Provided in the present application are a composition and method useful for reducing the gene expression of LPA(Apo(a)) and for treating LPA-related diseases and conditions. Further provided are an LPA dsRNA agent, an LPA antisense polynucleotide agent, a composition comprising the LPA dsRNA agent and a composition comprising the LPA antisense polynucleotide agent, which are useful for reducing the expression of LPA in a cell and a subject.

## Description

### Technical Field

Some embodiments of the present invention relate to compositions and methods that can be used to inhibit LPA (Apo(a)) protein expression.

### Background Art

Lp(a) particles are heterogeneous low density lipoprotein particles expressed predominantly in the liver (Witztum and Ginsberg, J Lipid Res. March 2016; 57 (3): 336 - 9). They consist of Apolipoprotein (a) (Apo(a) or Lp(a) is encoded by the LPA gene) linked to LDL-like particles via the ApoB polypeptide. Genetically defined high Lp (a) particle serum levels are not affected by diet and exercise and are associated with an increased risk of developing cardiovascular disease through associated atherosclerotic potential (Alonso et al., Journal of the American College of Cardiology Vol. 63, No. 19, 2014). levels of Lp(a) particles in serum in patients are a highly prevalent independent genetic risk factor for coronary heart disease and aortic stenosis according to diagnostic and preventive medicine (Saeedi and Frohlich Clinical Diabetes and Endocrinology (2016) 2: 7). Analysis of Lp(a) levels in multiple studies suggests that a high Lp(a) level is an independent risk factor for cardiovascular disease, stroke, and other related conditions, including atherosclerotic stenosis. In addition, genome-wide association analyses have also identified LPA as a genetic risk factor for diseases such as atherosclerotic stenosis. A significant reduction in cardiovascular events is observed when both Lp(a) and LDL levels in hyperlipidemic patients are reduced using a therapeutic lipoprotein hematocatharsis. Therefore, therapeutic agents and treatments associated with these and other LPA-related diseases are needed.

However, other than indirect standard general LDL reduction measures, there are no currently approved specific Lp(a) particle reduction therapies. Therefore, there is currently a need for methods for effective treatment of, prevention of and reduction of the risk of suffering from the following and conditions related to the following: Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles and other related conditions, pathologies or syndromes that have not yet been identified. The present invention addresses this unmet medical need.

### Summary of the Invention

According to an aspect of the present invention, a double-stranded ribonucleic acid (dsRNA) agent that inhibits LPA (Apo(a)) expression is provided, the dsRNA agent comprises a sense strand and an antisense strand, and optionally comprises a targeting ligand. A region complementary to the LPA RNA transcript is included at nucleotide positions 2 to 18 in the antisense strand, wherein the complementary region comprises at least 15 continuous nucleotides that differ by 0, 1, 2 or 3 nucleotides from one of the antisense sequences listed in Tables 1-3. In some embodiments, the region complementing the LPARNA transcript comprises at least 15, 16, 17, 18 or 19 continuous nucleotides that differ by no more than 3 nucleotides from one of the antisense sequences listed in Tables 1-3. In certain embodiments, the antisense strand of the dsRNA is at least substantially complementary to any target region of the mRNA of the human LPA gene and is provided in one of Tables 1-3. In some embodiments the antisense strand of the dsRNA is completely complementary to any target region of the mRNA of the human LPA gene and is provided in one of Tables 1-3. In some embodiments, the dsRNA agent comprises any of the sense strand sequences listed in Tables 1-3, wherein the sense strand sequences are at least substantially complementary to the antisense strand sequences in the dsRNA agent. In certain embodiments, the dsRNA agent comprises any of the sense strand sequences listed in Tables 1-3, wherein the sense strand sequences are completely complementary to the antisense strand sequences in the dsRNA agent. In some embodiments, the dsRNA agent comprises any of the antisense strand sequences listed in Tables 1-3. In some embodiments, the dsRNA agent comprises any of the sequences listed as duplex sequences in Tables 1-3. In some embodiments, the dsRNA agent comprises a sense strand that differs from formula (A) by 0, 1, 2 or 3 nucleotides: 5'-Z₁GUUAUCGAGGCACAUAZ₂-3' Formula (A), wherein Z₁ is a nucleotide sequence comprising 0-15 nucleotide motifs, and Z₂ is selected from one of A, U, C, and G or absent. In certain embodiments, Z₂ is A. In some embodiments, Z₁ nucleotide sequence is selected from one of the following motifs: A, AA, UA, GA, CA, AGA, UGA, GGA, CGA, UAGA, CAGA, AAGA, ACAGA, GACAGA, GGACAGA, UGGACAGA, AUGGACAGA, AAUGGACAGA, UAAUGGACAGA, GUAAUGGACAGA, GGUAAUGGACAGA, UGGUAAUGGACAGA, AND AUGGUAAUGGACAGA or absent. In some embodiments, Z₁ is a nucleotide sequence comprising 1, 2, 3 or 4 nucleotide motifs selected from the following motifs: A, AA, UA, GA, CA, AGA, UGA, GGA, CGA, UAGA, CAGA, AAGA, AND ACAGA. In some embodiments, the dsRNA agent comprises an antisense strand that differs from formula (B) by 0, 1, 2, or 3 nucleotides: 5'-Z₃UAUGUGCCUCGAUAACZ₄-3' Formula (B), where Z₃ is selected from one of A, U, C, and G or absent, Z₄ is a nucleotide sequence comprising 0-15 nucleotide motifs. In certain embodiments, Z₃ is U. In some embodiments, Z₄ nucleotide sequence is selected from the following motifs: U, UU, UA, UC, UG, UCU, UCA, UCC, UCG, UCUC, UCUA, UCUG, UCUU, UCUGU, UCUGUC, UCUCUU, UCUCGA, UCUGUCC, UCUGUCCA, UCUGUCCAU, UCUGUCCAU, UCUGUCCAUU, UCUGUCCAUUA, UCUGUCCAUUAC, UCUGUCCAUUACC, UCUGUCCAUUACCA AND UCUGUCCAUUACCAU or absent. In some embodiments, Z₄ is a nucleotide sequence comprising 1, 2, 3 or 4 nucleotide motifs selected from the following motifs: U, UU, UA, UC, UG, UCU, UCA, UCC, UCG, UCUC, UCUA, UCUG AND UCUU. In some embodiments, the dsRNA agent comprises a sense strand and an antisense strand comprising a nucleotide sequence described herein that differs from Formula (A) and Formula (B) by 0, 1, 2 or 3 nucleotide, respectively, and optionally comprises a targeting ligand. In certain embodiments, the length of each of the sense strand (A) and the antisense strand (B) of the dsRNA agent does not exceed 35 nucleotides. In certain embodiments, Z₁ and Z₄ nucleotide motifs are completely or partially complementary. In certain embodiments, Z₂ and Z₃ nucleotide motifs are completely or partially complementary. In certain embodiments, the sense strand is complementary or substantially complementary to the antisense strand, and the length of the complementary region is between 16 and 23 nucleotides. In some embodiments, the complementary region is 19-21 nucleotides in length. In some embodiments, wherein the length of the sense strand does not exceed 35 nucleotides, including a region complementary to the antisense strand that includes at least 15, 16, 17, 18, or 19 nucleotides. In some embodiments, the dsRNA agent comprises a sense strand that differs from formula (C) by 0, 1, 2 or 3 nucleotides: 5'-Z₅CCAAGCUUGGUCAUCUZ₆-3' Formula (C), wherein Z₅ is a nucleotide sequence comprising 0-15 nucleotide motifs, and Z₆ is selected from one of A, U, C, and G or absent. In certain embodiments, Z₆ is A. In some embodiments, Z₅ nucleotide sequence is selected from one of the following motifs: G, AG, UG, GG, CG, AUG, UUG, GUG, CUG, UUUG, CUUG, AUUG, ACUUG, AACUUG, GAACUUG, AGAACUUG, AAGAACUUG, GAAGAACUUG, GGAAGAACUUG, AGGAAGAACUUG, CAGGAAGAACUUG, ACAGGAAGAACUUG AND CACAGGAAGAACUUG or absent. In some embodiments, Z₅ is a nucleotide sequence comprising 1, 2, 3 or 4 nucleotide motifs selected from the following motifs: G, AG, UG, GG, CG, AUG, UUG, GUG, CUG, UUUG, CUUG AND AUUG. In some embodiments, the dsRNA agent comprises an antisense strand that differs from formula (D) by 0, 1, 2, or 3 nucleotides: 5'-Z₇AGAUGACCAAGCUUGGZ₈-3' Formula (D), where Z₇ is selected from one of A, U, C, and G or absent, Z₈ is a nucleotide sequence comprising 0-15 nucleotide motifs. In certain embodiments, Z₇ is U. In some embodiments, Z₈ nucleotide sequence is selected from the following motifs: C, CU, CA, CC, CG, CAU, CAA, CAC, CAG, CAAC, CAAA, CAAG, CAAU, CAAGU, CAAGUU, CAACUU, CAACGA, CAAGUUC, CAAGUUCU, CAAGUUCUU, CAAGUUCUUC, CAAGUUCUUCC, CAAGUUCUUCCU, CAAGUUCUUCCUG, CAAGUUCUUCCUGU AND CAAGUUCUUCCUGUG or absent. In some embodiments, Z₈ is a nucleotide sequence comprising 1, 2, 3 or 4 nucleotide motifs selected from the following motifs: C, CU, CA, CC, CG, CAU, CAA, CAC, CAG, CAAC, CAAA, CAAG AND CAAU. In some embodiments, the dsRNA agent comprises a sense strand and an antisense strand comprising a nucleotide sequence described herein that differs from Formula (C) and Formula (D) by 0, 1, 2 or 3 nucleotide, respectively, and optionally comprises a targeting ligand. In certain embodiments, the length of each of the sense strand (C) and the antisense strand (D) of the dsRNA agent does not exceed 35 nucleotides. In certain embodiments, Z₅ and Z₈ nucleotide motifs are completely or partially complementary. In certain embodiments, Z₆ and Z₇ nucleotide motifs are completely or partially complementary. In certain embodiments, the sense strand is complementary or substantially complementary to the antisense strand, and the length of the complementary region is between 16 and 23 nucleotides. In some embodiments, the complementary region is 19-21 nucleotides in length. In some embodiments, wherein the length of the sense strand does not exceed 35 nucleotides, including a region complementary to the antisense strand that includes at least 15, 16, 17, 18, or 19 nucleotides. In some embodiments, the dsRNA agent comprises a sense strand that differs from formula (E) by 0, 1, 2 or 3 nucleotides: 5'-Z₉GACAGAGUUAUCGAGGZ₁₀-3' Formula (E), where Z₉ is a nucleotide sequence comprising 0-15 nucleotide motifs, and Z₁₀ is selected from one of A, U, C, and G or absent. In certain embodiments, Z₁₀ is A. In some embodiments, Z₉ nucleotide sequence is selected from one of the following motifs: G, AG, UG, GG, CG, AUG, UUG, GUG, CUG, CAUG, UAUG, GAUG, AAUG, UGAUG, GUGAUG, GGUGAUG, UGGUGAUG, AUGGUGAUG, CAUGGUGAUG, CCAUGGUGAUG, ACCAUGGUGAUG, UACCAUGGUGAUG, CUACCAUGGUGAUG AND GCUACCAUGGUGAUG or absent. In some embodiments, Z₉ is a nucleotide sequence comprising 1, 2, 3 or 4 nucleotide motifs selected from the following motifs: G, AG, UG, GG, CG, AUG, UUG, GUG, CUG, CAUG, UAUG, GAUG AND AAUG. In some embodiments, the dsRNA agent comprises an antisense strand that differs from formula (F) by 0, 1, 2, or 3 nucleotides: 5'-Z₁₁CCUCGAUAACUCUGUCZ₁₂-3' Formula (F), where Z₁₁ is selected from one of A, U, C, and G or absent, Z₁₂ is a nucleotide sequence comprising 0-15 nucleotide motifs. In certain embodiments, Z₁₁ is U. In some embodiments, Z₁₂ nucleotide sequence is selected from the following motifs: C, CU, CA, CC, CG, CAU, CAA, CAC, CAG, CAUA, CAUG, CAUC, CAUU, CAUCA, CAUCAC, CAUGUU, CAUGGA, CAUCACC, CAUCACCA, CAUCACCAU, CAUCACCAUG, CAUCACCAUGG, CAUCACCAUGGU, CAUCACCAUGGUA, CAUCACCAUGGUAG AND CAUCACCAUGGUAGC or absent. In some embodiments, Z₁₂ is a nucleotide sequence comprising 1, 2, 3 or 4 nucleotide motifs selected from the following motifs: C, CU, CA, CC, CG, CAU, CAA, CAC, CAG, CAUA, CAUG, CAUC AND CAUU. In some embodiments, the dsRNA agent comprises a sense strand and an antisense strand comprising a nucleotide sequence described herein that differs from Formula (E) and Formula (F) by 0, 1, 2 or 3 nucleotide, respectively, and optionally comprises a targeting ligand. In certain embodiments, the length of each of the sense strand (F) and the antisense strand (F) of the dsRNA agent does not exceed 35 nucleotides. In certain embodiments, Z₉ and Z₁₂ nucleotide motifs are completely or partially complementary. In certain embodiments, Z₁₀ and Z₁₁ nucleotide motifs are completely or partially complementary. In certain embodiments, the sense strand is complementary or substantially complementary to the antisense strand, and the length of the complementary region is between 16 and 23 nucleotides. In some embodiments, the complementary region is 19-21 nucleotides in length. In some embodiments, wherein the length of the sense strand does not exceed 35 nucleotides, including a region complementary to the antisense strand that includes at least 15, 16, 17, 18, or 19 nucleotides.

In some embodiments, the dsRNA agent comprises at least one modified nucleotide. In certain embodiments, all or substantially all nucleotides of the antisense strand are modified nucleotides. In some embodiments, at least one modified nucleotide includes: a 2'-O-methyl nucleotide, a 2'-fluoro nucleotide, a 2'-deoxynucleotide, a 2',3'-seco nucleotide mimic, a locked nucleotide, an unlocked nucleic acid (UNA) nucleotide, a glycol nucleic acid nucleotide (GNA), a 2'-F-arabinose nucleotide, a 2'-methoxyethyl nucleotide, an abasic nucleotide, a ribitol, a reverse nucleotide, a reverse abasic nucleotide, a reverse 2'-OMe nucleotide, a reverse 2'-deoxynucleotide, a 2'-amino modified nucleotide, a 2'-alkyl modified nucleotide, a morpholino nucleotide and a 3'-OMe nucleotide, a nucleotide including 5'-phosphorothioate group, or a terminal nucleotide linked to a cholesterol derivative or a dodecanoic acid bisdecylamide group, a 2'-amino modified nucleotide, a phosphoramidate, or a non-natural base comprising nucleotide. In some embodiments, the antisense strand comprises 15 or more modified nucleotides independently selected from 2'-O-methyl nucleotides and 2'-fluoro nucleotides, wherein there are less than six 2'-fluoro nucleotide modified nucleotides. In some embodiments, the antisense strand comprises three or five 2'-fluoro nucleotides, preferably, the antisense strand comprises five 2'-fluoro nucleotides. In some embodiments, the sense strand comprises 15 or more modified nucleotides independently selected from 2'-O-methyl nucleotides and 2'-fluoro nucleotides, wherein there are less than four 2'-fluoro nucleotide modified nucleotides. In certain embodiments, the sense strand comprises three 2'-fluoro nucleotides. In some embodiments, the antisense strand comprises 15 or more modified nucleotides independently selected from 2'-O-methyl nucleotides and 2'-fluoro nucleotides, wherein at least 16 modified nucleotides are 2'-O-methyl nucleotides and positions 2, 7, 12, 14 and/or 16 at the 5'-end of the antisense strand are 2'-fluoro nucleotide modified nucleotides (counting from the first paired nucleotide at the 5'-end of the antisense strand). In some embodiments, the sense strand comprises 15 or more modified nucleotides independently selected from 2'-O-methyl nucleotides and 2'-fluoro nucleotides, wherein at least 18 modified nucleotides are 2'-O-methyl nucleotides and positions 9, 11 and/or 13 at the 3'-end of the sense strand are 2 '-fluoro nucleotide modified nucleotides (counting from the first paired nucleotide of the 3'-end of the sense strand). In some embodiments, the antisense strand includes 2'-fluoro modified nucleotides at positions 2, 7, 12, 14 and 16 of the antisense strand in the direction from the 5'-end to the 3'-end; counting from the first paired nucleotide at the 5'-end of the antisense strand, the nucleotides at other positions in each antisense strand are independently non-fluoro modified nucleotides. In some embodiments, the antisense strand comprises 2'-fluoro modified nucleotides at positions 2, 5, 12, 14 and 18 of the antisense strand in the direction from the 5'-end to the 3'-end, counting from the first paired nucleotide at the 5'-end of the antisense strand, and each nucleotide at the other position in the antisense strand is independently a non-fluoro modified nucleotide. In some embodiments, the sense strand comprises 2'-fluoro modified nucleotides at inucleotide positions 9, 11 and 13 of the sense strand in the direction from the 3'-end to the 5'-end, counting from the first paired nucleotide at the 3'-end of the sense strand, and each nucleotide at the other position in the sense strand is independently a non-fluoro modified nucleotide. In some embodiments, the dsRNA agent comprises an E-vinyl phosphonate nucleotide at the 5'-end of the guide strand. In certain embodiments, the dsRNA agent comprises at least one phosphorothioate internucleoside linkage. In certain embodiments, the sense strand comprises at least one phosphorothioate internucleoside linkage. In some embodiments, the antisense strand comprises at least one phosphorothioate internucleoside linkage. In some embodiments, the sense strand comprises 1, 2, 3, 4, 5, or 6 phosphorothioate internucleoside linkages. In some embodiments, the antisense strand comprises 1, 2, 3, 4, 5, or 6 phosphorothioate internucleoside linkages. In certain embodiments, all or substantially all nucleotides of the sense and antisense strands are modified nucleotides. In some embodiments, the modified sense strand is the modified sense strand sequence listed in Tables 2-3. In some embodiments, the modified antisense strand is the modified antisense strand sequence listed in Tables 2-3. In certain embodiments, the sense strand is complementary or substantially complementary to the antisense strand, and the length of the complementary region is between 16 and 23 nucleotides. In some embodiments, the complementary region is 19-21 nucleotides in length. In some embodiments, the complementary region is 14, 15, 16, 17, 18, 19, 20, 21, 22, 23, 24, 25, 26, 27, 28, 29, or 30 nucleotides in length. In some embodiments, each strand is no longer than 40 nucleotides in length. In some embodiments, each strand is no longer than 30 nucleotides in length. In some embodiments, each strand is no longer than 25 nucleotides in length. In some embodiments, each strand is no longer than 23 nucleotides in length. In certain embodiments, the dsRNA agent comprises at least one modified nucleotide and further comprises one or more targeting groups or linking groups. In some embodiments, one or more targeting groups or linking groups are conjugated to the sense strand. In some embodiments, the targeting groups or linking groups comprise N-acetylgalactosamine (GalNAc). In some embodiments, the targeting moieties of the targeting groups have the following structural fragments, 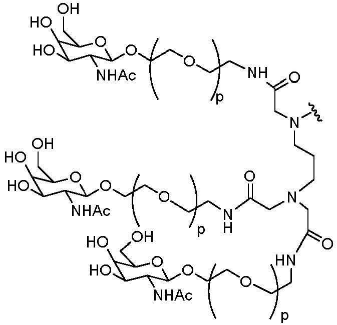 p is 1 or 2.

In some embodiments, the targeting groups have the following structures:

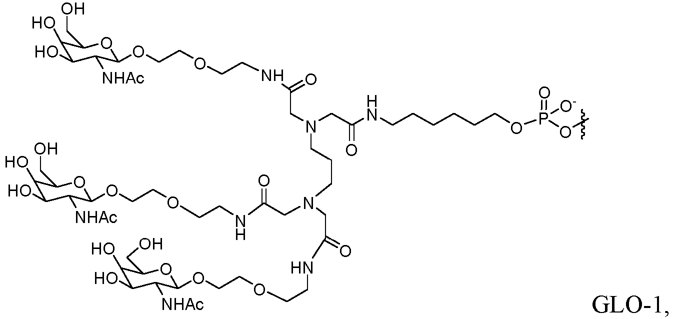

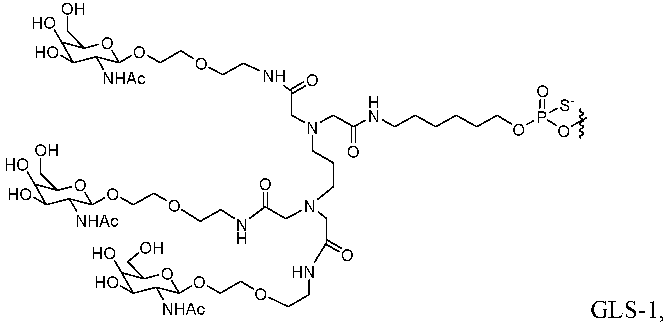

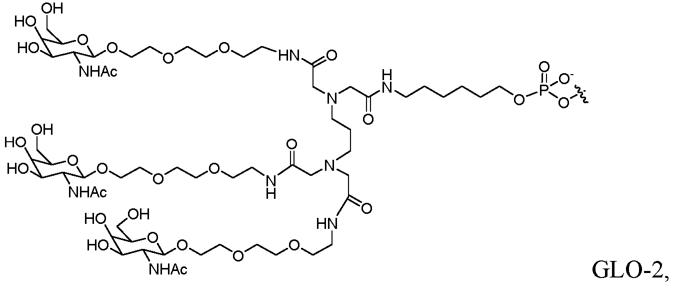

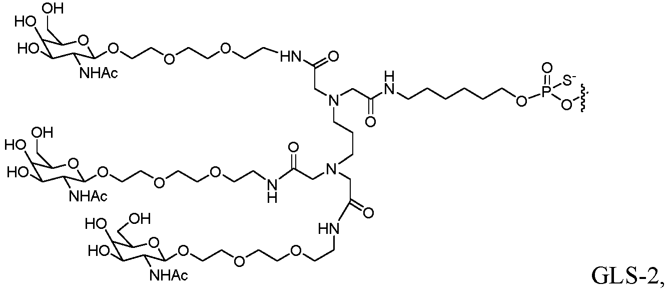

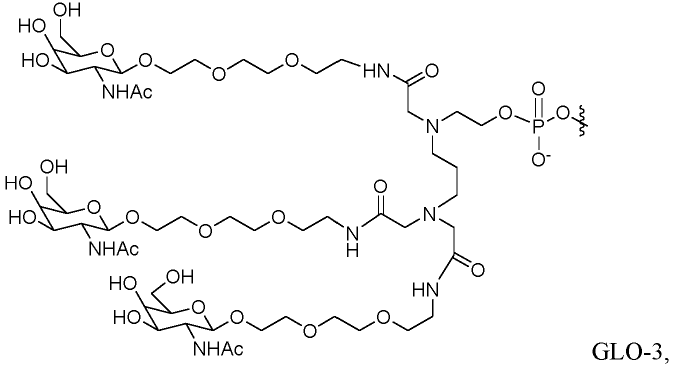

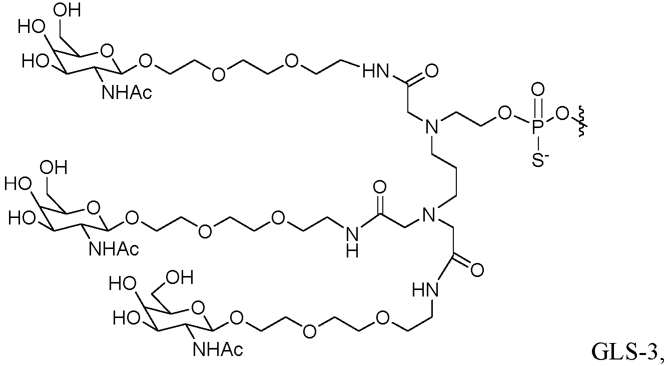

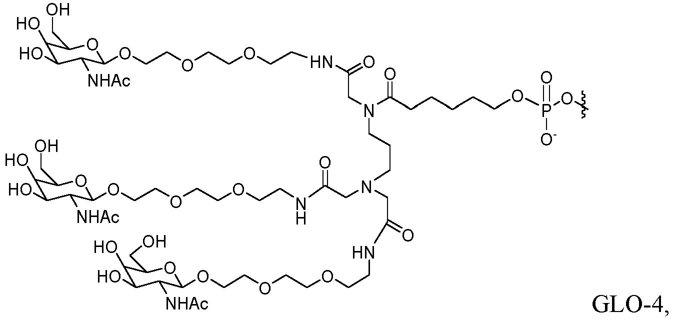

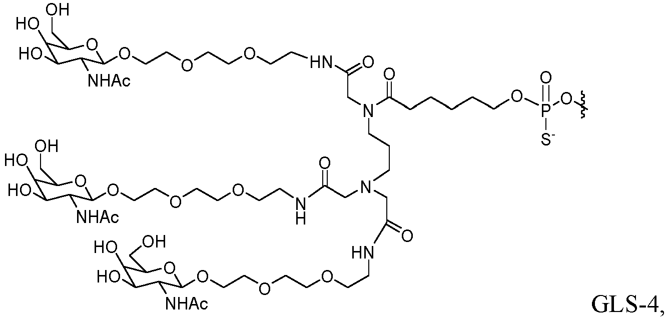

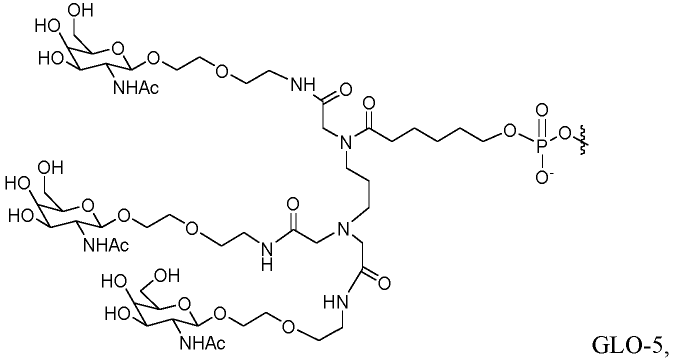

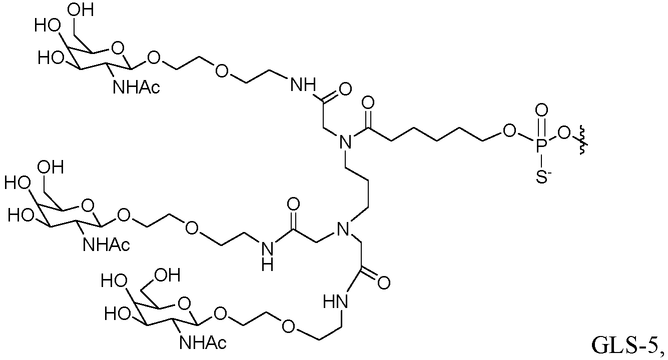

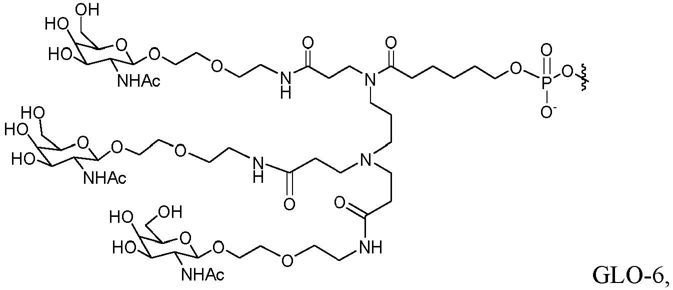

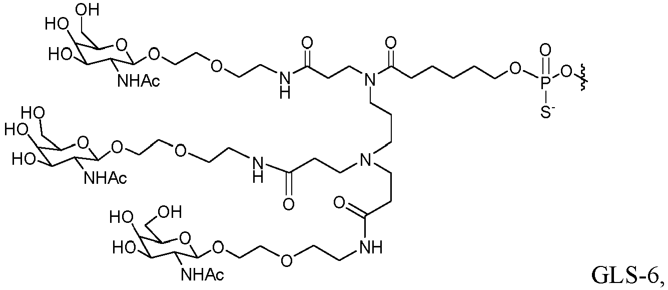

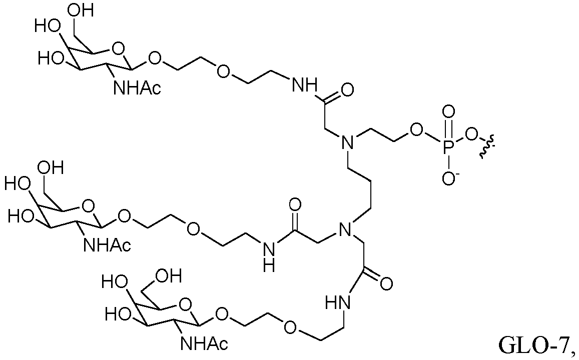

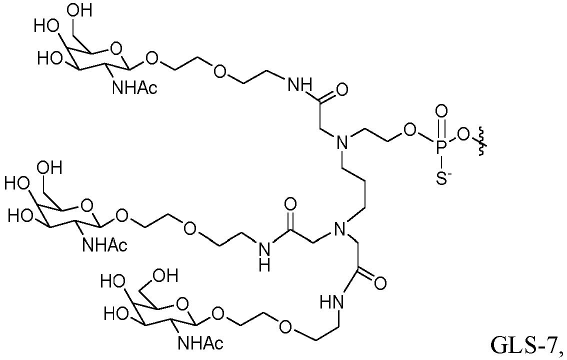

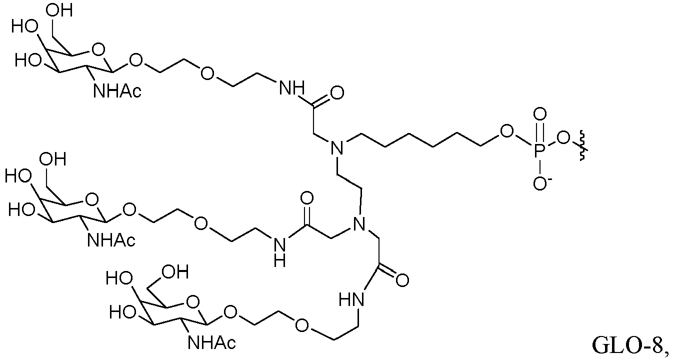

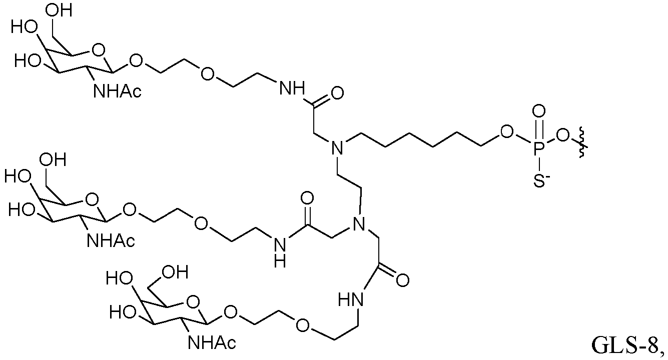

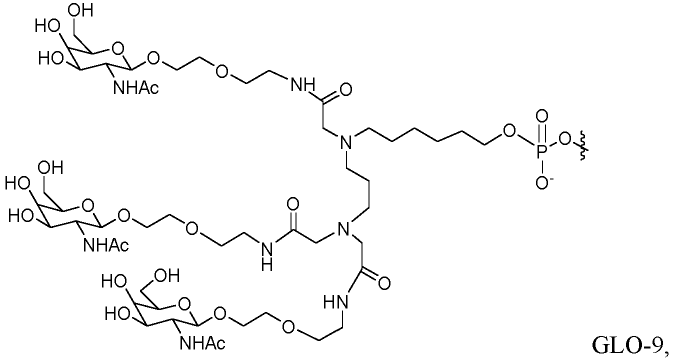

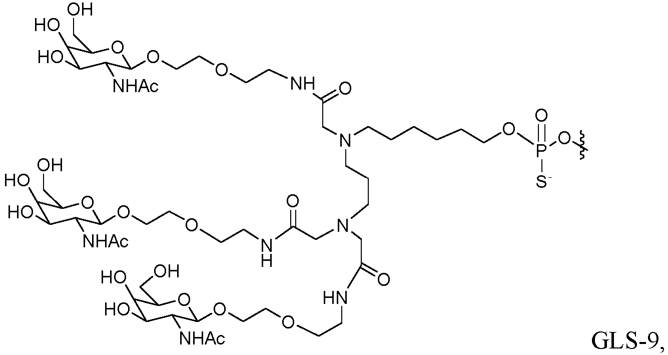

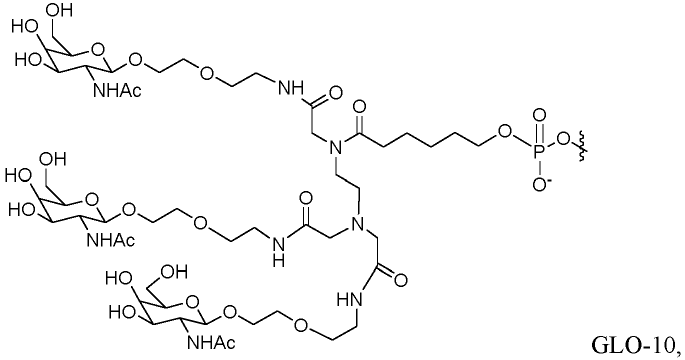

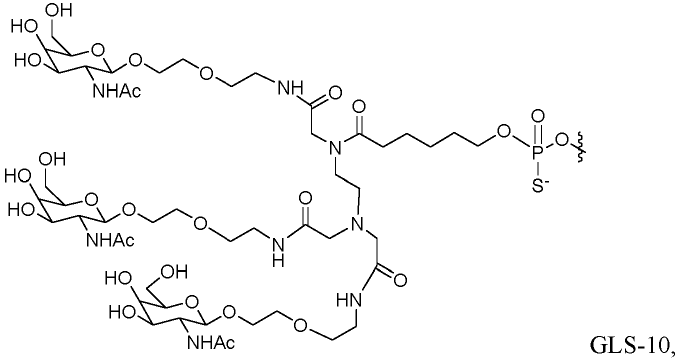

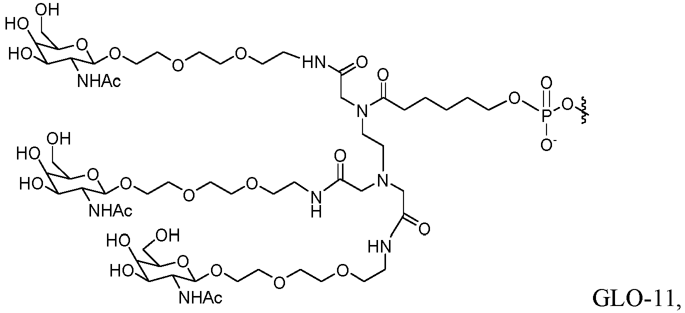

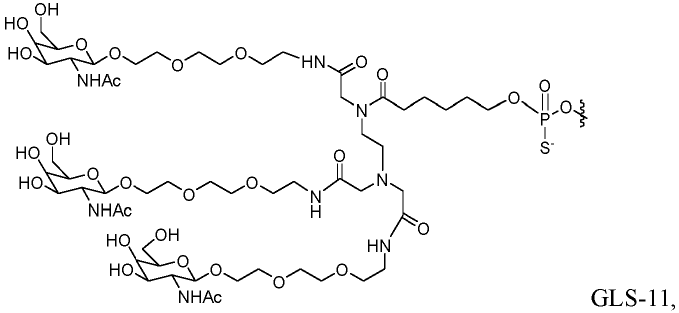

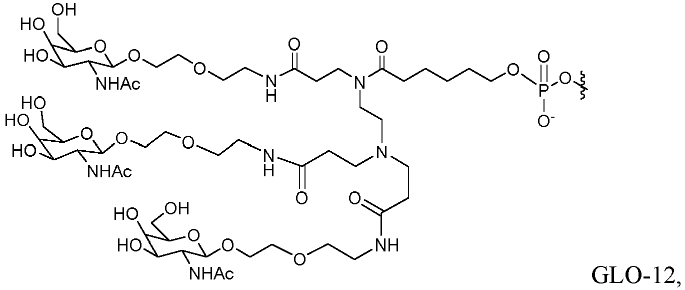

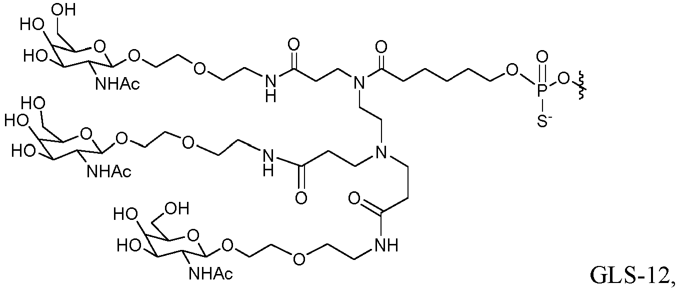

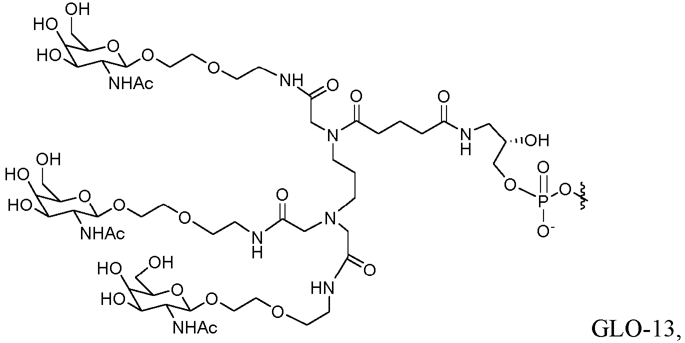

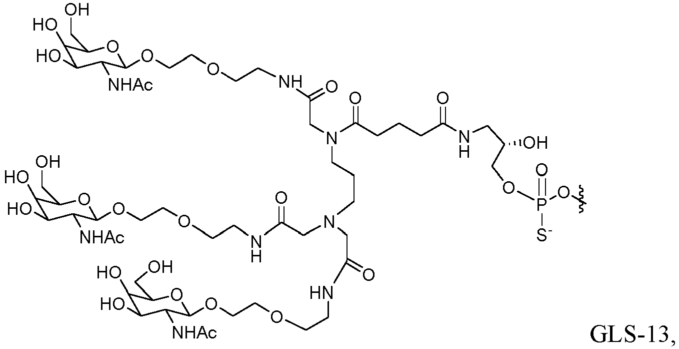

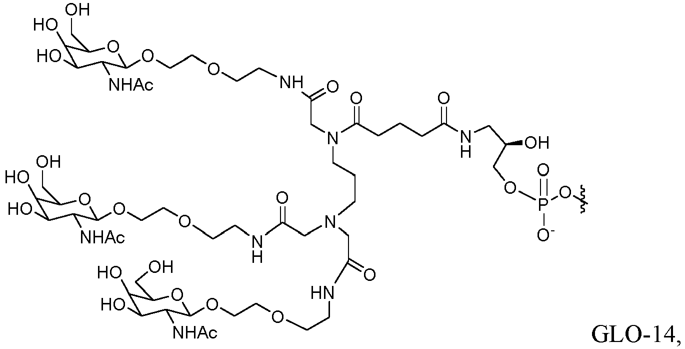

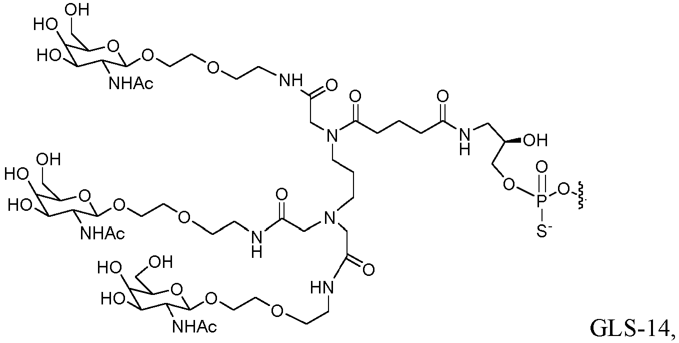

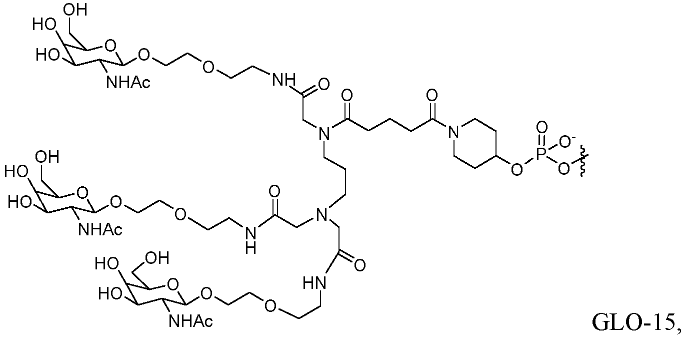

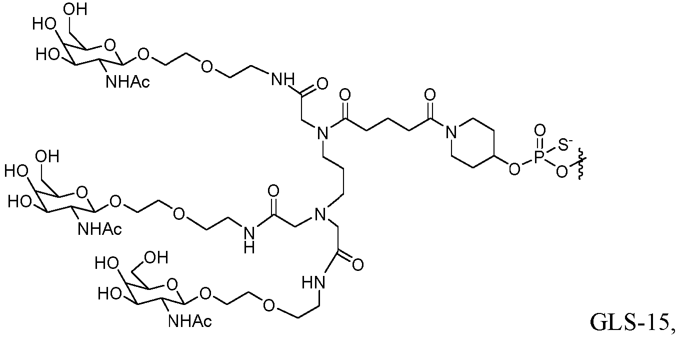

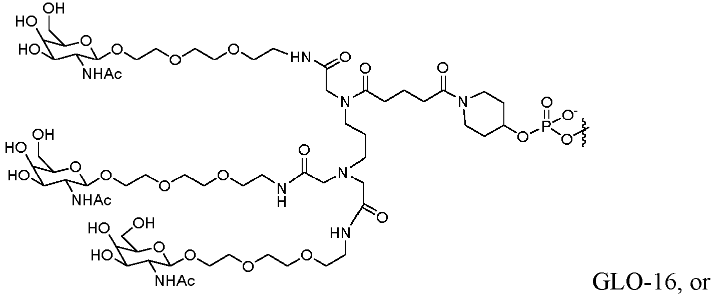

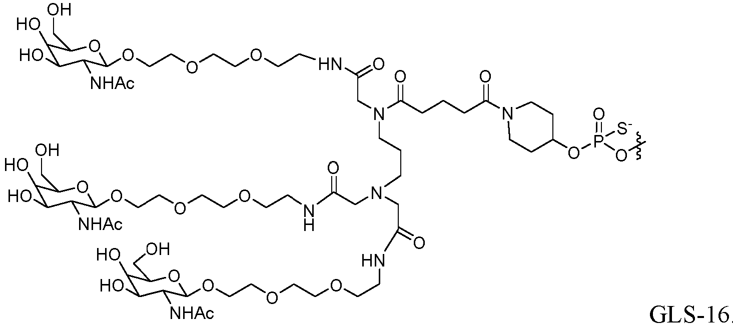

In certain embodiments, the dsRNA agent comprises a targeting group conjugated to the 5'-end of the sense strand. In some embodiments, the dsRNA agent comprises a targeting group conjugated to the 3'-end of the sense strand. In some embodiments, the antisense strand comprises one reverse abasic residue at the 3'-end. In certain embodiments, the sense strand comprises one or two reverse abasic residues at the 3'-end or/and 5'-end. In certain embodiments, the sense strand comprises one or two isomannitol residues at the 3'-end or/and 5'-end. In certain embodiments, the sense strand independently comprises one isomannitol residue at the 3'-end and 5'-end, respectively. In some embodiments, the sense chain independently comprises one isomannitol residue at the 3'-end and the 5'-end respectively, and further comprises a 5'-end conjugated targeting group, preferably GLS-15 as described above. In some embodiments, the dsRNA agent has two blunt ends. In some embodiments, at least one strand comprises a 3' overhang having at least 1 nucleotide. In some embodiments, at least one strand comprises a 3' overhang having at least 2 nucleotide.

In certain embodiments, a double-stranded ribonucleic acid (dsRNA) agent for inhibiting LPA (Apo (a)) expression comprises a sense strand and an antisense strand, and the nucleotide positions 2 to 18 in the antisense strand comprise a region complementary to an LPA RNA transcript, the antisense strand is fully or partially complementary to the sense strand, and the agent optionally comprises a targeting ligand, wherein each strand is 14 to 30 nucleotides in length, wherein the sense strand sequence may be represented by formula (I):
5'-(N'_{L})_{n'} N'_{L}N'_{L}N'_{L}N'_{N1} N'_{N2} N'_{F}N'_{L}N'_{F}N'_{L}N'_{N3} N'_{N4}N'_{L} N'_{L}N'_{L}N'_{L}N'_{L}N'_{L}(N'_{L})_{m'}-3' (I) where: each N'_{F} represents a 2'-fluoro modified nucleotide; each of N'_{N1}, N'_{N2}, N'_{N3} and N'_{N4} independently represents a modified or unmodified nucleotide; each N'_{L} independently represents a modified or unmodified nucleotide but not a 2'-fluoro modified nucleotide, n' is an integer of 0-7 and m' is an integer of 0-3. In some embodiments, each N'_{N3} represents a 2'-fluoro modified nucleotide, N'_{N1}, N'_{N2} and N'_{N4} independently represent a modified or unmodified nucleotide but does not represent 2'-fluoro modified nucleotides, and m is 1. In some embodiments, each N'_{N4} represents a 2'-fluoro modified nucleotide, N'_{N1}, N'_{N2} and N'_{N3} independently represent a modified or unmodified nucleotide but does not represent 2'-fluoro modified nucleotides, and m is 1. In some embodiments, n' is 3 and m' is 1; or n' is 0 and m' is 0; or n' is 3 and m' is 3. In certain embodiments, there are only three 2'-fluoro modified nucleotides in formula (I).

In certain embodiments, the present invention relates to an unlocked nucleic acid (UNA) oligomer for therapeutic use. An unlocked nucleic acid (UNA) is an acyclic analog of RNA in which the bond between the C2' and C3' atoms of the ribose ring has been broken. It has been demonstrated that incorporation of UNA is well tolerated for the siRNA gene silencing activity and even enhances the siRNA gene silencing activity in some cases (Meghan A. et al. "Locked vs. unlocked nucleic acids (LNA vs. UNA): contrasting structures work towards common therapeutic goals". Chem. Soc. Rev., 2011, 40, 5680-5689*).*

UNA is a thermally labile modification, and substitution of ribonucleotides with UNA reduces base pairing strength and duplex stability. Strategically placing UNA in the seed region of the siRNA antisense strand reduces off-target activity in the mechanism of gene silencing mediated by microRNA (miRNA). MiRNAs identify target genes primarily by base pairing between the antisense seed region (positions 2-8 starting from the 5'-end) and the target mRNA for gene suppression. Each miRNA potentially regulates a large number of genes. The siRNA antisense strands loaded by RNA-induced silencing complexes (RISCs) can also potentially regulate a large number of unintended genes through miRNA-mediated mechanisms. Thus, incorporation of thermally labile nucleotides, such as UNA, in the seed region of siRNA can reduce off-target activity (Lam JK, Chow MY, Zhang Y, Leung SW. siRNA Versus miRNA as Therapeutics for Gene Silencing. Mol Ther Nucleic Acids. 2015 Sep 15;4(9):e252. doi: 10.1038/mtna.2015.23. PMID: 26372022; PMCID: PMC4877448.). Specifically, such RNA oligonucleotides or complexes of RNA oligonucleotides comprise at least one UNA nucleotide monomer in the seed region (Narendra Vaish et al. "Improved specificity of gene silencing by siRNAs containing unlocked nucleobase analog". Nucleic Acids Research, 2011, Vol. 39, No. 5 1823-1832)*.*

According to the present technical solution, potential advantages of incorporating UNA in RNA oligonucleotides or complexes of RNA oligonucleotides include, but are not limited to:
1. The off-target activity was reduced. The addition of UNA in the siRNA seed region reduces the base pairing strength in the seed region, thereby reducing the potential off-target activity caused by the micro-RNA mechanism.
2. UNA was well tolerated in terms of siRNA activity. In some cases, UNA can lead to enhanced activity.

Exemplary UNA monomers that may be used in the present technical solution include, but are not limited to:

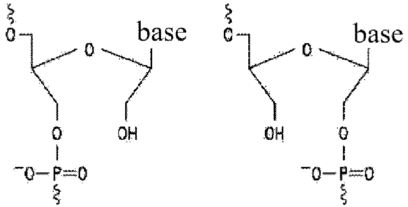

According to one aspect of the present invention, a composition is provided comprising any embodiment of the dsRNA agent described above of the present invention. In certain embodiments, the composition further comprises a pharmaceutically acceptable carrier. In some embodiments, the composition further comprises one or more additional therapeutic agents, such as a HMg Co-A reductase inhibitor (statins), ezetimibe, a PCSK-9 inhibitor, a CTEP inhibitor, an ANGPTL3-targeting therapy, an AGT-targeting therapy, an APOC3-targeting therapy and niacin, or any combination thereof. In some embodiments, the composition is packaged in a kit, container, packaging, dispenser, pre-filled syringe, or vial. In some embodiments, compositions are formulated for subcutaneous administration or are formulated for intravenous (IV) administration.

According to another aspect of the present invention, a cell is provided that comprises any embodiment of the dsRNA agent described above of the present invention. In some embodiments, the cell is a mammalian cell and optionally a human cell.

According to another aspect of the present invention, a method for inhibiting LPA gene expression in a cell is provided, the method comprises: (i) preparing a cell containing any embodiment of an effective amount of the dsRNA agent described above or the composition described above of the present invention. In certain embodiments, the method further comprises: (ii) maintaining the prepared cell for sufficient time to obtain degradation of the mRNA transcript of the LPA gene, thereby inhibiting the expression of the LPA gene in the cell. In some embodiments, the cell is in the subject and the dsRNA agent is administered subcutaneously to the subject. In some embodiments, the cell is in the subject and the dsRNA agent is administered to the subject via IV administration. In certain embodiments, the method further comprises evaluating the inhibitory effect on the LPA gene after administration of a dsRNA agent to a subject, wherein the means for evaluation includes: (i) determining one or more physiological characteristics of an LPA-related disease or condition in the subject, and (ii) comparing the identified physiological characteristics with baseline physiological characteristics of the LPA-related disease or condition before treatment and/or control physiological characteristics of the LPA-related disease or condition, wherein the comparison results indicate the presence or absence of inhibition of LPA gene expression in the subject. In some embodiments, the identified physiological characteristic is the Lp(a) level in the blood. A decrease in LPA level in blood indicates a decrease in LPA gene expression in the subject.

According to another aspect of the present invention, a method of inhibiting LPA gene expression in a subject is provided, the method comprises administrating an effective amount of embodiments of the dsRNA agent described above or embodiments of the composition described above to the subject. In some embodiments, the dsRNA agent is subcutaneously administered to the subject. In certain embodiments, the dsRNA agent is administered to the subject via IV administration. In some embodiments, the method further comprises evaluating the inhibitory effect on the LPA gene after administration of a dsRNA agent, wherein the means for evaluation includes: (i) determining one or more physiological characteristics of an LPA-related disease or condition in the subject, and (ii) comparing the identified physiological characteristics with baseline physiological characteristics of the LPA-related disease or condition before treatment and/or control physiological characteristics of the LPA-related disease or condition, wherein the comparison results indicate the presence or absence of inhibition of LPA gene expression in the subject. In some embodiments, the identified physiological characteristic is the Lp(a) level in the blood. A decrease in LPA level in blood indicates a decrease in LPA gene expression in the subject.

According to another aspect of the present invention, provided is a method of treating a disease or condition associated with an LPA protein comprising administering to a subject an effective amount of any embodiment of the aforementioned dsRNA agent of the present invention or any embodiment of the aforementioned composition of the present invention to inhibit LPA gene expression. In certain embodiments, the LPA-related disorder is a cardiovascular disease, wherein the cardiovascular disease includes Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles. In some embodiments, the method further comprises administering an additional therapeutic regimen to the subject. In some embodiments, the additional therapeutic regimen comprises treatment of LPA-related diseases or conditions In some embodiments, additional therapeutic regimens include: administering one or more LPA antisense polynucleotides of the present invention to a subject; administering a non-LPA dsRNA therapeutic agent to a subject; and effecting a behavioral modifications in a subject. In some embodiments, the non-LPA dsRNA therapeutic agent is one of additional therapeutic agents such as a HMg Co-A reductase inhibitor (statins), ezetimibe, a PCSK-9 inhibitor, a CTEP inhibitor, an ANGPTL3-targeting therapy, an APOC3-targeting therapy and niacin, or any combination thereof.

In some embodiments, the dsRNA agent is subcutaneously administered to the subject. In certain embodiments, the dsRNA agent is administered to the subject via IV administration. In some embodiments, the method further comprises determining the efficacy of the administered double-stranded ribonucleic acid (dsRNA) agent in the subject. In some embodiments, means of determining the efficacy of a treatment in a subject include: (i) determining one or more physiological characteristics of an LPA-related disease or condition in the subject; (ii) comparing the determined physiological profile to the baseline physiological characteristics of the LPA-related disease or condition before treatment, wherein the comparison results indicate one or more of the presence, absence, and levels of efficacy of the double-stranded ribonucleic acid (dsRNA) agent administered to the subject. In some embodiments, the identified physiological characteristic is the Lp(a) level in the blood. The decrease in LPA level in the blood indicates the existence of the effectiveness of the administration of double-stranded ribonucleic acid (dsRNA) agent to the subject.

According to another aspect of the present invention, provided is a method of reducing the LPA protein level in a subject compared to the baseline level of the LPA protein in the subject before treatment, the method comprises administering to the subject an effective amount of any embodiment of the aforementioned dsRNA agent of the present invention or any embodiment of the aforementioned composition of the present invention to reduce the level of LPA gene expression. In some embodiments, the dsRNA agent is administered subcutaneously to the subject or administered to the subject via IV

According to another aspect of the present invention, provided is a method of altering the physiological characteristics of an LPA-related disease or condition in a subject compared to the baseline physiological characteristics of the LPA-related disease or condition in the subject before treatment, the method comprises administering to the subject an effective amount of any embodiment of the aforementioned dsRNA agent of the present invention or any embodiment of the aforementioned composition of the present invention to alter the physiological characteristics of the LPA-related disease or condition in the subject. In some embodiments, the dsRNA agent is administered subcutaneously to the subject or administered to the subject via IV In certain embodiments, the physiological characteristic is the Lp(a) level in the blood.

### Description Of Sequences

Duplex AV00122 to AD00484-1, AD00474-2, AV01867-AV01968 are shown in Table 1 and their sense strand sequences are shown.

Duplex AV00122 to AD00484-1, AD00474-2, AV01867-AV01968 are shown in Table 1 and their antisense strand sequences are shown.

In the sequence shown in Table 2, chemical modifications are indicated as: capital: 2'-fluoro; lowercase: 2'-OMe; phosphorothioate: *.

In the sequences shown in Table 3, the delivery molecule used in the in vivo study is denoted as "GLO-0" at the 3'-end of each sense strand. The delivery molecule used in the in vivo study is denoted as "GLS-5" or "GLS-15" at the 5'-end of each sense strand, and chemical modifications are indicated as: capital: 2'-fluoro; lowercase: 2'-OMe; phosphorothioate: *, and unlocked nucleic acid: UNA.

The following is the mRNA sequence of human Lp(a) (SEQ ID NO: 1): NM_005577.4 Homo sapiens lipoprotein(a) (LPA), mRNA

GTAAGTCAACAATGTCCTGGGATTGGGACACACTTTCTGGGCACTGCTGGCCAGTCCCA
AAATGGAACATAAGGAAGTGGTTCTTCTACTTCTTTTATTTCTGAAATCAGCAGCACCTG
AGCAAAGCCATGTGGTCCAGGATTGCTACCATGGTGATGGACAGAGTTATCGAGGCACG
TACTCCACCACTGTCACAGGAAGGACCTGCCAAGCTTGGTCATCTATGACACCACATCA
ACATAATAGGACCACAGAAAACTACCCAAATGCTGGCTTGATCATGAACTACTGCAGGA
ATCCAGATGCTGTGGCAGCTCCTTATTGTTATACGAGGGATCCCGGTGTCAGGTGGGAGT
ACTGCAACCTGACGCAATGCTCAGACGCAGAAGGGACTGCCGTCGCGCCTCCGACTGT
TACCCCGGTTCCAAGCCTAGAGGCTCCTTCCGAACAAGCACCGACTGAGCAAAGGCCT
GGGGTGCAGGAGTGCTACCATGGTAATGGACAGAGTTATCGAGGCACATACTCCACCAC
TGTCACAGGAAGAACCTGCCAAGCTTGGTCATCTATGACACCACACTCGCATAGTCGGA
CCCCAGAATACTACCCAAATGCTGGCTTGATCATGAACTACTGCAGGAATCCAGATGCTG
TGGCAGCTCCTTATTGTTATACGAGGGATCCCGGTGTCAGGTGGGAGTACTGCAACCTG
ACGCAATGCTCAGACGCAGAAGGGACTGCCGTCGCGCCTCCGACTGTTACCCCGGTTCC
AAGCCTAGAGGCTCCTTCCGAACAAGCACCGACTGAGCAAAGGCCTGGGGTGCAGGAG
TGCTACCATGGTAATGGACAGAGTTATCGAGGCACATACTCCACCACTGTCACAGGAAG
AACCTGCCAAGCTTGGTCATCTATGACACCACACTCGCATAGTCGGACCCCAGAATACTA
CCCAAATGCTGGCTTGATCATGAACTACTGCAGGAATCCAGATGCTGTGGCAGCTCCTTA
TTGTTATACGAGGGATCCCGGTGTCAGGTGGGAGTACTGCAACCTGACGCAATGCTCAG
ACGCAGAAGGGACTGCCGTCGCGCCTCCGACTGTTACCCCGGTTCCAAGCCTAGAGGC
TCCTTCCGAACAAGCACCGACTGAGCAGAGGCCTGGGGTGCAGGAGTGCTACCACGGT
AATGGACAGAGTTATCGAGGCACATACTCCACCACTGTCACTGGAAGAACCTGCCAAGC
TTGGTCATCTATGACACCACACTCGCATAGTCGGACCCCAGAATACTACCCAAATGCTGG
CTTGATCATGAACTACTGCAGGAATCCAGATGCTGTGGCAGCTCCTTATTGTTATACGAG
GGATCCCGGTGTCAGGTGGGAGTACTGCAACCTGACGCAATGCTCAGACGCAGAAGGG
ACTGCCGTCGCGCCTCCGACTGTTACCCCGGTTCCAAGCCTAGAGGCTCCTTCCGAACA
AGCACCGACTGAGCAAAGGCCTGGGGTGCAGGAGTGCTACCATGGTAATGGACAGAGT
TATCGAGGCACATACTCCACCACTGTCACAGGAAGAACCTGCCAAGCTTGGTCATCTAT

GACACCACACTCGCATAGTCGGACCCCAGAATACTACCCAAATGCTGGCTTGATCATGA
ACTACTGCAGGAATCCAGATGCTGTGGCAGCTCCTTATTGTTATACGAGGGATCCCGGTG
TCAGGTGGGAGTACTGCAACCTGACGCAATGCTCAGACGCAGAAGGGACTGCCGTCGC
GCCTCCGACTGTTACCCCGGTTCCAAGCCTAGAGGCTCCTTCCGAACAAGCACCGACTG
AGCAAAGGCCTGGGGTGCAGGAGTGCTACCATGGTAATGGACAGAGTTATCGAGGCAC
ATACTCCACCACTGTCACAGGAAGAACCTGCCAAGCTTGGTCATCTATGACACCACACT
CGCATAGTCGGACCCCAGAATACTACCCAAATGCTGGCTTGATCATGAACTACTGCAGG
AATCCAGATGCTGTGGCAGCTCCTTATTGTTATACGAGGGATCCCGGTGTCAGGTGGGAG
TACTGCAACCTGACGCAATGCTCAGACGCAGAAGGGACTGCCGTCGCGCCTCCGACTG
TTACCCCGGTTCCAAGCCTAGAGGCTCCTTCCGAACAAGCACCGACTGAGCAAAGGCC
TGGGGTGCAGGAGTGCTACCATGGTAATGGACAGAGTTATCGAGGCACATACTCCACCA
CTGTCACAGGAAGAACCTGCCAAGCTTGGTCATCTATGACACCACACTCGCATAGTCGG
ACCCCAGAATACTACCCAAATGCTGGCTTGATCATGAACTACTGCAGGAATCCAGATGCT
GTGGCAGCTCCTTATTGTTATACGAGGGATCCCGGTGTCAGGTGGGAGTACTGCAACCT
GACGCAATGCTCAGACGCAGAAGGGACTGCCGTCGCGCCTCCGACTGTTACCCCGGTTC
CAAGCCTAGAGGCTCCTTCCGAACAAGCACCGACTGAGCAGAGGCCTGGGGTGCAGGA
GTGCTACCACGGTAATGGACAGAGTTATCGAGGCACATACTCCACCACTGTCACTGGAA
GAACCTGCCAAGCTTGGTCATCTATGACACCACACTCGCATAGTCGGACCCCAGAATAC
TACCCAAATGCTGGCTTGATCATGAACTACTGCAGGAATCCAGATCCTGTGGCAGCCCCT
TATTGTTATACGAGGGATCCCAGTGTCAGGTGGGAGTACTGCAACCTGACACAATGCTC
AGACGCAGAAGGGACTGCCGTCGCGCCTCCAACTATTACCCCGATTCCAAGCCTAGAGG
CTCCTTCTGAACAAGCACCAACTGAGCAAAGGCCTGGGGTGCAGGAGTGCTACCACGG
AAATGGACAGAGTTATCAAGGCACATACTTCATTACTGTCACAGGAAGAACCTGCCAAG
CTTGGTCATCTATGACACCACACTCGCATAGTCGGACCCCAGCATACTACCCAAATGCTG
GCTTGATCAAGAACTACTGCCGAAATCCAGATCCTGTGGCAGCCCCTTGGTGTTATACAA
CAGATCCCAGTGTCAGGTGGGAGTACTGCAACCTGACACGATGCTCAGATGCAGAATGG
ACTGCCTTCGTCCCTCCGAATGTTATTCTGGCTCCAAGCCTAGAGGCTTTTTTTGAACAA
GCACTGACTGAGGAAACCCCCGGGGTACAGGACTGCTACTACCATTATGGACAGAGTTA
CCGAGGCACATACTCCACCACTGTCACAGGAAGAACTTGCCAAGCTTGGTCATCTATGA

CACCACACCAGCATAGTCGGACCCCAGAAAACTACCCAAATGCTGGCCTGACCAGGAA
CTACTGCAGGAATCCAGATGCTGAGATTCGCCCTTGGTGTTACACCATGGATCCCAGTGT
CAGGTGGGAGTACTGCAACCTGACACAATGCCTGGTGACAGAATCAAGTGTCCTTGCA
ACTCTCACGGTGGTCCCAGATCCAAGCACAGAGGCTTCTTCTGAAGAAGCACCAACGG
AGCAAAGCCCCGGGGTCCAGGATTGCTACCATGGTGATGGACAGAGTTATCGAGGCTCA
TTCTCTACCACTGTCACAGGAAGGACATGTCAGTCTTGGTCCTCTATGACACCACACTG
GCATCAGAGGACAACAGAATATTATCCAAATGGTGGCCTGACCAGGAACTACTGCAGGA
ATCCAGATGCTGAGATTAGTCCTTGGTGTTATACCATGGATCCCAATGTCAGATGGGAGT
ACTGCAACCTGACACAATGTCCAGTGACAGAATCAAGTGTCCTTGCGACGTCCACGGCT
GTTTCTGAACAAGCACCAACGGAGCAAAGCCCCACAGTCCAGGACTGCTACCATGGTG
ATGGACAGAGTTATCGAGGCTCATTCTCCACCACTGTTACAGGAAGGACATGTCAGTCT
TGGTCCTCTATGACACCACACTGGCATCAGAGAACCACAGAATACTACCCAAATGGTGG
CCTGACCAGGAACTACTGCAGGAATCCAGATGCTGAGATTCGCCCTTGGTGTTATACCAT
GGATCCCAGTGTCAGATGGGAGTACTGCAACCTGACGCAATGTCCAGTGATGGAATCAA
CTCTCCTCACAACTCCCACGGTGGTCCCAGTTCCAAGCACAGAGCTTCCTTCTGAAGAA
GCACCAACTGAAAACAGCACTGGGGTCCAGGACTGCTACCGAGGTGATGGACAGAGTT
ATCGAGGCACACTCTCCACCACTATCACAGGAAGAACATGTCAGTCTTGGTCGTCTATG
ACACCACATTGGCATCGGAGGATCCCATTATACTATCCAAATGCTGGCCTGACCAGGAAC
TACTGCAGGAATCCAGATGCTGAGATTCGCCCTTGGTGTTACACCATGGATCCCAGTGTC
AGGTGGGAGTACTGCAACCTGACACGATGTCCAGTGACAGAATCGAGTGTCCTCACAA
CTCCCACAGTGGCCCCGGTTCCAAGCACAGAGGCTCCTTCTGAACAAGCACCACCTGA
GAAAAGCCCTGTGGTCCAGGATTGCTACCATGGTGATGGACGGAGTTATCGAGGCATAT
CCTCCACCACTGTCACAGGAAGGACCTGTCAATCTTGGTCATCTATGATACCACACTGGC
ATCAGAGGACCCCAGAAAACTACCCAAATGCTGGCCTGACCGAGAACTACTGCAGGAA
TCCAGATTCTGGGAAACAACCCTGGTGTTACACAACCGATCCGTGTGTGAGGTGGGAGT
ACTGCAATCTGACACAATGCTCAGAAACAGAATCAGGTGTCCTAGAGACTCCCACTGTT
GTTCCAGTTCCAAGCATGGAGGCTCATTCTGAAGCAGCACCAACTGAGCAAACCCCTGT
GGTCCGGCAGTGCTACCATGGTAATGGCCAGAGTTATCGAGGCACATTCTCCACCACTGT
CACAGGAAGGACATGTCAATCTTGGTCATCCATGACACCACACCGGCATCAGAGGACCC

CAGAAAACTACCCAAATGATGGCCTGACAATGAACTACTGCAGGAATCCAGATGCCGAT
ACAGGCCCTTGGTGTTTTACCATGGACCCCAGCATCAGGTGGGAGTACTGCAACCTGAC
GCGATGCTCAGACACAGAAGGGACTGTGGTCGCTCCTCCGACTGTCATCCAGGTTCCAA
GCCTAGGGCCTCCTTCTGAACAAGACTGTATGTTTGGGAATGGGAAAGGATACCGGGGC
AAGAAGGCAACCACTGTTACTGGGACGCCATGCCAGGAATGGGCTGCCCAGGAGCCCC
ATAGACACAGCACGTTCATTCCAGGGACAAATAAATGGGCAGGTCTGGAAAAAAATTAC
TGCCGTAACCCTGATGGTGACATCAATGGTCCCTGGTGCTACACAATGAATCCAAGAAA
ACTTTTTGACTACTGTGATATCCCTCTCTGTGCATCCTCTTCATTTGATTGTGGGAAGCCT
CAAGTGGAGCCGAAGAAATGTCCTGGAAGCATTGTAGGGGGGTGTGTGGCCCACCCAC
ATTCCTGGCCCTGGCAAGTCAGTCTCAGAACAAGGTTTGGAAAGCACTTCTGTGGAGG
CACCTTAATATCCCCAGAGTGGGTGCTGACTGCTGCTCACTGCTTGAAGAAGTCCTCAA
GGCCTTCATCCTACAAGGTCATCCTGGGTGCACACCAAGAAGTGAACCTCGAATCTCAT
GTTCAGGAAATAGAAGTGTCTAGGCTGTTCTTGGAGCCCACACAAGCAGATATTGCCTT
GCTAAAGCTAAGCAGGCCTGCCGTCATCACTGACAAAGTAATGCCAGCTTGTCTGCCAT
CCCCAGACTACATGGTCACCGCCAGGACTGAATGTTACATCACTGGCTGGGGAGAAACC
CAAGGTACCTTTGGGACTGGCCTTCTCAAGGAAGCCCAGCTCCTTGTTATTGAGAATGA
AGTGTGCAATCACTATAAGTATATTTGTGCTGAGCATTTGGCCAGAGGCACTGACAGTTG
CCAGGGTGACAGTGGAGGGCCTCTGGTTTGCTTCGAGAAGGACAAATACATTTTACAAG
GAGTCACTTCTTGGGGTCTTGGCTGTGCACGCCCCAATAAGCCTGGTGTCTATGCTCGTG
TTTCAAGGTTTGTTACTTGGATTGAGGGAATGATGAGAAATAATTAATTGGACGGGAGAC
AGAGTGAAGCATCAACCTACTTAGAAGCTGAAACGTGGGTAAGGATTTAGCATGCTGGA
AATAATAGACAGCAATCAAACGAAGACACTGTTCCCAGCTACCAGCTATGCCAAACCTT
GGCATTTTTGGTATTTTTGTGTATAAGCTTTTAAGGTCTGACTGACAAATTCTGTATTAAG
GTGTCATAGCTATGACATTTGTTAAAAATAAACTCTGCACTTATTTTGATTTGAA

### Brief Description of the Drawings

FIG. 1 shows a schematic diagram of serum LPA protein levels in monkeys;
FIG. 2 shows a schematic diagram of serum LPA protein levels of AD00480-8 at a dose of 2 mpk in monkeys.

### Detailed Description of the Embodiments

Some embodiments of the present invention include RNAi agents capable of inhibiting LPA (Apo(a)) gene expression, such as, but not limited to, double-stranded (ds) RNAi agents. Some embodiments of the present invention further include compositions comprising LPARNAi agents and methods for using the compositions. The LPA RNAi agents disclosed herein may be attached to a delivery compound for delivery to cells, including delivery to hepatocytes. The pharmaceutical composition of the present invention may comprise at least one dsRNA agent and delivery compound. In some embodiments of the present invention, the delivery compound is a GalNAc-containing delivery compound. The LPARNAi agent delivered to cells can inhibit LPA gene expression, thereby reducing the LPA protein product of the gene. The dsRNAi agent of the present invention can be used to treat LPA-related diseases and conditions. Such dsRNAi agents include, for example, the duplex AV00122 to AD00484-1, AD00474-2, AV01867-AV01968 shown in Table 1. In other embodiments, such dsRNAi agents include duplex variants, such as variants of duplex AV00122 to AD00484-1, AD00474-2, and AV01867-AV01968.

In some embodiments of the present invention, reducing LPA expression in cells or subjects treats diseases or conditions associated with LPA expression in cells or subjects, respectively. Non-limiting examples of the disease and condition that may be treated by reducing LPA expression are cardiovascular diseases, including Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles.

How to prepare and use compositions comprising LPA single-stranded (ssRNA) and double-stranded (dsRNA) agents to inhibit LPA gene expression, and compositions and methods for treating diseases and conditions caused or regulated by LPA gene expression are described below. The term "RNAi" is also known in the art and may be referred to as "siRNA".

As used herein, the term "RNAi" refers to agents that contain RNA and mediate targeted cleavage of RNA transcripts through the RNA-induced silencing complex (RISC) pathway. As is known in the art, the RNAi target region refers to a contiguous portion of the nucleotide sequence of an RNA molecule formed during gene transcription, which includes messenger RNA (mRNA) that is a processed product of the primary transcription product RNA. The target portion of this sequence will be at least long enough to be used as a substrate for RNAi-directed cleavage at or near that portion. A target sequence may be 8-30 nucleotides (inclusive) in length, 10-30 nucleotides (inclusive) in length, 12-25 nucleotides (inclusive) in length, 15-23 nucleotides (inclusive) in length, 16-23 nucleotides (inclusive) in length, or 18-23 nucleotides (inclusive) in length, and include all shorter lengths within each prescribed range. In some embodiments of the present invention, the target sequence is 9, 10, 11, 12, 13, 14, 15, 16, 17, 18, 19, 20, 21, 22, 23, 24, 25 or 26 nucleotides in length. In certain embodiments, the length of the target sequence is between 9 and 26 nucleotides (inclusive), including all sub-ranges and integers therebetween. For example, while not intended to be limiting, in some embodiments of the present invention, the target sequence is 8, 9, 10, 11, 12, 13, 14, 15, 16, 17, 18, 19, 20, 21, 22, 23, 24, 25, 26, 27, 28, 29, or 30 nucleotides in length, which is completely or at least substantially complementary to at least a portion of the RNA transcript of the LPA gene. Some aspects of the present invention include pharmaceutical compositions comprising one or more LPA dsRNA agents and pharmaceutically acceptable carriers. In some embodiments of the present invention, LPA RNAi as described herein inhibits the expression of LPA protein.

As used herein, "dsRNA agent" refers to a composition containing RNA or RNA-like (e.g., chemically modified RNA) oligonucleotide molecules capable of degrading target mRNA transcripts or inhibiting translation of target mRNA transcripts. Although not wishing to be limited to particular theories, a dsRNA agent of the present invention may function by an RNA interference mechanism (i.e., inducing the production of RNA interference by interacting with an RNA interference pathway mechanism in mammalian cells (RNA-induced silencing complex or RISC)), or by any alternative mechanism or pathway. Methods for achieving gene silencing in plant, invertebrate and vertebrate cells are well known in the art (see, for example, Sharp et al., Genes Dev. 2001, 15:485; Bernstein, et al., (2001) Nature 409:363; Nykanen, et al., (2001) Cell 107:309; and Elbashir, et al., (2001) Genes Dev. 15: 188)), the respective disclosures of which are incorporated herein by reference in their entirety. Gene silencing means known in the art may be used in conjunction with the disclosures provided herein to achieve inhibition of expression of LPA.

The dsRNA agent disclosed herein consists of a sense strand and an antisense strand, and includes but is not limited to: short interfering RNA (siRNA), RNAi agent, microRNA (miRNA), short hairpin RNA (shRNA), and a Dicer substrate. The antisense strand of the dsRNA agent described herein is at least partially complementary to the targeted mRNA, and it is understood in the art that dsRNA duplex structures of various lengths can be used to inhibit target gene expression. For example, dsRNAs with duplex structures of 19, 20, 21, 22 and 23 base pairs are known to efficiently induce RNA interference (Elbashir et al., EMBO 2001, 20: 6877-6888). It is also known in the art that shorter or longer RNA duplex structures can also effectively induce RNA interference. The LPA dsRNA in certain embodiments of the present invention may comprise at least one strand of at least 21 nt in length, or the duplex may have a length minus 1, 2, 3 nt or less based on the length of one of any of the sequences listed in Tables 1-3. Decreasing 4 nucleotides at one or both ends of the dsRNA can also be effective compared to the dsRNAs listed in Tables 1-3, respectively. In some embodiments of the present invention, an LPA dsRNA agent may have partial sequences of at least 15, 16, 17, 18, 19, 20 or more contiguous nucleotides from one or more sequences of Tables 1-3, and their ability to inhibit LPA gene expression differs by no more than 5%, 10%, 15%, 20%, 25% or 30% from the level of inhibition produced by dsRNA comprising the full sequence (herein also referred to as "parent" sequences).

Certain embodiments of compositions and methods of the present invention include single-stranded RNA in compositions and/or administrating single-stranded RNA to a subject. For example, the antisense strands listed in any of Tables 1-3 may be used as or within a composition which, when administered to a subject, reduces the expression of the LPA polypeptide and/or the LPA gene in the subject. Tables 1-3 show the antisense strand and sense strand core extension base sequences of some LPA dsRNA agents. Single-stranded antisense molecules that may be included in certain compositions of the present invention and/or administered in certain methods of the present invention are referred to herein as "single-stranded antisense agent" or "antisense polynucleotide agent". Single-stranded sense molecules that can be included in certain compositions and/or administered in certain methods of the present invention are referred to herein as "single-stranded sense agent" or "sense polynucleotide agent". The term "base sequence" is used herein to refer to polynucleotide sequences without chemical modifications or delivery compounds. For example, the sense strand shown in Table 1 corresponds to the corresponding base sequence in Table 3; but the respective chemical modifications and delivery compounds are shown in the corresponding sequences in Table 3. Sequences disclosed herein may be assigned identifiers. For example, a single-stranded sense sequence can be identified by "sense strand SS#"; a single-stranded antisense sequence can be identified by "antisense strand AS#"; and a duplex comprising a sense strand and an antisense strand can be identified by "duplex AD#".

Table 1 includes both the sense and antisense strands and provides identification numbers for the duplex formed by the sense and antisense strands on the same row in Table 1. In some embodiments of the present invention, the antisense sequence contains either a nucleobase u or a nucleobase a at its position 1. In some embodiments of the present invention, the antisense sequence contains a nucleobase u at position 1 of the antisense sequence. As used here, the term "matching position" in a sense refers to the position in each chain that "pairs" with each other when two chains form a duplex. For example, in the 21-nucleobase sense strand and the 21-nucleobase antisense strand, the position 1 of the sense strand is in the "matching position" with the nucleobase at the position 21 of the antisense strand. In another non-limiting example, for the 23-nucleobase sense strand and the 23-nucleobase antisense strand, the nucleobase at position 2 of the sense strand is in the "matching position" with the nucleobase at the position 22 of the antisense strand. In another non-limiting example, in the 18-nucleobase sense strand and the 18-nucleobase antisense strand, the nucleobase at position 1 of the sense strand is in a matching position with the nucleobase at position 18 of the antisense strand; And the nucleobase at position 4 in the sense strand is in a matching position with the nucleobase at position 15 in the antisense strand. A technician will understand how to identify matching positions between the sense and antisense strands of double and paired strands.

A column in Table 1 represents the duplex AV#, AD# of the duplex, which contains both sense and antisense sequences in the same row in the table. For example, Table 1 discloses a duplex designated as "duplex AV00122", which contains corresponding sense strand sequence and antisense strand sequence. Thus, each row in Table 1 identifies the duplex of the present invention, and each duplex comprises a sense sequence and an antisense sequence displayed in the same row, and the designated identifier for each duplex is displayed in the last column of the row.

In some embodiments of the method of the present invention, an RNAi agent comprising the polynucleotide sequence shown in Table 1 is administered to the subject. In some embodiments of the present invention, the RNAi agent administered to the subject comprises a duplex comprising at least one of the base sequences listed in Table 1 and comprising 0, 1, 2, 3, 4, 5, 6, 7, 8, 9, 10, 11, 12, 13, 14, 15, 16, 17, 18, 19, 20, 21, 22, 23, or 24 sequence modifications. In some embodiments of the method of the present invention, it is also included to attach RNAi agent of the polynucleotide sequence shown in Table 1 to a delivery molecule, the non-limiting example of which is a GalNAc-containing delivery compound.

**Table 1: Antisense and sense strand sequences of LPA RNAi agent without modification. All sequences are shown in 5' to 3' directions. The duplex AV# or AD# is the number assigned to the duplex of two chains in the same row in the table**

| Duplex number | Sense chain base sequence | Seq ID NO | Antisense strand base sequence | Seq ID NO |
|---|---|---|---|---|
| AV00122 | CUGGUCCAGGAUUGCUACCAA | 2 | UUGGUAGCAAUCCUGGACC AG | 129 |
| AV00123 | CUGAUGGACAGAGUUAUCGAA | 3 | UUCGAUAACUCUGUCCAUC AG | 130 |
| AV00124 | CAUGGACAGAGUUAUCGAGGA | 4 | UCCUCGAUAACUCUGUCCA UG | 131 |
| AV00125 | GCCUGCCAAGCUUGGUCAUCA | 5 | UGAUGACCAAGCUUGGCAG GC | 132 |
| AV00126 | GCUGCCAAGCUUGGUCAUCUA | 6 | UAGAUGACCAAGCUUGGCA | 133 |
| | | | GC | |
| AV00127 | CCCAAGCUUGGUCAUCUAUGA | 7 | UCAUAGAUGACCAAGCUUG GG | 134 |
| AV00128 | GAAGCUUGGUCAUCUAUGACA | 8 | UGUCAUAGAUGACCAAGCU UC | 135 |
| AV00129 | GACUACCCAAAUGCUGGCUUA | 9 | UAAGCCAGCAUUUGGGUAG UC | 136 |
| AV00130 | GUACCCAAAUGCUGGCUUGAA | 10 | UUCAAGCCAGCAUUUGGGU AC | 137 |
| AV00131 | GACCCAAAUGCUGGCUUGAUA | 11 | UAUCAAGCCAGCAUUUGGG UC | 138 |
| AV00132 | GGCUCCUUAUUGUUAUACGAA | 12 | UUCGUAUAACAAUAAGGAG CC | 139 |
| AV00133 | GGCCUAGAGGCUCCUUCCGAA | 13 | UUCGGAAGGAGCCUCUAGG CC | 140 |
| AV00134 | GUAGAGGCUCCUUCCGAACAA | 14 | UUGUUCGGAAGGAGCCUCU AC | 141 |
| AV00135 | GAAGCACCGACUGAGCAAAGA | 15 | UCUUUGCUCAGUCGGUGCU UC | 142 |
| AV00136 | CGAGUGCUACCAUGGUAAUGA | 16 | UCAUUACCAUGGUAGCACU CG | 143 |
| AV00137 | GGUGCUACCAUGGUAAUGGAA | 17 | UUCCAUUACCAUGGUAGCA CC | 144 |
| AV00138 | GACCAUGGUAAUGGACAGAGA | 18 | UCUCUGUCCAUUACCAUGG UC | 145 |
| AV00139 | GUGGUAAUGGACAGAGUUAUA | 19 | UAUAACUCUGUCCAUUACC AC | 146 |
| AV00140 | GGGUAAUGGACAGAGUUAUCA | 20 | UGAUAACUCUGUCCAUUAC | 147 |
| | | | CC | |
| AV00141 | CUAAUGGACAGAGUUAUCGAA | 21 | UUCGAUAACUCUGUCCAUU AG | 148 |
| AV00142 | GAGAGUUAUCGAGGCACAUAA | 22 | UUAUGUGCCUCGAUAACUC UC | 149 |
| AV00143 | CAGUUAUCGAGGCACAUACUA | 23 | UAGUAUGUGCCUCGAUAAC UG | 150 |
| AV00144 | GACCACGGUAAUGGACAGAGA | 24 | UCUCUGUCCAUUACCGUGG UC | 151 |
| AV00145 | GACUGCAGGAAUCCAGAUCCA | 25 | UGGAUCUGGAUUCCUGCAG UC | 152 |
| AV00146 | GGGCAGCCCCUUAUUGUUAUA | 26 | UAUAACAAUAAGGGCUGC CC | 153 |
| AV00147 | GAGCCCCUUAUUGUUAUACGA | 27 | UCGUAUAACAAUAAGGGGC UC | 154 |
| AV00148 | GCAGUGUCAGGUGGGAGUACA | 28 | UGUACUCCCACCUGACACUG C | 155 |
| AV00149 | CUACUGCAACCUGACACAAUA | 29 | UAUUGUGUCAGGUUGCAGU AG | 156 |
| AV00150 | GGACACAAUGCUCAGACGCAA | 30 | UUGCGUCUGAGCAUUGUGU CC | 157 |
| AV00151 | GUAGAGGCUCCUUCUGAACAA | 31 | UUGUUCAGAAGGAGCCUCU AC | 158 |
| AV00152 | CAGGCUCCUUCUGAACAAGCA | 32 | UGCUUGUUCAGAAGGAGCC UG | 159 |
| AV00153 | GAAGCACCAACUGAGCAAAGA | 33 | UCUUUGCUCAGUUGGUGCU UC | 160 |
| AV00154 | GGGAAAUGGACAGAGUUAUCA | 34 | UGAUAACUCUGUCCAUUUC | 161 |
| | | | CC | |
| AV00155 | CAAAUGGACAGAGUUAUCAAA | 35 | UUUGAUAACUCUGUCCAUU UG | 162 |
| AV00156 | GGGACAGAGUUAUCAAGGCAA | 36 | UUGCCUUGAUAACUCUGUC CC | 163 |
| AV00157 | GUCAAGAACUACUGCCGAAAA | 37 | UUUUCGGCAGUAGUUCUUG AC | 164 |
| AV00158 | GCAAGAACUACUGCCGAAAUA | 38 | UAUUUCGGCAGUAGUUCUU GC | 165 |
| AV00159 | GAAGAACUACUGCCGAAAUCA | 39 | UGAUUUCGGCAGUAGUUCU UC | 166 |
| AV00160 | GACUGCCGAAAUCCAGAUCCA | 40 | UGGAUCUGGAUUUCGGCAG UC | 167 |
| AV00161 | CUACUGCAACCUGACACGAUA | 41 | UAUCGUGUCAGGUUGCAGU AG | 168 |
| AV00162 | GGACACGAUGCUCAGAUGCAA | 42 | UUGCAUCUGAGCAUCGUGU CC | 169 |
| AV00163 | CGACUGCUACUACCAUUAUGA | 43 | UCAUAAUGGUAGUAGCAGU CG | 170 |
| AV00164 | CACUGCUACUACCAUUAUGGA | 44 | UCCAUAAUGGUAGUAGCAG UG | 171 |
| AV00165 | GCUGCUACUACCAUUAUGGAA | 45 | UUCCAUAAUGGUAGUAGCA GC | 172 |
| AV00166 | GACUACCAUUAUGGACAGAGA | 46 | UCUCUGUCCAUAAUGGUAG UC | 173 |
| AV00167 | GCUUGCCAAGCUUGGUCAUCA | 47 | UGAUGACCAAGCUUGGCAA GC | 174 |
| AV00168 | GUUGCCAAGCUUGGUCAUCUA | 48 | UAGAUGACCAAGCUUGGCA | 175 |
| | | | AC | |
| AV00169 | GAUAGUCGGACCCCAGAAAAA | 49 | UUUUUCUGGGGUCCGACUA UC | 176 |
| AV00170 | GCAGGAACUACUGCAGGAAUA | 50 | UAUUCCUGCAGUAGUUCCU GC | 177 |
| AV00171 | CAAUCCAGAUGCUGAGAUUCA | 51 | UGAAUCUCAGCAUCUGGAU UG | 178 |
| AV00172 | CAGAUUCGCCCUUGGUGUUAA | 52 | UUAACACCAAGGGCGAAUC UG | 179 |
| AV00173 | CCCCUUGGUGUUACACCAUGA | 53 | UCAUGGUGUAACACCAAGG GG | 180 |
| AV00174 | GUGGUGACAGAAUCAAGUGUA | 54 | UACACUUGAUUCUGUCACC AC | 181 |
| AV00175 | GAUCAAGUGUCCUUGCAACUA | 55 | UAGUUGCAAGGACACUUGA UC | 182 |
| AV00176 | CGGGUCCAGGAUUGCUACCAA | 56 | UUGGUAGCAAUCCUGGACC CG | 183 |
| AV00177 | GUGUCACAGGAAGGACAUGUA | 57 | UACAUGUCCUUCCUGUGAC AC | 184 |
| AV00178 | CGAAGGACAUGUCAGUCUUGA | 58 | UCAAGACUGACAUGUCCUU CG | 185 |
| AV00179 | CAAGGACAUGUCAGUCUUGGA | 59 | UCCAAGACUGACAUGUCCU UG | 186 |
| AV00180 | CUCAGUCUUGGUCCUCUAUGA | 60 | UCAUAGAGGACCAAGACUG AG | 187 |
| AV00181 | CUCCUUGGUGUUAUACCAUGA | 61 | UCAUGGUAUAACACCAAGG AG | 188 |
| AV00182 | GUGGUGUUAUACCAUGGAUCA | 62 | UGAUCCAUGGUAUAACACC | 189 |
| | | | AC | |
| AV00183 | GGGUGUUAUACCAUGGAUCCA | 63 | UGGAUCCAUGGUAUAACAC CC | 190 |
| AV00184 | CUUAUACCAUGGAUCCCAAUA | 64 | UAUUGGGAUCCAUGGUAUA AG | 191 |
| AV00185 | GAUACCAUGGAUCCCAAUGUA | 65 | UACAUUGGGAUCCAUGGUA UC | 192 |
| AV00186 | GCCUGACACAAUGUCCAGUGA | 66 | UCACUGGACAUUGUGUCAG GC | 193 |
| AV00187 | GGACACAAUGUCCAGUGACAA | 67 | UUGUCACUGGACAUUGUGU CC | 194 |
| AV00188 | GAAUGUCCAGUGACAGAAUCA | 68 | UGAUUCUGUCACUGGACAU UC | 195 |
| AV00189 | GAUGUCCAGUGACAGAAUCAA | 69 | UUGAUUCUGUCACUGGACA UC | 196 |
| AV00190 | GUGUCCAGUGACAGAAUCAAA | 70 | UUUGAUUCUGUCACUGGAC AC | 197 |
| AV00191 | GGUCCAGUGACAGAAUCAAGA | 71 | UCUUGAUUCUGUCACUGGA CC | 198 |
| AV00192 | GCAGUGACAGAAUCAAGUGUA | 72 | UACACUUGAUUCUGUCACU GC | 199 |
| AV00193 | GAUUCUCCACCACUGUUACAA | 73 | UUGUAACAGUGGUGGAGAA UC | 200 |
| AV00194 | GCCACCACUGUUACAGGAAGA | 74 | UCUUCCUGUAACAGUGGUG GC | 201 |
| AV00195 | GACAGAAUACUACCCAAAUGA | 75 | UCAUUUGGGUAGUAUUCUG UC | 202 |
| AV00196 | GCAGAAUACUACCCAAAUGGA | 76 | UCCAUUUGGGUAGUAUUCU | 203 |
| | | | GC | |
| AV00197 | GGAUUCGCCCUUGGUGUUAUA | 77 | UAUAACACCAAGGGCGAAU CC | 204 |
| AV00198 | CAUUCGCCCUUGGUGUUAUAA | 78 | UUAUAACACCAAGGGCGAA UG | 205 |
| AV00199 | GUCGCCCUUGGUGUUAUACCA | 79 | UGGUAUAACACCAAGGGCG AC | 206 |
| AV00200 | CCCCUUGGUGUUAUACCAUGA | 80 | UCAUGGUAUAACACCAAGG GG | 207 |
| AV00201 | GGAUGUCCAGUGACAGAAUCA | 81 | UGAUUCUGUCACUGGACAU CC | 208 |
| AV00202 | GCAGAGGCUCCUUCUGAACAA | 82 | UUGUUCAGAAGGAGCCUCU GC | 209 |
| AV00203 | GAUCUUGGUCAUCUAUGAUAA | 83 | UUAUCAUAGAUGACCAAGA UC | 210 |
| AV00204 | GGACACAAUGCUCAGAAACAA | 84 | UUGUUUCUGAGCAUUGUGU CC | 211 |
| AV00205 | CCAGUGCUACCAUGGUAAUGA | 85 | UCAUUACCAUGGUAGCACU GG | 212 |
| AD0014 9 | CAGCCCCUUAUUGUUAUACGA | 86 | UCGUAUAACAAUAAGGGGC UG | 213 |
| AD0015 0 | AGUUAUCGAGGCUCAUUCUCA | 87 | UGAGAAUGAGCCUCGAUAA CU | 214 |
| AD0015 1 | UGACACAAUGCUCAGACGCAA | 88 | UUGCGUCUGAGCAUUGUGU CA | 215 |
| AD0033 1 | GACCCAAAUGCUGGCUUGAUA | 89 | UAUCAAGCCAGCAUUUGGG UC | 216 |
| AD00332 | GGGUAAUGGACAGAGUUAUCA | 90 | UGAUAACUCUGUCCAUUAC | 217 |
| | | | CC | |
| AD0033 3 | CUAAUGGACAGAGUUAUCGAA | 91 | UUCGAUAACUCUGUCCAUU AG | 218 |
| AD0033 4 | CAGUUAUCGAGGCACAUACUA | 92 | UAGUAUGUGCCUCGAUAAC UG | 219 |
| AD0033 5 | CUACUGCAACCUGACACAAUA | 93 | UAUUGUGUCAGGUUGCAGU AG | 220 |
| AD0033 6 | CGACUGCUACUACCAUUAUGA | 94 | UCAUAAUGGUAGUAGCAGU CG | 221 |
| AD0033 7 | GUUGCCAAGCUUGGUCAUCUA | 95 | UAGAUGACCAAGCUUGGCA AC | 222 |
| AD0033 8 | CAGAUUCGCCCUUGGUGUUAA | 96 | UUAACACCAAGGGCGAAUC UG | 223 |
| AD0033 9 | CGAAGGACAUGUCAGUCUUGA | 97 | UCAAGACUGACAUGUCCUU CG | 224 |
| AD0034 0 | CUCCUUGGUGUUAUACCAUGA | 98 | UCAUGGUAUAACACCAAGG AG | 225 |
| AD0034 1 | CCCCUUGGUGUUAUACCAUGA | 99 | UCAUGGUAUAACACCAAGG GG | 226 |
| AD0034 2 | GGACACAAUGCUCAGAAACAA | 100 | UUGUUUCUGAGCAUUGUGU CC | 227 |
| AD0043 3 | CUGAUGGACAGAGUUAUCGAA | 101 | UUCGAUAACUCUGUCCAUC AG | 228 |
| AD0043 4 | GGCUCCUUAUUGUUAUACGAA | 102 | UUCGUAUAACAAUAAGGAG CC | 229 |
| AD0043 5 | GAAGCACCAACUGAGCAAAGA | 103 | UCUUUGCUCAGUUGGUGCU UC | 230 |
| AD00436 | GGGAAAUGGACAGAGUUAUCA | 104 | UGAUAACUCUGUCCAUUUC | 231 |
| | | | CC | |
| AD0043 7 | GCUGCUACUACCAUUAUGGAA | 105 | UUCCAUAAUGGUAGUAGCA GC | 232 |
| AD0043 8 | CAAUCCAGAUGCUGAGAUUCA | 106 | UGAAUCUCAGCAUCUGGAU UG | 233 |
| AD0043 9 | GAUUCUCCACCACUGUUACAA | 107 | UUGUAACAGUGGUGGAGAA UC | 234 |
| AD0044 0 | GCAGAAUACUACCCAAAUGGA | 108 | UCCAUUUGGGUAGUAUUCU GC | 235 |
| AD0044 1 | GGAUUCGCCCUUGGUGUUAUA | 109 | UAUAACACCAAGGGCGAAU CC | 236 |
| AD0044 2 | CAUUCGCCCUUGGUGUUAUAA | 110 | UUAUAACACCAAGGGCGAA UG | 237 |
| AD0047 4 | CAUGGACAGAGUUAUCGAGGA | 111 | UCCUCGAUAACUCUGUCCA UG | 238 |
| AD0047 5 | GCUGCCAAGCUUGGUCAUCUA | 112 | UAGAUGACCAAGCUUGGCA GC | 239 |
| AD0047 6 | CCCAAGCUUGGUCAUCUAUGA | 113 | UCAUAGAUGACCAAGCUUG GG | 240 |
| AD0047 7 | GGUGCUACCAUGGUAAUGGAA | 114 | UUCCAUUACCAUGGUAGCA CC | 241 |
| AD0047 8 | GACCAUGGUAAUGGACAGAGA | 115 | UCUCUGUCCAUUACCAUGG UC | 242 |
| AD0047 9 | GUGGUAAUGGACAGAGUUAUA | 116 | UAUAACUCUGUCCAUUACC AC | 243 |
| AD0048 0 | GAGAGUUAUCGAGGCACAUAA | 117 | UUAUGUGCCUCGAUAACUC UC | 244 |
| AD00481 | CAAAUGGACAGAGUUAUCAAA | 118 | UUUGAUAACUCUGUCCAUU | 245 |
| | | | UG | |
| AD0048 2 | GGGACAGAGUUAUCAAGGCAA | 119 | UUGCCUUGAUAACUCUGUC CC | 246 |
| AD0048 3 | GAUCAAGUGUCCUUGCAACUA | 120 | UAGUUGCAAGGACACUUGA UC | 247 |
| AD0048 4 | CGGGUCCAGGAUUGCUACCAA | 121 | UUGGUAGCAAUCCUGGACC CG | 248 |
| AD0048 5 | GUGUCACAGGAAGGACAUGUA | 122 | UACAUGUCCUUCCUGUGAC AC | 249 |
| AD0048 6 | GAUGUCCAGUGACAGAAUCAA | 123 | UUGAUUCUGUCACUGGACA UC | 250 |
| AD0048 7 | GCAGAGGCUCCUUCUGAACAA | 124 | UUGUUCAGAAGGAGCCUCU GC | 251 |
| AD0047 4-1 | CAUGGACAGAGUUAUCGAGGA | 125 | UCCUCGAUAACUCUGUCCA UG | 252 |
| AD0047 8-1 | GACCAUGGUAAUGGACAGAGA | 126 | UCUCUGUCCAUUACCAUGG UC | 253 |
| AD0048 2-1 | GGGACAGAGUUAUCAAGGCAA | 127 | UUGCCUUGAUAACUCUGUC CC | 254 |
| AD0048 4-1 | CGGGUCCAGGAUUGCUACCAA | 128 | UUGGUAGCAAUCCUGGACC CG | 255 |
| AD0047 4-2 | GAUGGACAGAGUUAUCGAGGU | 514 | ACCUCGAUAACUCUGUCCA UC | 608 |
| AV01867 | GAGAGUUAUCGAGGCACAUAA | 515 | UUAUGUGCCUCGAUAACUC UC | 609 |
| AV01868 | GAGAGUUAUCGAGGCACAUAU | 516 | AUAUGUGCCUCGAUAACUC UC | 610 |
| AV01869 | GAGAGUUAUCGAGGCACAUAC | 517 | GUAUGUGCCUCGAUAACUC UC | 611 |
| AV01870 | GAGAGUUAUCGAGGCACAUAG | 518 | CUAUGUGCCUCGAUAACUC UC | 612 |
| AV01871 | GUUAUCGAGGCACAUAA | 519 | UUAUGUGCCUCGAUAAC | 613 |
| AV01872 | AGUUAUCGAGGCACAUAA | 520 | UUAUGUGCCUCGAUAACU | 614 |
| AV01873 | AAGUUAUCGAGGCACAUAA | 521 | UUAUGUGCCUCGAUAACUU | 615 |
| AV01874 | UAGUUAUCGAGGCACAUAA | 522 | UUAUGUGCCUCGAUAACUA | 616 |
| AV01875 | GAGUUAUCGAGGCACAUAA | 523 | UUAUGUGCCUCGAUAACUC | 617 |
| AV01876 | CAGUUAUCGAGGCACAUAA | 524 | UUAUGUGCCUCGAUAACUG | 618 |
| AV01877 | AGAGUUAUCGAGGCACAUAA | 525 | UUAUGUGCCUCGAUAACUC U | 619 |
| AV01878 | UGAGUUAUCGAGGCACAUAA | 526 | UUAUGUGCCUCGAUAACUC A | 620 |
| AV01879 | GGAGUUAUCGAGGCACAUAA | 527 | UUAUGUGCCUCGAUAACUC C | 621 |
| AV01880 | GGAGUUAUCGAGGCACAUAA | 528 | UUAUGUGCCUCGAUAACUC G | 622 |
| AV01881 | UAGAGUUAUCGAGGCACAUAA | 529 | UUAUGUGCCUCGAUAACUC UA | 623 |
| AV01882 | CAGAGUUAUCGAGGCACAUAA | 530 | UUAUGUGCCUCGAUAACUC UG | 624 |
| AV01883 | AAGAGUUAUCGAGGCACAUAA | 531 | UUAUGUGCCUCGAUAACUC UU | 625 |
| AV01884 | ACAGAGUUAUCGAGGCACAUA A | 532 | UUAUGUGCCUCGAUAACUC UGU | 626 |
| AV01885 | GACAGAGUUAUCGAGGCACAU AA | 533 | UUAUGUGCCUCGAUAACUC UGUC | 627 |
| AV01886 | GGACAGAGUUAUCGAGGCACA UAA | 534 | UUAUGUGCCUCGAUAACUC UGUCC | 628 |
| AV01887 | UGGACAGAGUUAUCGAGGCAC AUAA | 535 | UUAUGUGCCUCGAUAACUC UGUCCA | 629 |
| AV01888 | AUGGACAGAGUUAUCGAGGCA CAUAA | 536 | UUAUGUGCCUCGAUAACUC UGUCCAU | 630 |
| AV01889 | AUGGUAAUGGACAGAGUUAUC GAGGCACAUAA | 537 | UUAUGUGCCUCGAUAACUC UGUCCAUUACCAU | 631 |
| AV01890 | GAGAGUUAUCGAGGCACAUAA | 538 | UUAUGUGCCUCGAUAACUC UCUU | 632 |
| AV01891 | GAGAGUUAUCGAGGCACAUAA | 539 | UUAUGUGCCUCGAUAACUC UCGA | 633 |
| AV01892 | GAGAGUUAUCGAGGCACAUAA | 540 | UUAUGUGCCUCGAUAACUC UC | 634 |
| AV01893 | GAGAGUUAUCGAGGCACAUAA | 541 | UUAUGUGCCUCGAUAACUC UC | 635 |
| AV01894 | GAGAGUUAUCGAGGCACAUAA | 542 | UUAUGUGCCUCGAUAACUC UC | 636 |
| AV01895 | GAGAGUUAUCGAGGCGCAUAA | 543 | UUAUGUGCCUCGAUAACUC UC | 637 |
| AV01896 | GAGAGUUAUCGAGGCACAUUA | 544 | UAAUGUGCCUCGAUAACUC UC | 638 |
| AV01897 | GAGAGUUAUCGAGGCACAUAA | 545 | UUAUGUGCCUCGAUAACUC UC | 639 |
| AV01898 | GUUGCCAAGCUUGGUCAUCUA | 546 | UAGAUGACCAAGCUUGGCA AC | 640 |
| AV01899 | GUUGCCAAGCUUGGUCAUCUU | 547 | AAGAUGACCAAGCUUGGCA AC | 641 |
| AV01900 | GUUGCCAAGCUUGGUCAUCUC | 548 | GAGAUGACCAAGCUUGGCA AC | 642 |
| AV01901 | GUUGCCAAGCUUGGUCAUCUG | 549 | CAGAUGACCAAGCUUGGCA AC | 643 |
| AV01902 | CCAAGCUUGGUCAUCUA | 550 | UAGAUGACCAAGCUUGG | 644 |
| AV01903 | GCCAAGCUUGGUCAUCUA | 551 | UAGAUGACCAAGCUUGGC | 645 |
| AV01904 | AGCCAAGCUUGGUCAUCUA | 552 | UAGAUGACCAAGCUUGGCU | 646 |
| AV01905 | UGCCAAGCUUGGUCAUCUA | 553 | UAGAUGACCAAGCUUGGCA | 647 |
| AV01906 | GGCCAAGCUUGGUCAUCUA | 554 | UAGAUGACCAAGCUUGGCC | 648 |
| AV01907 | CGCCAAGCUUGGUCAUCUA | 555 | UAGAUGACCAAGCUUGGCG | 649 |
| AV01908 | AUGCCAAGCUUGGUCAUCUA | 556 | UAGAUGACCAAGCUUGGCA U | 650 |
| AV01909 | UUGCCAAGCUUGGUCAUCUA | 557 | UAGAUGACCAAGCUUGGCA A | 651 |
| AV01910 | GUGCCAAGCUUGGUCAUCUA | 558 | UAGAUGACCAAGCUUGGCA C | 652 |
| AV01911 | CUGCCAAGCUUGGUCAUCUA | 559 | UAGAUGACCAAGCUUGGCA G | 653 |
| AV01912 | UUUGCCAAGCUUGGUCAUCUA | 560 | UAGAUGACCAAGCUUGGCA AA | 654 |
| AV01913 | CUUGCCAAGCUUGGUCAUCUA | 561 | UAGAUGACCAAGCUUGGCA AG | 655 |
| AV01914 | AUUGCCAAGCUUGGUCAUCUA | 562 | UAGAUGACCAAGCUUGGCA AU | 656 |
| AV01915 | ACUUGCCAAGCUUGGUCAUCU A | 563 | UAGAUGACCAAGCUUGGCA AGU | 657 |
| AV01916 | AACUUGCCAAGCUUGGUCAUC UA | 564 | UAGAUGACCAAGCUUGGCA AGUU | 658 |
| AV01917 | GAACUUGCCAAGCUUGGUCAU CUA | 565 | UAGAUGACCAAGCUUGGCA AGUUC | 659 |
| AV01918 | AGAACUUGCCAAGCUUGGUCA UCUA | 566 | UAGAUGACCAAGCUUGGCA AGUUCU | 660 |
| AV01919 | AAGAACUUGCCAAGCUUGGUC AUCUA | 567 | UAGAUGACCAAGCUUGGCA AGUUCUU | 661 |
| AV01920 | CACAGGAAGAACUUGCCAAGC UUGGUCAUCUA | 568 | UAGAUGACCAAGCUUGGCA AGUUCUUCCUGUG | 662 |
| AV01921 | GUUGCCAAGCUUGGUCAUCUA | 569 | UAGAUGACCAAGCUUGGCA ACUU | 663 |
| AV01922 | GUUGCCAAGCUUGGUCAUCUA | 570 | UAGAUGACCAAGCUUGGCA ACGA | 664 |
| AV01923 | GUUGCCAAGCUUGGUCAUCUA | 571 | UAGAUGACCAAGCUUGGCA AC | 665 |
| AV01924 | GUUGCCAAGCUUGGUCAUCUA | 572 | UAGAUGACCAAGCUUGGCA AC | 666 |
| AV01925 | GUUGCCAAGCUUGGUCAUCUA | 573 | UAGAUGACCAAGCUUGGCA AC | 667 |
| AV01926 | GUUGCCAAGCUUGGUUAUCUA | 574 | UAGAUGACCAAGCUUGGCA AC | 668 |
| AV01927 | GUUGCCAAGCUUGGUCAUCCA | 575 | UGGAUGACCAAGCUUGGCA AC | 669 |
| AV01928 | GUUGCCAAGCUUGGUCAUCUA | 576 | UAGAUGACCAAGCUUGGCA AC | 670 |
| AV01929 | CAUGGACAGAGUUAUCGAGGA | 577 | UCCUCGAUAACUCUGUCCA UG | 671 |
| AV01930 | CAUGGACAGAGUUAUCGAGGU | 578 | ACCUCGAUAACUCUGUCCA UG | 672 |
| AV01931 | CAUGGACAGAGUUAUCGAGGC | 579 | GCCUCGAUAACUCUGUCCA UG | 673 |
| AV01932 | CAUGGACAGAGUUAUCGAGGG | 580 | CCCUCGAUAACUCUGUCCAU G | 674 |
| AV01933 | GACAGAGUUAUCGAGGA | 581 | UCCUCGAUAACUCUGUC | 675 |
| AV01934 | GGACAGAGUUAUCGAGGA | 582 | UCCUCGAUAACUCUGUCC | 676 |
| AV01935 | AGGACAGAGUUAUCGAGGA | 583 | UCCUCGAUAACUCUGUCCU | 677 |
| AV01936 | UGGACAGAGUUAUCGAGGA | 584 | UCCUCGAUAACUCUGUCCA | 678 |
| AV01937 | GGGACAGAGUUAUCGAGGA | 585 | UCCUCGAUAACUCUGUCCC | 679 |
| AV01938 | CGGACAGAGUUAUCGAGGA | 586 | UCCUCGAUAACUCUGUCCG | 680 |
| AV01939 | AUGGACAGAGUUAUCGAGGA | 587 | UCCUCGAUAACUCUGUCCA U | 681 |
| AV01940 | UUGGACAGAGUUAUCGAGGA | 588 | UCCUCGAUAACUCUGUCCA A | 682 |
| AV01941 | GUGGACAGAGUUAUCGAGGA | 589 | UCCUCGAUAACUCUGUCCAC | 683 |
| AV01942 | GUGGACAGAGUUAUCGAGGA | 590 | UCCUCGAUAACUCUGUCCA G | 684 |
| AV01943 | UAUGGACAGAGUUAUCGAGGA | 591 | UCCUCGAUAACUCUGUCCA UA | 685 |
| AV01944 | GAUGGACAGAGUUAUCGAGGA | 592 | UCCUCGAUAACUCUGUCCA UC | 686 |
| AV01945 | AAUGGACAGAGUUAUCGAGGA | 593 | UCCUCGAUAACUCUGUCCA UU | 687 |
| AV01946 | UGAUGGACAGAGUUAUCGAGG A | 594 | UCCUCGAUAACUCUGUCCA UCA | 688 |
| AV01947 | GUGAUGGACAGAGUUAUCGAG GA | 595 | UCCUCGAUAACUCUGUCCA UCAC | 689 |
| AV01948 | GGUGAUGGACAGAGUUAUCGA GGA | 596 | UCCUCGAUAACUCUGUCCA UCACC | 690 |
| AV01949 | UGGUGAUGGACAGAGUUAUCG AGGA | 597 | UCCUCGAUAACUCUGUCCA UCACCA | 691 |
| AV01950 | AUGGUGAUGGACAGAGUUAUC GAGGA | 598 | UCCUCGAUAACUCUGUCCA UCACCAU | 692 |
| AV01951 | GCUACCAUGGUGAUGGACAGA GUUAUCGAGGA | 599 | UCCUCGAUAACUCUGUCCA UCACCAUGGUAGC | 693 |
| AV01952 | CAUGGACAGAGUUAUCGAGGA | 600 | UCCUCGAUAACUCUGUCCA UGUU | 694 |
| AV01953 | CAUGGACAGAGUUAUCGAGGA | 601 | UCCUCGAUAACUCUGUCCA UGGA | 695 |
| AV01954 | CAUGGACAGAGUUAUCGAGGA | 602 | UCCUCGAUAACUCUGUCCA UG | 696 |
| AV01955 | CAUGGACAGAGUUAUCGAGGA | 603 | UCCUCGAUAACUCUGUCCA UG | 697 |
| AV01956 | CAUGGACAGAGUUAUCGAGGA | 604 | UCCUCGAUAACUCUGUCCA UG | 698 |
| AV01957 | CAUGGACAGAGUUAUUGAGGA | 605 | UCCUCGAUAACUCUGUCCA UG | 699 |
| AV01958 | CAUGGACAGAGUUAUCGAGAA | 606 | UUCUCGAUAACUCUGUCCA UG | 700 |
| AV01959 | CAUGGACAGAGUUAUCGAGGA | 607 | UCCUCGAUAACUCUGUCCA UG | 701 |

Table 2 shows antisense and sense strand sequences of certain chemically modified LPA RNAi agents of the present invention. In some embodiments of the method of the present invention, RNAi agents having the polynucleotide sequences shown in Table 2 are administered to cells and/or subjects.

In some embodiments of the method of the present invention, RNAi agents having the polynucleotide sequences shown in Table 2 are administered to subjects. In some embodiments of the present invention, the RNAi agent administered to the subject comprises a duplex labeled in the first column of Table 2, and sequence modifications in the sense and antisense strand sequences shown in the third and sixth columns of the same row in Table 2, respectively. In some embodiments of the method of the present invention, the sequences shown in Table 2 may be attached to (also referred to herein as "conjugated to") compounds capable of delivering RNAi agents to the cells and/or tissues of the subject. Non-limiting examples of delivery compounds that may be used in certain embodiments of the present invention are GalNAc-containing compounds. In Table 2, the first column represents the duplex AD # of the base sequence, corresponding to Table 1. The base sequences identified by the duplex AD # not only shows the base sequences contained in the sense and antisense strands but also have the designated chemical modifications shown in the same row of Table 2. For example, the first row of Table 1 shows the sense and antisense base single-stranded sequences, which together form a duplex identified as: duplex AV00122; the duplex AV00122 listed in Table 2, as a duplex, comprises the base sequences of AV00122-SS and AV00122-AS, and comprises chemical modifications in the sense sequences and the antisense sequences shown in the third and sixth columns, respectively. The "sense strand SS#" in the second column of Table 2 is the designated identifier of the sense sequence (including modification) shown in the third column of the same row. The "antisense strand AS #" in the fifth column of Table 2 is the designated identifier of the antisense sequence (including modification) shown in the sixth column.

**Table 2: Antisense and sense strand sequences of chemically modified LPA RNAi agent. All sequences are shown in 5' to 3'. These sequences were used in some of the in vitro test studies described herein. Chemical modifications are indicated as: capital: 2'-fluoro; lowercase: 2'-OMe; phosphorothioate: ***

| Duplex AV# | Sense chain SS# | Sense sequence | SEQ ID NO | Antisense strand AS# | Antisense sequence | SEQ ID NO |
|---|---|---|---|---|---|---|
| AV001 22 | AV00122-SS | c*u*gguccaGgAuUgc uacca*a | 256 | AV00122-AS | u*U*gguaGcaauCcUg Gacc*a*g | 340 |
| AV001 23 | AV00123-SS | c*u*gauggaCaGaGuu aucga*a | 257 | AV00123-AS | u*U*cgauAacucUgUc Cauc*a*g | 341 |
| AV001 24 | AV00124-SS | c*a*uggacaGaGuUauc gagg*a | 258 | AV00124-AS | u*C*cucgAuaacUcUg Ucca*u*g | 342 |
| AV001 25 | AV00125-SS | g*c*cugccaAgCuUgg ucauc*a | 259 | AV00125-AS | u*G*augaCcaagCuUg Gcag*g*c | 343 |
| AV001 26 | AV00126-SS | g*c*ugccaaGcUuGgu caucu*a | 260 | AV00126-AS | u*A*gaugAccaaGcUu Ggca*g*c | 344 |
| AV001 27 | AV00127-SS | c*c*caagcuUgGuCauc uaug*a | 261 | AV00127-AS | u*C*auagAugacCaAg Cuug*g*g | 345 |
| AV001 28 | AV00128-SS | g*a*agcuugGuCaUcu augac*a | 262 | AV00128-AS | u*G*ucauAgaugAcCa Agcu*u*c | 346 |
| AV001 29 | AV00129-SS | g*a*cuacccAaAuGcug gcuu*a | 263 | AV00129-AS | u*A*agccAgcauUuGg Guag*u*c | 347 |
| AV001 30 | AV00130-SS | g*u*acccaaAuGcUggc uuga*a | 264 | AV00130-AS | u*U*caagCcagcAuUu Gggu*a*c | 348 |
| AV001 31 | AV00131-SS | g*a*cccaaaUgCuGgcu ugau*a | 265 | AV00131-AS | u*A*ucaaGccagCaUu Uggg*u*c | 349 |
| AV001 32 | AV00132-SS | g*g*cuccuuAuUgUua uacga*a | 266 | AV00132-AS | u*U*cguaUaacaAuAa Ggag*c*c | 350 |
| AV001 33 | AV00133-SS | g*g*ccuagaGgCuCcu uccga*a | 267 | AV00133-AS | u*U*cggaAggagCcUc Uagg*c*c | 351 |
| AV00134 | AV00134-SS | g*u*agaggcUcCuUcc | 268 | AV00134-AS | u*U*guucGgaagGaGc | 352 |
| | | gaaca*a | | | Cucu*a*c | |
| AV001 35 | AV00135-SS | g*a*agcaccGaCuGagc aaag*a | 269 | AV00135-AS | u*C*uuugCucagUcGg Ugcu*u*c | 353 |
| AV001 36 | AV00136-SS | c*g*agugcuAcCaUgg uaaug*a | 270 | AV00136-AS | u*C*auuaCcaugGuAg Cacu*c*g | 354 |
| AV001 37 | AV00137-SS | g*g*ugcuacCaUgGua augga*a | 271 | AV00137-AS | u*U*ccauUaccaUgGu Agca*c*c | 355 |
| AV001 38 | AV00138-SS | g*a*ccauggUaAuGga cagag*a | 272 | AV00138-AS | u*C*ucugUccauUaCc Augg*u*c | 356 |
| AV001 39 | AV00139-SS | g*u*gguaauGgAcAga guuau*a | 273 | AV00139-AS | u*A*uaacUcuguCcAu Uacc*a*c | 357 |
| AV001 40 | AV00140-SS | g*g*guaaugGaCaGag uuauc*a | 274 | AV00140-AS | u*G*auaaCucugUcCa Uuac*c*c | 358 |
| AV001 41 | AV00141-SS | c*u*aauggaCaGaGuua ucga*a | 275 | AV00141-AS | u*U*cgauAacucUgUc Cauu*a*g | 359 |
| AV001 42 | AV00142-SS | g*a*gaguuaUcGaGgc acaua*a | 276 | AV00142-AS | u*U*auguGccucGaUa Acuc*u*c | 360 |
| AV001 43 | AV00143-SS | c*a*guuaucGaGgCaca uacu*a | 277 | AV00143-AS | u*A*guauGugccUcGa Uaac*u*g | 361 |
| AV001 44 | AV00144-SS | g*a*ccacggUaAuGgac agag*a | 278 | AV00144-AS | u*C*ucugUccauUaCc Gugg*u*c | 362 |
| AV001 45 | AV00145-SS | g*a*cugcagGaAuCcag aucc*a | 279 | AV00145-AS | u*G*gaucUggauUcCu Gcag*u*c | 363 |
| AV001 46 | AV00146-SS | g*g*gcagccCcUuAuu guuau*a | 280 | AV00146-AS | u*A*uaacAauaaGgGg Cugc*c*c | 364 |
| AV001 47 | AV00147-SS | g*a*gccccuUaUuGuu auacg*a | 281 | AV00147-AS | u*C*guauAacaaUaAg Gggc*u*c | 365 |
| AV001 48 | AV00148-SS | g*c*agugucAgGuGgg | 282 | AV00148-AS | u*G*uacuCccacCuGa | 366 |
| | | aguac*a | | | Cacu*g*c | |
| AV001 49 | AV00149-SS | c*u*acugcaAcCuGaca caau*a | 283 | AV00149-AS | u*A*uuguGucagGuUg Cagu*a*g | 367 |
| AV001 50 | AV00150-SS | g*g*acacaaUgCuCaga cgca*a | 284 | AV00150-AS | u*U*gcguCugagCaUu Gugu*c*c | 368 |
| AV001 51 | AV00151-SS | g*u*agaggcUcCuUcu gaaca*a | 285 | AV00151-AS | u*U*guucAgaagGaGc Cucu*a*c | 369 |
| AV001 52 | AV00152-SS | c*a*ggcuccUuCuGaac aagc*a | 286 | AV00152-AS | u*G*cuugUucagAaGg Agcc*u*g | 370 |
| AV001 53 | AV00153-SS | g*a*agcaccAaCuGagc aaag*a | 287 | AV00153-AS | u*C*uuugCucagUuGg Ugcu*u*c | 371 |
| AV001 54 | AV00154-SS | g*g*gaaaugGaCaGag uuauc*a | 288 | AV00154-AS | u*G*auaaCucugUcCa Uuuc*c*c | 372 |
| AV001 55 | AV00155-SS | c*a*aauggaCaGaGuua ucaa*a | 289 | AV00155-AS | u*U*ugauAacucUgUc Cauu*u*g | 373 |
| AV001 56 | AV00156-SS | g*g*gacagaGuUaUcaa ggca*a | 290 | AV00156-AS | u*U*gccuUgauaAcUc Uguc*c*c | 374 |
| AV001 57 | AV00157-SS | g*u*caagaaCuAcUgcc gaaa*a | 291 | AV00157-AS | u*U*uucgGcaguAgUu Cuug*a*c | 375 |
| AV001 58 | AV00158-SS | g*c*aagaacUaCuGccg aaau*a | 292 | AV00158-AS | u*A*uuucGgcagUaGu Ucuu*g*c | 376 |
| AV001 59 | AV00159-SS | g*a*agaacuAcUgCcga aauc*a | 293 | AV00159-AS | u*G*auuuCggcaGuAg Uucu*u*c | 377 |
| AV001 60 | AV00160-SS | g*a*cugccgAaAuCcag aucc*a | 294 | AV00160-AS | u*G*gaucUggauUuCg Gcag*u*c | 378 |
| AV001 61 | AV00161-SS | c*u*acugcaAcCuGaca cgau*a | 295 | AV00161-AS | u*A*ucguGucagGuUg Cagu*a*g | 379 |
| AV00162 | AV00162-SS | g*g*acacgaUgCuCaga | 296 | AV00162-AS | u*U*gcauCugagCaUc | 380 |
| | | ugca*a | | | Gugu*c*c | |
| AV001 63 | AV00163-SS | c*g*acugcuAcUaCcau uaug*a | 297 | AV00163-AS | u*C*auaaUgguaGuAg Cagu*c*g | 381 |
| AV001 64 | AV00164-SS | c*a*cugcuaCuAcCauu augg*a | 298 | AV00164-AS | u*C*cauaAugguAgUa Gcag*u*g | 382 |
| AV001 65 | AV00165-SS | g*c*ugcuacUaCcAuua ugga*a | 299 | AV00165-AS | u*U*ccauAauggUaGu Agca*g*c | 383 |
| AV001 66 | AV00166-SS | g*a*cuaccaUuAuGgac agag*a | 300 | AV00166-AS | u*C*ucugUccauAaUg Guag*u*c | 384 |
| AV001 67 | AV00167-SS | g*c*uugccaAgCuUgg ucauc*a | 301 | AV00167-AS | u*G*augaCcaagCuUg Gcaa*g*c | 385 |
| AV001 68 | AV00168-SS | g*u*ugccaaGcUuGgu caucu*a | 302 | AV00168-AS | u*A*gaugAccaaGcUu Ggca*a*c | 386 |
| AV001 69 | AV00169-SS | g*a*uagucgGaCcCcag aaaa*a | 303 | AV00169-AS | u*U*uuucUggggUcCg Acua*u*c | 387 |
| AV001 70 | AV00170-SS | g*c*aggaacUaCuGcag gaau*a | 304 | AV00170-AS | u*A*uuccUgcagUaGu Uccu*g*c | 388 |
| AV001 71 | AV00171-SS | c*a*auccagAuGcUgag auuc*a | 305 | AV00171-AS | u*G*aaucUcagcAuCu Ggau*u*g | 389 |
| AV001 72 | AV00172-SS | c*a*gauucgCcCuUgg uguua*a | 306 | AV00172-AS | u*U*aacaCcaagGgCg Aauc*u*g | 390 |
| AV001 73 | AV00173-SS | c*c*ccuuggUgUuAca ccaug*a | 307 | AV00173-AS | u*C*auggUguaaCaCc Aagg*g*g | 391 |
| AV001 74 | AV00174-SS | g*u*ggugacAgAaUca agugu*a | 308 | AV00174-AS | u*A*cacuUgauuCuGu Cacc*a*c | 392 |
| AV001 75 | AV00175-SS | g*a*ucaaguGuCcUug caacu*a | 309 | AV00175-AS | u*A*guugCaaggAcAc Uuga*u*c | 393 |
| AV00176 | AV00176-SS | c*g*gguccaGgAuUgc | 310 | AV00176-AS | u*U*gguaGcaauCcUg | 394 |
| | | uacca*a | | | Gacc*c*g | |
| AV001 77 | AV00177-SS | g*u*gucacaGgAaGga caugu*a | 311 | AV00177-AS | u*A*caugUccuuCcUg Ugac*a*c | 395 |
| AV001 78 | AV00178-SS | c*g*aaggacAuGuCag ucuug*a | 312 | AV00178-AS | u*C*aagaCugacAuGu Ccuu*c*g | 396 |
| AV001 79 | AV00179-SS | c*a*aggacaUgUcAguc uugg*a | 313 | AV00179-AS | u*C*caagAcugaCaUg Uccu*u*g | 397 |
| AV001 80 | AV00180-SS | c*u*cagucuUgGuCcu cuaug*a | 314 | AV00180-AS | u*C*auagAggacCaAg Acug*a*g | 398 |
| AV001 81 | AV00181-SS | c*u*ccuuggUgUuAua ccaug*a | 315 | AV00181-AS | u*C*auggUauaaCaCc Aagg*a*g | 399 |
| AV001 82 | AV00182-SS | g*u*gguguuAuAcCau ggauc*a | 316 | AV00182-AS | u*G*auccAugguAuAa Cacc*a*c | 400 |
| AV001 83 | AV00183-SS | g*g*guguuaUaCcAug gaucc*a | 317 | AV00183-AS | u*G*gaucCauggUaUa Acac*c*c | 401 |
| AV001 84 | AV00184-SS | c*u*uauaccAuGgAuc ccaau*a | 318 | AV00184-AS | u*A*uuggGauccAuGg Uaua*a*g | 402 |
| AV001 85 | AV00185-SS | g*a*uaccauGgAuCcca augu*a | 319 | AV00185-AS | u*A*cauuGggauCcAu Ggua*u*c | 403 |
| AV001 86 | AV00186-SS | g*c*cugacaCaAuGucc agug*a | 320 | AV00186-AS | u*C*acugGacauUgUg Ucag*g*c | 404 |
| AV001 87 | AV00187-SS | g*g*acacaaUgUcCagu gaca*a | 321 | AV00187-AS | u*U*gucaCuggaCaUu Gugu*c*c | 405 |
| AV001 88 | AV00188-SS | g*a*auguccAgUgAca gaauc*a | 322 | AV00188-AS | u*G*auucUgucaCuGg Acau*u*c | 406 |
| AV001 89 | AV00189-SS | g*a*uguccaGuGaCaga auca*a | 323 | AV00189-AS | u*U*gauuCugucAcUg Gaca*u*c | 407 |
| AV00190 | AV00190-SS | g*u*guccagUgAcAga | 324 | AV00190-AS | u*U*ugauUcuguCaCu | 408 |
| | | aucaa*a | | | Ggac*a*c | |
| AV001 91 | AV00191-SS | g*g*uccaguGaCaGaau caag*a | 325 | AV00191-AS | u*C*uugaUucugUcAc Ugga*c*c | 409 |
| AV001 92 | AV00192-SS | g*c*agugacAgAaUcaa gugu*a | 326 | AV00192-AS | u*A*cacuUgauuCuGu Cacu*g*c | 410 |
| AV001 93 | AV00193-SS | g*a*uucuccAcCaCugu uaca*a | 327 | AV00193-AS | u*U*guaaCagugGuGg Agaa*u*c | 411 |
| AV001 94 | AV00194-SS | g*c*caccacUgUuAcag gaag*a | 328 | AV00194-AS | u*C*uuccUguaaCaGu Ggug*g*c | 412 |
| AV001 95 | AV00195-SS | g*a*cagaauAcUaCcca aaug*a | 329 | AV00195-AS | u*C*auuuGgguaGuAu Ucug*u*c | 413 |
| AV001 96 | AV00196-SS | g*c*agaauaCuAcCcaa augg*a | 330 | AV00196-AS | u*C*cauuUggguAgUa Uucu*g*c | 414 |
| AV001 97 | AV00197-SS | g*g*auucgcCcUuGgu guuau*a | 331 | AV00197-AS | u*A*uaacAccaaGgGc Gaau*c*c | 415 |
| AV001 98 | AV00198-SS | c*a*uucgccCuUgGug uuaua*a | 332 | AV00198-AS | u*U*auaaCaccaAgGg Cgaa*u*g | 416 |
| AV001 99 | AV00199-SS | g*u*cgcccuUgGuGuu auacc*a | 333 | AV00199-AS | u*G*guauAacacCaAg Ggcg*a*c | 417 |
| AV002 00 | AV00200-SS | c*c*ccuuggUgUuAua ccaug*a | 334 | AV00200-AS | u*C*auggUauaaCaCc Aagg*g*g | 418 |
| AV002 01 | AV00201-SS | g*g*auguccAgUgAca gaauc*a | 335 | AV00201-AS | u*G*auucUgucaCuGg Acau*c*c | 419 |
| AV002 02 | AV00202-SS | g*c*agaggcUcCuUcu gaaca*a | 336 | AV00202-AS | u*U*guucAgaagGaGc Cucu*g*c | 420 |
| AV002 03 | AV00203-SS | g*a*ucuuggUcAuCua ugaua*a | 337 | AV00203-AS | u*U*aucaUagauGaCc Aaga*u*c | 421 |
| AV00204 | AV00204-SS | g*g*acacaaUgCuCaga | 338 | AV00204-AS | u*U*guuuCugagCaUu | 422 |
| | | aaca*a | | | Gugu*c*c | |
| AV002 05 | AV00205-SS | c*c*agugcuAcCaUgg uaaug*a | 339 | AV00205-AS | u*C*auuaCcaugGuAg Cacu*g*g | 423 |
| AV018 67 | AV01867-SS | g*a*gaguuaUcGaGgc acaua*a | 702 | AV01867-AS | u*U*auguGccucGaUa Acuc*u*c | 795 |
| AV018 68 | AV01868-SS | g*a*gaguuaUcGaGgc acaua*u | 703 | AV01868-AS | a*U*auguGccucGaUa Acuc*u*c | 796 |
| AV018 69 | AV01869-SS | g*a*gaguuaUcGaGgc acaua*c | 704 | AV01869-AS | g*U*auguGccucGaUa Acuc*u*c | 797 |
| AV018 70 | AV01870-SS | g*a*gaguuaUcGaGgc acaua*g | 705 | AV01870-AS | c*U*auguGccucGaUa Acuc*u*c | 798 |
| AV018 71 | AV01871-SS | g*u*uaUcGaGgcacaua *a | 706 | AV01871-AS | u*U*auguGccucGaUa *A*c | 799 |
| AV018 72 | AV01872-SS | a*g*uuaUcGaGgcacau a*a | 707 | AV01872-AS | u*U*auguGccucGaUa A*c*u | 800 |
| AV018 73 | AV01873-SS | a*a*guuaUcGaGgcaca ua*a | 708 | AV01873-AS | u*U*auguGccucGaUa Ac*u*u | 801 |
| AV018 74 | AV01874-SS | u*a*guuaUcGaGgcaca ua*a | 709 | AV01874-AS | u*U*auguGccucGaUa Ac*u*a | 802 |
| AV018 75 | AV01875-SS | g*a*guuaUcGaGgcaca ua*a | 710 | AV01875-AS | u*U*auguGccucGaUa Ac*u*c | 803 |
| AV018 76 | AV01876-SS | c*a*guuaUcGaGgcaca ua*a | 711 | AV01876-AS | u*U*auguGccucGaUa Ac*u*g | 804 |
| AV018 77 | AV01877-SS | a*g*aguuaUcGaGgcac aua*a | 712 | AV01877-AS | u*U*auguGccucGaUa Acu*c*u | 805 |
| AV018 78 | AV01878-SS | u*g*aguuaUcGaGgca caua*a | 713 | AV01878-AS | u*U*auguGccucGaUa Acu*c*a | 806 |
| AV018 79 | AV01879-SS | g*g*aguuaUcGaGgca caua*a | 714 | AV01879-AS | u*U*auguGccucGaUa Acu*c*c | 807 |
| AV018 80 | AV01880-SS | c*g*aguuaUcGaGgcac aua*a | 715 | AV01880-AS | u*U*auguGccucGaUa Acu*c*g | 808 |
| AV018 81 | AV01881-SS | u*a*gaguuaUcGaGgc acaua*a | 716 | AV01881-AS | u*U*auguGccucGaUa Acuc*u*a | 809 |
| AV018 82 | AV01882-SS | c*a*gaguuaUcGaGgca caua*a | 717 | AV01882-AS | u*U*auguGccucGaUa Acuc*u*g | 810 |
| AV018 83 | AV01883-SS | a*a*gaguuaUcGaGgca caua*a | 718 | AV01883-AS | u*U*auguGccucGaUa Acuc*u*u | 811 |
| AV018 84 | AV01884-SS | a*c*agaguuaUcGaGgc acaua*a | 719 | AV01884-AS | u*U*auguGccucGaUa Acucu*g*u | 812 |
| AV018 85 | AV01885-SS | g*a*cagaguuaUcGaG gcacaua*a | 720 | AV01885-AS | u*U*auguGccucGaUa Acucug*u*c | 813 |
| AV018 86 | AV01886-SS | g*g*acagaguuaUcGa Ggcacaua*a | 721 | AV01886-AS | u*U*auguGccucGaUa Acucugu*c*c | 814 |
| AV018 87 | AV01887-SS | u*g*gacagaguuaUcGa Ggcacaua*a | 722 | AV01887-AS | u*U*auguGccucGaUa Acucuguc*c*a | 815 |
| AV018 88 | AV01888-SS | a*u*ggacagaguuaUcG aGgcacaua*a | 723 | AV01888-AS | u*U*auguGccucGaUa Acucugucc*a*u | 816 |
| AV018 89 | AV01889-SS | a*u*gguaauggacagagu uaUcGaGgcacaua*a | 724 | AV01889-AS | u*U*auguGccucGaUa Acucuguccauuacc*a*u | 817 |
| AV018 90 | AV01890-SS | g*a*gaguuaUcGaGgc acaua*a | 725 | AV01890-AS | u*U*auguGccucGaUa Acucuc*u*u | 818 |
| AV018 91 | AV01891-SS | g*a*gaguuaUcGaGgc acaua*a | 726 | AV01891-AS | u*U*auguGccucGaUa Acucuc*g*a | 819 |
| AV018 92 | AV01892-SS | g*a*gaguuaUcGaGgc acaua*a | 727 | AV01892-AS | u*U*aug(uUNA)Gccu cGaUaAcuc*u*c | 820 |
| AV018 93 | AV01893-SS | g*a*gaguuaUcGaGgc acaua*a | 728 | AV01893-AS | u*U*augu(gUNA)ccuc GaUaAcuc*u*c | 821 |
| AV018 94 | AV01894-SS | g*a*gaguuaUCGaggc acaua*a | 729 | AV01894-AS | u*U*augUgccucgaUa Acuc*u*c | 822 |
| AV018 95 | AV01895-SS | g*a*gaguuaUcGaGgc gcaua*a | 730 | AV01895-AS | u*U*auguGccucGaUa Acuc*u*c | 823 |
| AV018 96 | AV01896-SS | g*a*gaguuaUcGaGgc acauu*a | 731 | AV01896-AS | u*A*auguGccucGaUa Acuc*u*c | 824 |
| AV018 97 | AV01897-SS | g*a*gaguuaUcGaGgc acau*a*a | 732 | AV01897-AS | u*U*auguGccucGaUa Acuc*u*c | 825 |
| AV018 98 | AV01898-SS | g*u*ugccaaGcUuGgu caucu*a | 733 | AV01898-AS | u*A*gaugAccaaGcUu Ggca*a*c | 826 |
| AV018 99 | AV01899-SS | g*u*ugccaaGcUuGgu caucu*u | 734 | AV01899-AS | a*A*gaugAccaaGcUu Ggca*a*c | 827 |
| AV019 00 | AV01900-SS | g*u*ugccaaGcUuGgu caucu*c | 735 | AV01900-AS | g*A*gaugAccaaGcUu Ggca*a*c | 828 |
| AV019 01 | AV01901-SS | g*u*ugccaaGcUuGgu caucu*g | 736 | AV01901-AS | c*A*gaugAccaaGcUu Ggca*a*c | 829 |
| AV019 02 | AV01902-SS | c*c*aaGcUuGgucaucu *a | 737 | AV01902-AS | u*A*gaugAccaaGcUu *G*g | 830 |
| AV019 03 | AV01903-SS | g*c*caaGcUuGgucauc u*a | 738 | AV01903-AS | u*A*gaugAccaaGcUu G*g*c | 831 |
| AV019 04 | AV01904-SS | a*g*ccaaGcUuGgucau cu*a | 739 | AV01904-AS | u*A*gaugAccaaGcUu Gg*c*u | 832 |
| AV019 05 | AV01905-SS | u*g*ccaaGcUuGguca ucu*a | 740 | AV01905-AS | u*A*gaugAccaaGcUu Gg*c*a | 833 |
| AV019 06 | AV01906-SS | g*g*ccaaGcUuGguca ucu*a | 741 | AV01906-AS | u*A*gaugAccaaGcUu Gg*c*c | 834 |
| AV019 07 | AV01907-SS | c*g*ccaaGcUuGgucau cu*a | 742 | AV01907-AS | u*A*gaugAccaaGcUu Gg*c*g | 835 |
| AV019 08 | AV01908-SS | a*u*gccaaGcUuGguca ucu*a | 743 | AV01908-AS | u*A*gaugAccaaGcUu Ggc*a*u | 836 |
| AV019 09 | AV01909-SS | u*u*gccaaGcUuGguc aucu*a | 744 | AV01909-AS | u*A*gaugAccaaGcUu Ggc*a*a | 837 |
| AV019 10 | AV01910-SS | g*u*gccaaGcUuGguc aucu*a | 745 | AV01910-AS | u*A*gaugAccaaGcUu Ggc*a*c | 838 |
| AV019 11 | AV01911-SS | c*u*gccaaGcUuGguca ucu*a | 746 | AV01911-AS | u*A*gaugAccaaGcUu Ggc*a*g | 839 |
| AV019 12 | AV01912-SS | u*u*ugccaaGcUuGgu caucu*a | 747 | AV01912-AS | u*A*gaugAccaaGcUu Ggca*a*a | 840 |
| AV019 13 | AV01913-SS | c*u*ugccaaGcUuGgu caucu*a | 748 | AV01913-AS | u*A*gaugAccaaGcUu Ggca*a*g | 841 |
| AV019 14 | AV01914-SS | a*u*ugccaaGcUuGgu caucu*a | 749 | AV01914-AS | u*A*gaugAccaaGcUu Ggca*a*u | 842 |
| AV019 15 | AV01915-SS | a*c*uugccaaGcUuGg ucaucu*a | 750 | AV01915-AS | u*A*gaugAccaaGcUu Ggcaa*g*u | 843 |
| AV019 16 | AV01916-SS | a*a*cuugccaaGcUuGg ucaucu*a | 751 | AV01916-AS | u*A*gaugAccaaGcUu Ggcaag*u*u | 844 |
| AV019 17 | AV01917-SS | g*a*acuugccaaGcUuG gucaucu*a | 752 | AV01917-AS | u*A*gaugAccaaGcUu Ggcaagu*u*c | 845 |
| AV019 18 | AV01918-SS | a*g*aacuugccaaGcUu Ggucaucu*a | 753 | AV01918-AS | u*A*gaugAccaaGcUu Ggcaaguu*c*u | 846 |
| AV019 19 | AV01919-SS | a*a*gaacuugccaaGcU uGgucaucu*a | 754 | AV01919-AS | u*A*gaugAccaaGcUu Ggcaaguuc*u*u | 847 |
| AV019 20 | AV01920-SS | c*a*caggaagaacuugcc aaGcUuGgucaucu*a | 755 | AV01920-AS | u*A*gaugAccaaGcUu Ggcaaguucuuccug*u*g | 848 |
| AV019 21 | AV01921-SS | g*u*ugccaaGcUuGgu caucu*a | 756 | AV01921-AS | u*A*gaugAccaaGcUu Ggcaac*u*u | 849 |
| AV019 22 | AV01922-SS | g*u*ugccaaGcUuGgu caucu*a | 757 | AV01922-AS | u*A*gaugAccaaGcUu Ggcaac*g*a | 850 |
| AV019 23 | AV01923-SS | g*u*ugccaaGcUuGgu caucu*a | 758 | AV01923-AS | u*A*gau(gUNA)Accaa GcUuGgca*a*c | 851 |
| AV019 24 | AV01924-SS | g*u*ugccaaGcUuGgu caucu*a | 759 | AV01924-AS | u*A*gaug(aUNA)ccaa GcUuGgca*a*c | 852 |
| AV019 25 | AV01925-SS | g*u*ugccaaGCUuggu caucu*a | 760 | AV01925-AS | u*A*gauGaccaagcUuG gca*a*c | 853 |
| AV019 26 | AV01926-SS | g*u*ugccaaGcUuGgu uaucu*a | 761 | AV01926-AS | u*A*gaugAccaaGcUu Ggca*a*c | 854 |
| AV019 27 | AV01927-SS | g*u*ugccaaGcUuGgu caucc*a | 762 | AV01927-AS | u*G*gaugAccaaGcUu Ggca*a*c | 855 |
| AV019 28 | AV01928-SS | g*u*ugccaaGcUuGgu cauc*u*a | 763 | AV01928-AS | u*A*gaugAccaaGcUu Ggca*a*c | 856 |
| AV019 29 | AV01929-SS | c*a*uggacaGaGuUauc gagg*a | 764 | AV01929-AS | u*C*cucgAuaacUcUg Ucca*u*g | 857 |
| AV019 30 | AV01930-SS | c*a*uggacaGaGuUauc gagg*u | 765 | AV01930-AS | a*C*cucgAuaacUcUg Ucca*u*g | 858 |
| AV019 31 | AV01931-SS | c*a*uggacaGaGuUauc gagg*c | 766 | AV01931-AS | g*C*cucgAuaacUcUg Ucca*u*g | 859 |
| AV019 32 | AV01932-SS | c*a*uggacaGaGuUauc gagg*g | 767 | AV01932-AS | c*C*cucgAuaacUcUg Ucca*u*g | 860 |
| AV019 33 | AV01933-SS | g*a*caGaGuUaucgag g*a | 768 | AV01933-AS | u*C*cucgAuaacUcUg* U*c | 861 |
| AV019 34 | AV01934-SS | g*g*acaGaGuUaucga gg*a | 769 | AV01934-AS | u*C*cucgAuaacUcUg U*c*c | 862 |
| AV019 35 | AV01935-SS | a*g*gacaGaGuUaucga gg*a | 770 | AV01935-AS | u*C*cucgAuaacUcUg Uc*c*u | 863 |
| AV019 36 | AV01936-SS | u*g*gacaGaGuUaucg agg*a | 771 | AV01936-AS | u*C*cucgAuaacUcUg Uc*c*a | 864 |
| AV019 37 | AV01937-SS | g*g*gacaGaGuUaucg agg*a | 772 | AV01937-AS | u*C*cucgAuaacUcUg Uc*c*c | 865 |
| AV019 38 | AV01938-SS | c*g*gacaGaGuUaucga gg*a | 773 | AV01938-AS | u*C*cucgAuaacUcUg Uc*c*g | 866 |
| AV019 39 | AV01939-SS | a*u*ggacaGaGuUauc gagg*a | 774 | AV01939-AS | u*C*cucgAuaacUcUg Ucc*a*u | 867 |
| AV019 40 | AV01940-SS | u*u*ggacaGaGuUauc gagg*a | 775 | AV01940-AS | u*C*cucgAuaacUcUg Ucc*a*a | 868 |
| AV019 41 | AV01941-SS | g*u*ggacaGaGuUauc gagg*a | 776 | AV01941-AS | u*C*cucgAuaacUcUg Ucc*a*c | 869 |
| AV019 42 | AV01942-SS | c*u*ggacaGaGuUauc gagg*a | 777 | AV01942-AS | u*C*cucgAuaacUcUg Ucc*a*g | 870 |
| AV019 43 | AV01943-SS | u*a*uggacaGaGuUau cgagg*a | 778 | AV01943-AS | u*C*cucgAuaacUcUg Ucca*u*a | 871 |
| AV019 44 | AV01944-SS | g*a*uggacaGaGuUau cgagg*a | 779 | AV01944-AS | u*C*cucgAuaacUcUg Ucca*u*c | 872 |
| AV019 45 | AV01945-SS | a*a*uggacaGaGuUauc gagg*a | 780 | AV01945-AS | u*C*cucgAuaacUcUg Ucca*u*u | 873 |
| AV019 46 | AV01946-SS | u*g*auggacaGaGuUa ucgagg*a | 781 | AV01946-AS | u*C*cucgAuaacUcUg Uccau*c*a | 874 |
| AV019 47 | AV01947-SS | g*u*gauggacaGaGuU aucgagg*a | 782 | AV01947-AS | u*C*cucgAuaacUcUg Uccauc*a*c | 875 |
| AV019 48 | AV01948-SS | g*g*ugauggacaGaGu Uaucgagg*a | 783 | AV01948-AS | u*C*cucgAuaacUcUg Uccauca*c*c | 876 |
| AV019 49 | AV01949-SS | u*g*gugauggacaGaGu Uaucgagg*a | 784 | AV01949-AS | u*C*cucgAuaacUcUg Uccaucac*c*a | 877 |
| AV019 50 | AV01950-SS | a*u*ggugauggacaGaG uUaucgagg*a | 785 | AV01950-AS | u*C*cucgAuaacUcUg Uccaucacc*a*u | 878 |
| AV019 51 | AV01951-SS | g*c*uaccauggugaugga caGaGuUaucgagg*a | 786 | AV01951-AS | u*C*cucgAuaacUcUg Uccaucaccauggua*g*c | 879 |
| AV019 52 | AV01952-SS | c*a*uggacaGaGuUauc gagg*a | 787 | AV01952-AS | u*C*cucgAuaacUcUg Uccaug*u*u | 880 |
| AV019 53 | AV01953-SS | c*a*uggacaGaGuUauc gagg*a | 788 | AV01953-AS | u*C*cucgAuaacUcUg Uccaug*g*a | 881 |
| AV019 54 | AV01954-SS | c*a*uggacaGaGuUauc gagg*a | 789 | AV01954-AS | u*C*cuc(gUNA)Auaac UcUgUcca*u*g | 882 |
| AV019 55 | AV01955-SS | c*a*uggacaGaGuUauc gagg*a | 790 | AV01955-AS | u*C*cucg(aUNA)uaac UcUgUcca*u*g | 883 |
| AV019 56 | AV01956-SS | c*a*uggacaGAGuuau cgagg*a | 791 | AV01956-AS | u*C*cucGauaacucUgU cca*u*g | 884 |
| AV019 57 | AV01957-SS | c*a*uggacaGaGuUau ugagg*a | 792 | AV01957-AS | u*C*cucgAuaacUcUg Ucca*u*g | 885 |
| AV019 58 | AV01958-SS | c*a*uggacaGaGuUauc gaga*a | 793 | AV01958-AS | u*U*cucgAuaacUcUg Ucca*u*g | 886 |
| AV019 59 | AV01959-SS | c*a*uggacaGaGuUauc gag*g*a | 794 | AV01959-AS | u*C*cucgAuaacUcUg Ucca*u*g | 887 |

Table 3 shows antisense and sense strand sequences of certain chemically modified LPA RNAi agents of the present invention. In some embodiments of the method of the present invention, the RNAi agents shown in Table 3 are administered to cells and/or subjects. In some embodiments of the method of the present invention, RNAi agents having the polynucleotide sequences shown in Table 3 are administered to subjects. In some embodiments of the present invention, the RNAi agent administered to the subject comprises the duplex identified in the first column of Table 3 and comprises the sequence modifications and/or delivery compounds shown in the sense and antisense strand sequences in the third and sixth columns of the same row in Table 3, respectively. This sequence is used in some in vivo test studies described elsewhere herein. In some embodiments of the method of the present invention, the sequences shown in Table 3 may be linked (also referred to herein as "conjugated to") to compounds for delivery, the non-limiting example of which is GalNAc-containing compounds, i.e., having the compound for delivery labelled "GLX-n" on the sense strand of the third column in Table 3. As used herein and shown in Table 3, "GLX-n" is used to denote the linked GalNAc-containing compound, which is any of the compounds GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15 and GLO-16. The structure of each of these is provided elsewhere herein. The first column of Table 3 provides the duplex AD # assigned to the duplex of the sense and antisense sequences in the row in the table. For example, the duplex AD00122 is a duplex composed of the sense strand AD00122-SS and the antisense strand AD00122-AS. Each row in Table 3 provides a sense strand and an antisense strand, and discloses the duplex formed by the indicated sense strand and antisense strand. The "sense strand SS#" in the second column of Table 3 is the designated identifier of the sense sequence (including modification) shown in the third column of the same row. The "antisense strand AS #" in the fifth column of Table 3 is the designated identifier of the antisense sequence (including modification) shown in the sixth column. Certain identifiers of linked GalNAc-containing GLO compounds are shown as GLO-0, and it should be understood that another of the GLO-n or GLS-n compounds may be substituted for the compound shown as GLO-0, and the resulting compound is also included in embodiments of the methods and/or compositions of the present invention.

Table 3 provides antisense and sense strand sequences of chemically modified LPARNAi agent for in vivo test. All sequences are shown in 5' to 3'. These sequences are used in some in vivo test studies described elsewhere herein. The delivery molecule used in the in vivo study is denoted as "GLO-0" at the 3'-end of each sense strand. The delivery molecule used in the in vivo study is denoted as "GLS-5" or "GLS-15" at the 5'-end of each sense strand. Chemical modifications are indicated as: capital: 2 '-fluoro; lowercase: 2'-OMe; phosphorothioate: *; unlocked nucleic acid: UNA; invab = reverse abasic; imann: when at the end of each strand : 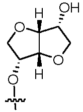 or when further coupling to a delivery molecule:

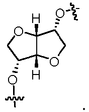

| Duple x AD# | Sense chain SS# | Sense sequence | SEQ ID NO | Antise nse strand AS# | Antisense sequence | SEQ ID NO |
|---|---|---|---|---|---|---|
| AD00 149 | AD00 149-SS | c*a*gccccuUaUuGuuauacg*a( GLO-0) | 424 | AD00 149-AS | u*C*guauAacaaUaAg Gggc*u*g | 469 |
| AD00 150 | AD00 150-SS | a*g*uuaucgAgGcUcauucuc*a( GLO-0) | 425 | AD00 150-AS | u*G*agaaUgagcCuCg Auaa*c*u | 470 |
| AD00 151 | AD00 151-SS | u*g*acacaaUgCuCagacgca*a( GLO-0) | 426 | AD00 151-AS | u*U*gcguCugagCaUu Gugu*c*a | 471 |
| AD00 331 | AD00 331-SS | g*a*cccaaaUgCuGgcuugau*a( GLO-0) | 427 | AD00 331-AS | u*A*ucaaGccagCaUu Uggg*u*c | 472 |
| AD00 332 | AD00 332-SS | g*g*guaaugGaCaGaguuauc*a( GLO-0) | 428 | AD00 332-AS | u*G*auaaCucugUcCa Uuac*c*c | 473 |
| AD00 333 | AD00 333-SS | c*u*aauggaCaGaGuuaucga*a( GLO-0) | 429 | AD00 333-AS | u*U*cgauAacucUgUc Cauu*a*g | 474 |
| AD00 334 | AD00 334-SS | c*a*guuaucGaGgCacauacu*a( GLO-0) | 430 | AD00 334-AS | u*A*guauGugccUcGa Uaac*u*g | 475 |
| AD00 335 | AD00 335-SS | c*u*acugcaAcCuGacacaau*a( GLO-0) | 431 | AD00 335-AS | u*A*uuguGucagGuUg Cagu*a*g | 476 |
| AD00 336 | AD00 336-SS | c*g*acugcuAcUaCcauuaug*a( GLO-0) | 432 | AD00 336-AS | u*C*auaaUgguaGuAg Cagu*c*g | 477 |
| AD00 337 | AD00 337-SS | g*u*ugccaaGcUuGgucaucu*a( GLO-0) | 433 | AD00 337-AS | u*A*gaugAccaaGcUu Ggca*a*c | 478 |
| AD00 338 | AD00 338-SS | c*a*gauucgCcCuUgguguua*a( GLO-0) | 434 | AD00 338-AS | u*U*aacaCcaagGgCg Aauc*u*g | 479 |
| AD00 339 | AD00 339-SS | c*g*aaggacAuGuCagucuug*a( GLO-0) | 435 | AD00 339-AS | u*C*aagaCugacAuGu Ccuu*c*g | 480 |
| AD00 340 | AD00 340-SS | c*u*ccuuggUgUuAuaccaug*a( GLO-0) | 436 | AD00 340-AS | u*C*auggUauaaCaCc Aagg*a*g | 481 |
| AD00 341 | AD00 341-SS | c*c*ccuuggUgUuAuaccaug*a( GLO-0) | 437 | AD00 341-AS | u*C*auggUauaaCaCc Aagg*g*g | 482 |
| AD00 342 | AD00 342-SS | g*g*acacaaUgCuCagaaaca*a( GLO-0) | 438 | AD00 342-AS | u*U*guuuCugagCaUu Gugu*c*c | 483 |
| AD00 433 | AD00 433-SS | (GLS- 5)*(Invab)*cugauggaCaGaGuu aucgaa*(Invab) | 439 | AD00 433-AS | u*U*cgauAacucUgUc Cauc*a*g | 484 |
| AD00 434 | AD00 434-SS | (GLS- 5)*(Invab)*ggcuccuuAuGgUua uacgaa*(Invab) | 440 | AD00 434-AS | u*U*cguaUaacaAuAa Ggag*c*c | 485 |
| AD00 435 | AD00 435-SS | (GLS- 5)*(Invab)*gaagcaccAaCuGag caaaga*(Invab) | 441 | AD00 435-AS | u*C*uuugCucagUuGg Ugcu*u*c | 486 |
| AD00 436 | AD00 436-SS | (GLS- 5)*(Invab)*gggaaaugGaCaGag uuauca*(Invab) | 442 | AD00 436-AS | u*G*auaaCucugUcCa Uuuc*c*c | 487 |
| AD00 437 | AD00 437-SS | (GLS- 5)*(Invab)*gcugcuacUaCcAuu auggaa*(Invab) | 443 | AD00 437-AS | u*U*ccauAauggUaGu Agca*g*c | 488 |
| AD00 438 | AD00 438-SS | (GLS- 5)*(Invab)*caauccagAuGcUga gauuca*(Invab) | 444 | AD00 438-AS | u*G*aaucUcagcAuCu Ggau*u*g | 489 |
| AD00 439 | AD00 439-SS | (GLS- 5)*(Invab)*gauucuccAcCaCug uuacaa*(Invab) | 445 | AD00 439-AS | u*U*guaaCagugGuGg Agaa*u*c | 490 |
| AD00 440 | AD00 440-SS | (GLS- 5)*(Invab)*gcagaauaCuAcCca aaugga*(Invab) | 446 | AD00 440-AS | u*C*cauuUggguAgUa Uucu*g*c | 491 |
| AD00 441 | AD00 441-SS | (GLS- 5)*(Invab)*ggauucgcCcUuGgu guuaua*(Invab) | 447 | AD00 441-AS | u*A*uaacAccaaGgGc Gaau*c*c | 492 |
| AD00 442 | AD00 442-SS | (GLS- 5)*(Invab)*cauucgccCuUgGug uuauaa*(Invab) | 448 | AD00 442-AS | u*U*auaaCaccaAgGg Cgaa*u*g | 493 |
| AD00 474 | AD00 474-SS | (GLS- 15)*(Invab)*cauggacaGaGuUa ucgagga*(Invab) | 449 | AD00 474-AS | u*C*cuCg(aUNA)uaac UcUgUcca*u*g | 494 |
| AD00 475 | AD00 475-SS | (GLS- 15)*(Invab)*gcugccaaGcUuGg ucaucua*(Invab) | 450 | AD00 475-AS | u*A*gaugAccaaGcUu Ggca*g*c | 495 |
| AD00 476 | AD00 476-SS | (GLS- 15)*(Invab)*cccaagcuUgGuCa ucuauga*(Invab) | 451 | AD00 476-AS | u*C*auagAugacCaAg Cuug*g*g | 496 |
| AD00 477 | AD00 477-SS | (GLS- 15)*(Invab)*ggugcuacCaUgGu aauggaa*(Invab) | 452 | AD00 477-AS | u*U*ccauUaccaUgGu Agca*c*c | 497 |
| AD00 478 | AD00 478-SS | (GLS- 15)*(Invab)*gaccauggUaAuGg acagaga*(Invab) | 453 | AD00 478-AS | u*C*ucUg(uUNA)cca uUaCcAugg*u*c | 498 |
| AD00 479 | AD00 479-SS | (GLS- 15)*(Invab)*gugguaauGgAcA gaguuaua*(Invab) | 454 | AD00 479-AS | u*A*uaacUcuguCcAu Uacc*a*c | 499 |
| AD00 480 | AD00 480-SS | (GLS- 15)*(Invab)*gagaguuaUcGaGg cacauaa*(Invab) | 455 | AD00 480-AS | u*U*auguGccucGaUa Acuc*u*c | 500 |
| AD00 481 | AD00 481-SS | (GLS- 15)*(Invab)*caaauggaCaGaGu uaucaaa*(Invab) | 456 | AD00 481-AS | u*U*ugauAacucUgUc Cauu*u*g | 501 |
| AD00 482 | AD00 482-SS | (GLS- 15)*(Invab)*gggacagaGuUaUc aaggcaa*(Invab) | 457 | AD00 482-AS | u*U*gcCu(uUNA)gau aAcUcUguc*c*c | 502 |
| AD00 483 | AD00 483-SS | (GLS- 15)*(Invab)*gaucaaguGuCcUu gcaacua*(Invab) | 458 | AD00 483-AS | u*A*guugCaaggAcAc Uuga*u*c | 503 |
| AD00 484 | AD00 484-SS | (GLS- 15)*(Invab)*cggguccaGgAuUg cuaccaa*(Invab) | 459 | AD00 484-AS | u*U*ggUa(gUNA)caa uCcUgGacc*c*g | 504 |
| AD00 485 | AD00 485-SS | (GLS- 15)*(Invab)*gugucacaGgAaGg acaugua*(Invab) | 460 | AD00 485-AS | u*A*caugUccuuCcUg Ugac*a*c | 505 |
| AD00 486 | AD00 486-SS | (GLS- 15)*(Invab)*gauguccaGuGaCa gaaucaa*(Invab) | 461 | AD00 486-AS | u*U*gauuCugucAcUg Gaca*u*c | 506 |
| AD00 487 | AD00 487-SS | (GLS- 15)*(Invab)*gcagaggcUcCuUc ugaacaa*(Invab) | 462 | AD00 487-AS | u*U*guucAgaagGaGc Cucu*g*c | 507 |
| AD00 474-1 | AD00 474-1-SS | (GLS- 15)*(Invab)*cauggacaGaGuUa ucgagga*(Invab) | 463 | AD00 474-1-AS | u*C*cucgAuaacUcUg Ucca*u*g | 508 |
| AD00 478-1 | AD00 478-1-SS | (GLS- 15)*(Invab)*gaccauggUaAuGg acagaga*(Invab) | 464 | AD00 478-1-AS | u*C*ucugUccauUaCc Augg*u*c | 509 |
| AD00 482-1 | AD00 482-1-SS | (GLS- 15)*(Invab)*gggacagaGuUaUc aaggcaa*(Invab) | 465 | AD00 482-1-AS | u*U*gccuUgauaAcUc Uguc*c*c | 510 |
| AD00 484-1 | AD00 484-1-SS | (GLS- 15)*(Invab)*cggguccaGgAuUg cuaccaa*(Invab) | 466 | AD00 484-1-AS | u*U*gguaGcaauCcUg Gacc*c*g | 511 |
| AD00 | AD00 | (GLS- | 467 | AD00 | u*A*gaugAccaaGcUu | 512 |
| 337-1 | 337-1-SS | 15)*(Invab)*guugccaaGcUuGg ucaucua*(Invab) | | 337-1-AS | Ggca*a*c | |
| AD00 436-1 | AD00 436-1-SS | (GLS- 15)*(Invab)*gggaaaugGaCaGa guuauca*(Invab) | 468 | AD00 436-1-AS | u*G*auaaCucugUcCa Uuuc*c*c | 513 |
| AD00 474-2 | AD00 474-2-SS | (GLS- 15)*(Invab)*gauggacaGaGuuA ucgagg*u*(Invab) | 888 | AD00 474-2-AS | a*C*cuCg(aUNA)uaac UcUgucCa*u*c | 892 |
| AD00 480-8 | AD00 480-8-SS | (GLS- 15)*(Imann)*gagaguuaUcGaG gcacaua*a*(Imann) | 889 | AD00 480-8-AS | u*U*auguGccucGaUa Acuc*u*c | 893 |
| AD00 480-1 | AD00 480-1-SS | (GLS- 15)*(Invab)*gagaguuaUcGagG cacaua*a*(Invab) | 890 | AD00 480-1-AS | u*U*augugccucGaUaa cuc*u*c | 894 |
| AD00 480-2 | AD00 480-2-SS | (GLS- 15)*(Invab)*gagaguuaU*cGag G*cacaua*a*(Invab) | 891 | AD00 480-2-AS | u*U*augugccucG*aU* aacuc*u*c | 895 |

### Mismatch

It is known to those skilled in the art that mismatches are tolerated for the efficacy of dsRNAs, especially where mismatches are within the terminal region of dsRNAs. Some mismatches are better tolerated, such as mismatches with wobble base pairs G:U and A:C are better tolerated for efficacy (Du et el., A systematic analysis of the silencing effects of an active siRNA at all single-nucleotide mismatched target sites. Nucleic Acids Res. 2005 Mar 21;33(5):1671-7. Doi: 10.1093/nar/gki312. Nucleic Acids Res. 2005;33(11):3698). In some embodiments of the methods and compounds of the present invention, the LPA dsRNA agent may contain one or more mismatches to the LPA target sequence. In some embodiments, the LPA dsRNA agent of the present invention does not comprise a mismatch. In certain embodiments, the LPA dsRNA agent of the present invention comprises no more than 1 mismatch. In some embodiments, the LPA dsRNA agent of the present invention comprises no more than 2 mismatch. In certain embodiments, the LPA dsRNA agent of the present invention comprises no more than 3 mismatch. In some embodiments of the present invention, the antisense strand of the LPA dsRNA agent comprises a mismatch to an LPA target sequence that is not at the center of the complementary region. In some embodiments, the antisense strand of the LPA dsRNA agent comprises 1, 2, 3, 4 or more mismatches located within the last 5, 4, 3, 2 or 1 nucleotide of one or both of the 5'-end or 3'-end of the complementary region. The methods described herein and/or methods known in the art may be used to determine whether LPA dsRNA agents comprising mismatches to LPA target sequences are effective in inhibiting LPA gene expression.

### Complementarity

As used herein, unless otherwise stated, the term "complementarity/complementary", when used to describe the correlation of a first nucleotide sequence (e.g., an LPA dsRNA agent sense strand or a target LPA mRNA) with a second nucleotide sequence (e.g., LPA dsRNA agent antisense strand or single-stranded antisense polynucleotide), refers to the ability of an oligonucleotide or polynucleotide comprising the first nucleotide sequence to hybridize with an oligonucleotide or polynucleotide comprising the second nucleotide sequence [to form hydrogen bonds between base pairs under mammalian physiological conditions (or similar conditions in vitro)], and to form a double helix or double helix structure under certain conditions. Other conditions may also be applied, such as physiologically relevant conditions that may be encountered in an organism. The technician will be able to determine the best set of conditions for testing the complementarity of the two sequences based on the final application of the hybridized nucleotides. Complementary sequences include Watson-Crick base pairs or non-Watson-Crick base pairs, and include natural or modified nucleotides or nucleotide mimics, as long as at least to the extent described above for hybridization. Sequence identity or complementarity is independent of modifications.

For example, a complementary sequence within an LPA dsRNA as described herein comprises base pairing of an oligonucleotide or polynucleotide containing a first nucleotide sequence with an oligonucleotide or polynucleotide containing a second nucleotide sequence on the full length of the one or two nucleotide sequences. Such sequences may herein be referred to as "completely complementary" to each other. It will be appreciated that in embodiments in which two oligonucleotides are designed to form one or more single-stranded overhangs upon hybridization, such overhangs are not herein considered as mismatches determined based on complementarity. For example, the LPA dsRNA agent comprises an oligonucleotide of 19 nucleotides in length and another oligonucleotide of 20 nucleotides in length, wherein the longer oligonucleotide comprises a sequence of 19 nucleotides completely complementary to the shorter oligonucleotide, which may be referred to as "completely complementary" for the purposes herein described. Thus, as used herein, "completely complementary" means that all (100%) bases in the contiguous sequence of the first polynucleotide will hybridize with the same number of bases in the contiguous sequence of the second polynucleotide. The contiguous sequence may comprise all or part of the first or second nucleotide sequence.

As used herein, the term "substantially complementary" means that in a hybrid pair of nucleobase sequences, at least about 85% (but not all) of the bases in the contiguous sequence of the first polynucleotide hybridize with the same number of bases in the contiguous sequence of the second polynucleotide. If two sequences comprise one or more mismatched base pairs during hybridization, such as at least 1, 2, 3, 4, or 5 mismatched base pairs, the term "substantially complementary" may be used to refer to the first sequence forming a duplex of up to 15, 16, 17, 18, 19, 20, 21, 22, 23, 24, 25, 26, 27, 28, 29, or 30 base pairs (bp) with the second sequence, while retaining the ability to hybridize under conditions most relevant to its final application, e.g., inhibition of LPA gene expression by the RISC pathway. The term "partially complementary" may be used herein to means that in a hybrid pair of nucleobase sequences, at least 75% (but not all) of the bases in the contiguous sequence of the first polynucleotide hybridize with the same number of bases in the contiguous sequence of the second polynucleotide. In some embodiments, "partially complementary" means that at least 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% of the bases in the contiguous sequence of the first polynucleotide will hybridize with the same number of bases in the contiguous sequence of the second polynucleotide.

The terms "complementary", "completely complementary", "substantially complementary" and "partially complementary" when used herein may be used to refer to a base match between a sense strand and an antisense strand of an LPA dsRNA agent, a base match between an antisense strand of an LPA dsRNA agent and a sequence of a target LPA mRNA, or a base match between a single-stranded antisense oligonucleotide and a sequence of a target LPA mRNA. It will be understood that the term "antisense strand of LPA dsRNA agent" may refer to the same sequence as "LPA antisense polynucleotide agent".

As used herein, the term "substantially identical" or "substantial identity" used when referring to nucleic acid sequences mean that the nucleic acid sequence comprises a sequence having at least about 85% or more sequence identity compared to the reference sequence, preferably at least 90%, at least 91%, at least 92%, at least 93%, at least 94%, at least 95%, at least 96%, at least 97%, at least 98%, or at least 99% identity. The percentage of sequence identity is determined by comparing the best alignment of the two sequences on the alignment window. Percentages are calculated by determining the number of positions where the same nucleic acid base occurs in both sequences to produce the number of match positions; dividing the number of match positions by the total number of positions in the alignment window, and the result was multiplied by 100 to arrive at the percentage of sequence identity. The inventions disclosed herein comprise substantially identical nucleotide sequences as those disclosed herein (e.g., in Tables 1-5). In some embodiments, the nucleotide sequence is completely identical or has at least about 85%, 86%, 87%, 88%, 89%, 90%, 91%, 92%, 93%, 94%, 95%, 96%, 97%, 98% or 99% identity to the sequence disclosed herein (e.g., in Tables 1-3).

As used herein, the term "sequence-containing chain" refers to an oligonucleotide containing a nucleotide chain described by a sequence indicated using standard nucleotide nomenclature. As used herein, the term "double-stranded RNA" or "dsRNA" refers to a sequence comprising an RNA molecule or an RNAi molecule complex having a hybrid double-stranded region comprising two antiparallel and substantially or completely complementary nucleic acid strands, which are referred to as having "sense" and "antisense" directions relative to a target LPA RNA, respectively. The double-stranded region can have any desired length that allows specific degradation of the target LPA RNA via the RISC pathway, but is typically 9 to 30 base pairs in length, e.g. 15-30 base pairs in length. Considering the duplex between 9 and 30 base pairs, the duplex may be any length within this range, for example, 9, 10, 11, 12, 13, 14, 15, 16, 17, 18, 19, 20, 21, 22, 23, 24, 25, 26, 27, 28, 29 or 30 base pairs, and any subrange thereof, including but not limited to 15-30 base pairs, 15-26 base pairs, 15-23 base pairs, 15-22 base pairs, 15-21 base pairs, 15-20 base pairs, 15-19 base pairs, 15-18 base pairs, 15-17 base pairs, 18-30 base pairs, 18-26 base pairs, 18-23 base pairs, 18-22 base pairs, 18-21 base pairs, 18-20 base pairs, 19-30 base pairs, 19-26 base pairs, 19-23 base pairs, 19-22 base pairs, 19-21 base pairs, 19-20 base pairs, 20-30 base pairs, 20-26 base pairs, 20-25 base pairs, 20-24 base pairs, 20-23 base pairs, 20-22 base pairs, 20-21 base pairs, 21-30 base pairs, 21-26 base pairs, 21-25 base pairs, 21-24 base pairs, 21-23 base pairs, or 21-22 base pairs. The length of LPA dsRNA agent produced in cells by processing with Dicer and similar enzymes is generally in the range of 19-22 base pairs. One strand of the double-stranded region of the LPA dsDNA agent contains a sequence substantially complementary to the region of the target LPARNA. The two strands forming the duplex structure may come from a single RNA molecule with at least one self-complementing region, or may be formed from two or more individual RNA molecules. In cases where the double-stranded region is formed by a single molecule, the molecule may have a duplex structure (referred to herein as a "hairpin loop") formed by one strand of a single-stranded nucleotide chain at the 3'-end and the corresponding other strand at the 5'-end. In some embodiments of the present invention, the hairpin configuration comprises at least 1, 2, 3, 4, 5, 6, 7, 8, 9, 10, 11, 12, 13, 14, 15, 16, 17, 18, 19, 20 or more unpaired nucleotides. When the essentially complementary two strands of the LPA dsRNA agent consist of individual RNA molecules, these molecules do not need to be covalently linked, but can be covalently linked. When two strands are covalently linked by means other than a hairpin loop, the linking structure is called a "linker". The term "siRNA" is also used herein to refer to dsRNA agent as described herein.

In some embodiments of the present invention, the LPA dsRNA agent may comprise sense and antisense sequences having unpaired nucleotides or nucleotide analogs at one or both ends of the dsRNA agent. Ends without unpaired nucleotides are called "blunt ends" and have no nucleotide overhangs. If both ends of the dsRNA agents are blunt ends, the dsRNA is referred to as "blunt endsed". In some embodiments of the present invention, the first end of the dsRNA agent is blunt-ended, in some embodiments, the second end of the dsRNA agent is blunt-ended, and in certain embodiments of the present invention, both ends of the LPA dsRNA agent are blunt-ended.

In some embodiments of the dsRNA agent of the present invention, the dsRNA does not have one or two blunt ends. In this case, there is at least one unpaired nucleotide at the end of one strand of the dsRNA agent. For example, a nucleotide overhang is present when the 3'-end of one strand of dsRNA extends beyond the 5'-end of another strand, and vice versa. The dsRNA may comprise an overhang of at least 1, 2, 3, 4, 5, 6 or more nucleotides. The nucleotide overhang may comprise or consist of a nucleotide/nucleoside analog, including a deoxynucleotide/nucleoside. It should be understood that in some embodiments, the nucleotide overhang is on the sense strand of the dsRNA agent, on the antisense strand of the dsRNA agent, or at both ends of the dsRNA agent, the nucleotide of the overhang may be present at the 5'-end, 3'-end, or both ends of the antisense strand or sense strand of the dsRNA. In some embodiments of the present invention, one or more nucleotides in the overhang are replaced by nucleoside phosphorothioate.

As used herein, the terms "antisense strand" or "guide strand" refer to a strand of LPA dsRNA agent that contains a region substantially complementary to the LPA target sequence. As used herein, the terms "sense strand" or "passenger strand" refer to a strand of an LPA dsRNA agent that contains a region substantially complementary to the region of the antisense strand of the LPA dsRNA agent.

### Modification

In some embodiments of the present invention, the RNA of the LPA RNAi agent is chemically modified to obtain enhanced stability and/or one or more other beneficial properties. Nucleic acids in certain embodiments of the present invention may be synthesized and/or modified by methods well known in the art, see, for example, "Current protocols in Nucleic Acid Chemistry," Beaucage, S. L. et al. (Eds.), John Wiley & Sons, Inc., New York, N.Y, USA, which is incorporated herein by reference. Modifications that may be present in certain embodiments of the LPA dsRNA agent of the present invention include, such as: (a) a terminal modification, such as a 5'-end modification (phosphorylation, conjugation, reverse linkage, etc.), a 3'-end modification (conjugation, DNA nucleotides, reverse linkage, etc.); (b) a base modification, such as, for a base pair, a base substitution with a stabilized base, a destabilized base, or an expanded pool of partners, a base deletion (an abasic nucleotide), or a base conjugation; (c) a sugar modification (e.g., at the 2' or 4' position) or a sugar substitution; and (d) a backbone modification, including a modification or a substitution of phosphodiester bond. Specific examples of RNA compounds available in certain embodiments of LPA dsRNA agent, LPA antisense polynucleotides, and LPA sense polynucleotides of the present invention include, but are not limited to, RNAs containing a modified backbone or without natural internucleoside linkage. As a non-limiting example, RNA with backbone modification may have no phosphorus atoms in the backbone. RNAs that have no phosphorus atoms in their internucleoside backbone can be called oligonucleosides. In some embodiments of the present invention, the modified RNA has phosphorus atoms in its internucleoside backbone.

It should be understood that the terms "RNA molecule" or "RNA" or "ribonucleic acid molecule" include not only RNA molecules expressed or found in nature, but also analogues and derivatives of RNA comprising one or more ribonucleotide/ribonucleoside analogues or derivatives as described herein or known in the art. The terms "ribonucleoside" and "ribonucleotide" are used interchangeably herein. RNA molecules can be modified in nucleobase structures or ribose-phosphate backbone structures (e.g., as described below), and molecules containing ribonucleoside analogs or derivatives must retain the ability to form duplex. As a non-limiting example, an RNA molecule may also comprise at least one modified ribonucleoside, including but not limited to a 2'-O-methyl-modified nucleoside, a nucleoside comprising a 5' phosphorothioate group, a terminal nucleoside linked to a cholesterol derivative or a dodecanoic acid bisdecylamide group, a locked nucleoside, an abasic nucleoside, a 2'-deoxy-2'-fluoro modified nucleoside, a 2'-amino modified nucleoside, a 2'-alkyl modified nucleoside, a morpholino nucleoside, an phosphoramidate, or a non-natural base comprising nucleoside, or any combination thereof. In some embodiments of the present invention, the RNA molecule comprises the following number of modified ribonucleosides: at least 1, 2, 3, 4, 5, 6, 7, 8, 9, 10, 11, 12, 13, 14, 15, 16, 17, 18, 19, 20 or up to the full length of the ribonucleosides of the LPA dsRNA agent molecule. For each of the multiple modified ribonucleosides in such RNA molecules, the modifications do not have to be the same.

In some embodiments, the dsRNA agent, LPA antisense polynucleotides and/or LPA sense polynucleotides of the present invention may comprise one or more independently selected modified nucleotides and/or one or more independently selected non-phosphodiester bonds. The term "independently selected" as used herein is used to mean selected elements, such as modified nucleotides, non-phosphodiester bonds, etc., to mean that two or more selected elements may be identical to each other but do not have to be identical to each other. As used herein, "nucleotide base", "nucleotide" or "nucleobase" are heterocyclic pyrimidine or purine compounds, which are standard components of all nucleic acids, and include nucleotide-forming bases: adenine (a), guanine (g), cytosine (c), thymine (t) and uracil (u). The nucleobases may be further modified to include, but are not intended to limit, universal bases, hydrophobic bases, ambiguous bases, size-enlarged bases, and fluorinated bases. The term "ribonucleotide" or "nucleotide" may be used herein to refer to an unmodified nucleotide, a modified nucleotide, or an alternative moiety. Those skilled in the art will recognize that guanine, cytosine, adenine and uracil can be replaced by other moieties without significantly altering the base pairing properties of oligonucleotides containing nucleotides bearing such replacement moieties.

In one embodiment, the modified RNA expected to be used in the methods and compositions described herein is a peptide nucleic acid (PNA) having the ability to form a desired duplex structure and to allow or mediate specific degradation of the target RNA via the RISC pathway. In some embodiments of the present invention, LPA RNA interference agents comprise single-stranded RNA that interacts with a target LPA RNA sequence to direct cleavage of the target LPA RNA.

Modified RNA backbones may comprise, such as, phosphorothioates, chiral phosphorothioates, phosphorodithioates, phosphotriesters, aminoalkylphosphotriesters, methyl and other alkyl phosphonates (including 3'-alkylene phosphonates and chiral phosphonates), phosphinate, phosphoramidates (including 3'-amino phosphoramidates and aminoalkylphosphoramidates), thionophosphoramidates, thionoalkylphosphonates, thionoalkylphosphotriesters, and boranophosphates (which have normal 3'-5' linkages, and 2'-5' linked analogues of these, and those with inverted polarity, wherein adjacent nucleoside unit pairs are linked in the form of 3'-5' to 5'-3' or 2'-5' to 5'-2'). Various salts, mixed salts, and free acid forms are also included. Methods for preparing phosphorus-containing bonds are conventional means in the art, and such methods may be used to prepare certain modified LPA dsRNA agent, certain modified LPA antisense polynucleotides, and/or certain modified LPA sense polynucleotides of the present invention.

The modified RNA backbone in which no phosphorus atoms are included has a backbone formed by short-chain alkyl or cycloalkyl internucleoside linkage, mixed heteroatom and alkyl or cycloalkyl internucleoside linkage, or one or more short-chain heteroatom or heterocycle internucleoside linkage, which comprises those having morpholine bonds (formed partly from the sugar moiety of the nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and other moieties mixed with N, O, S, and CH₂ components. Methods for preparing modified RNA backbones that do not contain phosphorus atoms are conventional practice in the art, and such methods may be used to prepare certain modified LPA dsRNA agent, certain modified LPA antisense polynucleotides, and/or certain modified LPA sense polynucleotides of the present invention.

In certain embodiments of the present invention, an RNA mimic is included in LPA dsRNA, LPA antisense polynucleotides, and/or LPA sense polynucleotides, for example but unlimited, sugars and internucleoside linkages (i.e., backbones) of nucleotide units are replaced with new groups. In such embodiments, base units are maintained to hybridize with suitable LPA nucleic acid target compounds. One such oligomeric compound, an RNA mimic that has been shown to have excellent hybridization properties, is called a peptide nucleic acid (PNA). In PNA compounds, the sugar backbone of RNA is replaced by an amide-containing backbone, especially an aminoethylglycine backbone. The nucleobases are retained and bind directly or indirectly to the aza nitrogen atoms of the amide moiety of the backbone. Methods for preparing RNA mimics are conventionally practiced in the art, and such methods may be used to prepare certain modified LPA dsRNA agent of the present invention.

Some embodiments of the present invention include RNA having a phosphorothioate backbone and oligonucleosides having a heteroatom backbone, in particular-CH₂--NH--CH₂-, --CH₂--N(CH₃)- -O-CH₂-[called methylene (methylimino) or MMI backbone], --CH₂--O--N(CH₃)--CH₂--, --CH₂-N(CH₃)--N(CH₃)--CH₂- and --N(CH₃)--CH₂----[where the natural phosphodiester backbone is denoted as --O--P--O--CH₂--]. Methods for preparing RNA having a phosphorothioate backbone and oligonucleosides having a heteroatom backbone are conventionally practiced in the art, and such methods may be used to prepare certain modified LPA dsRNA agents, certain LPA antisense polynucleotides and/or certain LPA sense polynucleotides of the present invention.

The modified RNA may also comprise one or more substituted sugar moieties. The LPA dsRNA, LPA antisense polynucleotide and/or LPA sense polynucleotide of the present invention may comprise one of the following at the 2' position: OH; F; O--, S--, or N-alkyl; O--, S--, or N-alkenyl; O-, S- or N-alkynyl; or O-alkyl-O-alkyl, wherein the alkyl, alkenyl and alkynyl can be substituted or unsubstituted C₁ to C₁₀ alkyl or C₂ to C₁₀ alkenyl and alkynyl. Exemplary suitable modifications include: O[(CH₂)ₙO]ₘCH₃, O(CH₂)ₙOCH₃, O(CH₂)ₙNH₂, O(CH₂)ₙCH₃, O(CH₂)ₙONH₂ and O(CH₂)ₙON[(CH₂)ₙCH₃)]₂, where n and m are 1 to about 10. In other embodiments, the dsRNA includes one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino; substituted silyl, a RNA cleavage group, a reporter group, an intercalating agent; a group used to improve the pharmacokinetic properties of the LPA dsRNA agent; or used to improve LPA dsRNA agent, a group for improving pharmacodynamic properties of the LPA dsRNA agent, LPA antisense polynucleotide and/or LPA sense polynucleotide, and other substituents with similar properties. In some embodiments, the modifications comprise 2'-methoxyethoxy (2'-O--CH₂CH₂OCH₃, also known as 2'-O-(2-methoxyethyl) or 2'-MOE) (Martin et al., Helv. Chim. Acta, 1995, 78:486-504), that is, alkoxy-alkoxy. Another exemplary modification is 2'-dimethylaminoethoxyethoxy, i.e. O(CH₂)₂ON(CH₃)₂ group, also referred to as 2'-DMAOE, as described in the examples below; and 2'-dimethylaminoethoxyethoxy (also known in the art as 2'-O-dimethylaminoethoxyethyl or 2'-DMAEOE), i.e. 2'-O--CH₂-O--CH₂--N(CH₂)₂. Methods for preparing those modified RNAs described are conventionally practiced in the art, and such methods may be used to prepare certain modified LPA dsRNA agents of the present invention.

Other modifications include 2'-methoxy (2'-OCH₃), 2'-aminopropoxy (2'-OCH₂CH₂CH₂NH₂) and 2'-fluoro (2'-F). Similar modifications may also be performed in the LPA dsRNA agents of the present invention, at other positions on the RNA of the LPA antisense polynucleotides, in LPA sense polynucleotides and/or at other positions of the LPA sense polynucleotides, in particular at the 3' position of sugars on 3' terminal nucleotides or in 2'-5' linked LPA dsRNA, LPA antisense polynucleotides or LPA sense polynucleotides, and at the 5' position of the 5' terminal nucleotides. LPA dsRNA agents, LPA antisense polynucleotides and/or LPA sense polynucleotides may also have sugar mimics, e.g., in place of the cyclobutyl moieties of pentofuranose. Methods for preparing for example those modified RNAs described are conventionally practiced in the art, and such methods may be used to prepare certain modified LPA dsRNA agents, LPA antisense polynucleotides and/or LPA sense polynucleotides of the present invention.

In some embodiments, LPA dsRNA agents, LPA antisense polynucleotides, and/or LPA sense polynucleotides may comprise nucleobase (generally referred to simply as "base" in the art) modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases, such as 5-methylcytosine (5-me-C), 5-hydroxymethyl cytosine, xanthine, hypoxanthine, 2-aminoadenine, 6-methyl and other alkyl derivatives of adenines and guanines, 2-propyl and other alkyl derivatives of adenines and guanines, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil; 8-halo, 8-amino, 8-thiol, 8-thioalkyl, 8-hydroxyl and other 8-substituted adenines and guanines; 5-halo, particularly 5-bromo, 5-trifluoromethyl and other 5-substituted uracils and cytosines; 7-methylguanine and 7-methyladenine, 8-azaguanine and 8-azaadenine, 7-azaguanine and 7-azaadenine, and 3-azaguanine and 3-azaadenine. Additional nucleobases that may be included in certain embodiments of the LPA dsRNA agent of the present invention are known in the art, see for example: Modified Nucleosides in Biochemistry, Biotechnology and Medicine, Herdewijn, P. Ed. Wiley-VCH, 2008; The Concise Encyclopedia Of Polymer Science And Engineering, pages 858-859, Kroschwitz, J. L, Ed. John Wiley & Sons, 1990, English et al., Angewandte Chemie, International Edition, 1991, 30, 613, Sanghvi, Y S., Chapter 15, dsRNA Research and Applications, pages 289-302, Crooke, S. T. and Lebleu, B., Ed., CRC Press, 1993. Methods for preparing dsRNAs, LPA antisense polynucleotides, and/or LPA sense polynucleotides (such as those described herein) comprising nucleobase modifications and/or substitutions are routinely practiced in the art, and such methods may be used to prepare certain modified LPA dsRNA agents, LPA sense polynucleotides, and/or LPA antisense polynucleotides of the present invention.

Certain embodiments of LPA dsRNA agents, LPA antisense polynucleotides and/or LPA sense polynucleotides of the present invention include RNA modified to include one or more locked nucleic acids (LNAs). Locked nucleic acids are nucleotides having such a modified ribose moiety that contain additional bridges linking 2' and 4' carbons. This structure effectively "locks" the ribose in the 3'-internal structure conformation. Addition of locked nucleic acids to LPA dsRNA agents, LPA antisense polynucleotides and/or LPA sense polynucleotides of the present invention can increase stability in serum and reduce off-target effects (Elmen, J. et al., (2005) Nucleic Acids Research 33(1):439-447; Mook, O R. et al., (2007)Mol Canc Ther 6(3):833-843; Grunweller, A. et al., (2003) Nucleic Acids Research 31(12):3185-3193). Methods for preparing dsRNA agents, LPA antisense polynucleotides, and/or LPA sense polynucleotides comprising locked nucleic acids are conventionally performed in the art, and such methods may be used to prepare certain modified LPA dsRNA agents of the present invention. Certain embodiments of LPA dsRNA compounds, sense polynucleotides, and/or antisense polynucleotides of the present invention include at least one modified nucleotide comprising: a 2'-O-methyl nucleotide, a 2'-fluoro nucleotide, a 2'-deoxynucleotide, a 2',3'-seco nucleotide mimic, a locked nucleotide, a 2'-F-arabinose nucleotide, a 2'-methoxyethyl nucleotide, a 2'-amino modified nucleotide, a 2'-alkyl modified nucleotide, a morpholino nucleotide and a 3'-Ome nucleotide, a nucleotide comprising 5'-phosphorothioate group, or a terminal nucleotide linked to a cholesterol derivative or a dodecanoic acid bisdecylamide group, a 2'-amino modified nucleotide, a phosphoramidate, or a non-natural base comprising nucleotide. In some embodiments, the LPA dsRNA compound comprises an E-vinyl phosphonate nucleotide at the 5'-end of the antisense strand (also referred to herein as a guide strand).

In certain embodiments of the present invention, at least one modified nucleotide is included in a LPA dsRNA compound, at 3'-end and 5'-end of a sense polynucleotide and/or at 3'-end of an antisense polynucleotide, wherein the at least one modified nucleotide includes an abasic nucleotide, a ribitol, a reverse nucleotide, a reverse abasic nucleotide, a reverse 2'-OMe nucleotide, a reverse 2'-deoxynucleotide. It is known to those skilled in the art that the inclusion of abasic or reverse abasic nucleotides at the ends of oligonucleotides can enhance stability (Czauderna et al. Structural variations and stabilizing modifications of synthetic siRNAs in mammalian cells. Nucleic Acids Res. 2003;31(11):2705-2716. doi:10.1093/nar/gkg393).

In some embodiments of the present invention, the LPA dsRNA comprises one or two isomannitol residues at the 3'-end and 5'-end of the sense strand. In certain embodiments, the sense chain independently comprises one isomannitol residue at the 3'-end or 5'-end, respectively. The case comprising an isomannitol residue has the following examples: 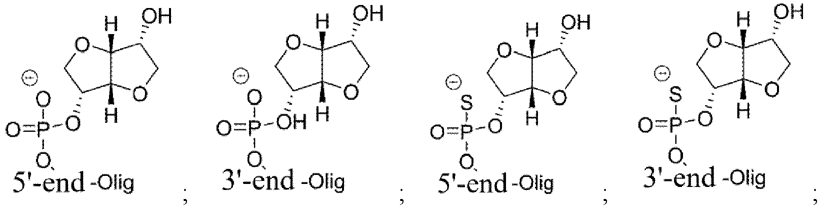 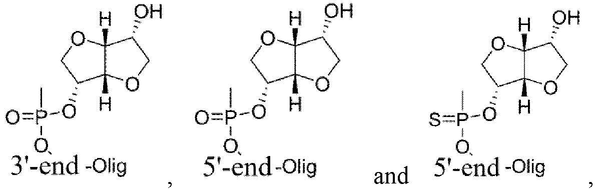 where each phrase "Olig" independently represents the polynucleotide moiety. Exemplary isomannitol residues (imann) include, but are not limited to, the following: 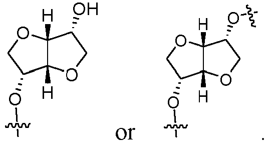 In some embodiments, the isomannitol residue may also be replaced with its stereoisomer, non-limiting example: 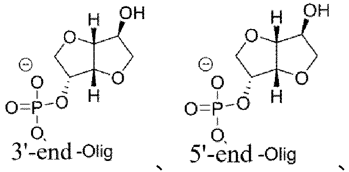 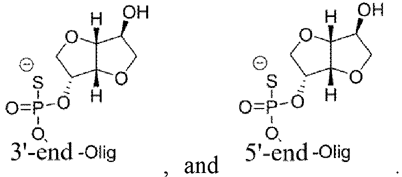 In some embodiments, the sense strand independently comprises one isomannitol residue (imann) at the 3'-end or the 5'-end, respectively, and further comprises a targeting group conjugated at the 5'-end, for example, the targeting group N-acetyl-galactosamine, preferably GLS-15 described above, with the following exemplary structure: . 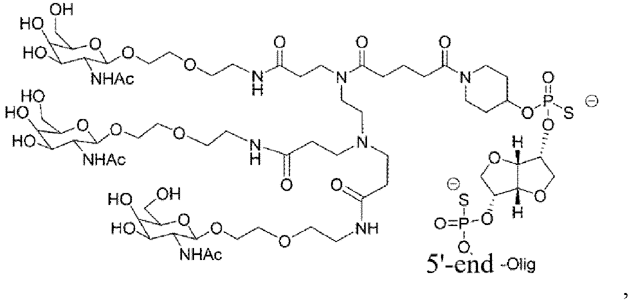 where each phrase "Olig" independently represents the polynucleotide moiety.

In certain embodiments of the present invention, an LPA dsRNA compound and/or an antisense polynucleotide comprises at least one modified nucleotide, wherein the at least one modified nucleotide comprises an unlocked nucleic acid (UNA) nucleotide and/or a glycol nucleic acid nucleotide (GNA). It is known to those skilled in the art that UNA and GNA are thermally labile chemical modifications that can significantly improve the off-target profile of siRNA compounds (Janas, et al., Selection of GalNAc-conjugated siRNAs with limited off target-driven rat hepatotoxicity. Nat Commun. 2018;9(1):723. doi:10.1038/s41467-018-02989-4; Laursen et al., Utilization of unlocked nucleic acid (UNA) to enhance siRNA performance in vitro and in vivo. Mol BioSyst. 2010;6:862-70).

Another modification may be included in the RNA of LPA dsRNA agents, LPA antisense polynucleotides and/or LPA sense polynucleotides of certain embodiments of the present invention, and includes one or more ligands, moieties or chemically linked conjugates to RNA that enhance one or more characteristics of LPA dsRNA agents, LPA antisense polynucleotides and/or LPA sense polynucleotides, respectively. Non-limiting examples of characteristics that may be enhanced are: activity of LPA dsRNA agents, LPA antisense polynucleotides and/or LPA sense polynucleotides, cellular distribution, delivery of LPA dsRNA agents, pharmacokinetic properties of LPA dsRNA agents, and cellular uptake of LPA dsRNA agents. In some embodiments of the present invention, the LPA dsRNA agent comprises one or more targeting or linking groups, which in some embodiments of the LPA dsRNA agent of the present invention are conjugated to the sense strand. A non-limiting example of the targeting group is a compound comprising N-acetyl-galactosamine (GalNAc). The terms "targeting group", "targeting agent", "linking agent", "targeting compound" and "targeting ligand" are used interchangeably herein. In some embodiments of the present invention, the LPA dsRNA agent comprises a targeting compound conjugated to the 5'-end of the sense strand. In some embodiments of the present invention, the LPA dsRNA agent comprises a targeting compound conjugated to the 3'-end of the sense strand. In some embodiments of the present invention, the LPA dsRNA agent comprises a targeting group containing GalNAc. In some embodiments of the present invention, the LPA dsRNA agent does not comprise a targeting compound conjugated to one or both of the 3'-end and 5'-end of the sense strand. In some embodiments of the present invention, the LPA dsRNA agent does not comprise a GalNAc-containing targeting compound conjugated to one or both of the 5'-end and 3'-end of the sense strand.

Additionally targeting and linking agents are well known in the art, for example, targeting and linking agents that may be used in certain embodiments of the present invention include, but are not limited to, lipid moieties, such as cholesterol moieties (Letsinger et al., Proc. Natl. Acid. Sci. USA, 1989, 86: 6553-6556), cholic acid (Manoharan et al., Bioorg. Med. Chem. Let., 1994, 4:1053-1060), thioethers, such as beryl-S-tritylthiol (Manoharan et al., Ann. N.Y. Acad. Sci., 1992, 660:306-309; Manoharan et al., Biorg. Med. Chem. Let., 1993, 3:2765-2770), thiocholesterol (Oberhauser et al., Nucl. Acids Res., 1992, 20:533-538), aliphatic chains, such as dodecanediol or undecyl residues (Saison-Behmoaras et al., EMBO J, 1991, 10:1111-1118; Kabanov et al., FEBS Lett., 1990, 259:327-330; Svinarchuk et al., Biochimie, 1993, 75:49-54), phospholipids, such as di-hexadecyl-rac-glycerol or triethyl-ammonium 1,2-di-O-hexadecyl-rac-glycerol-3-phosphonate (Manoharan et al., Tetrahedron Lett., 1995, 36:3651-3654; Shea et al., Nucl. Acids Res., 1990, 18:3777-3783), polyamine or polyethylene glycol chains (Manoharan et al., Nucleosides & Nucleotides, 1995, 14:969-973) or adamantane acetic acid (Manoharan et al., Tetrahedron Lett., 1995, 36:3651-3654), palmitoyl moiety (Mishra et al., Biochim. Biophys. Acta, 1995, 1264:229-237) or octadecylamine or hexamino-carbonyloxycholesterol moiety (Crooke et al., J. Pharmacol. Exp. Ther. , 1996, 277:923-937).

Certain embodiments of compositions comprising LPA dsRNA agents, LPA antisense polynucleotides, and/or LPA sense polynucleotides may comprise ligands that alter the distribution, targeting, and the like properties of LPA dsRNA agents. In some embodiments of compositions comprising LPA dsRNA agents of the present invention, ligands increase affinity for selected targets (such as molecules, cells or cell types, compartments, e.g., cell or organ compartments, tissues, organs or body regions), for example, compared to species in which such ligands are not present. Ligands useful in compositions and/or methods of the present invention may be naturally occurring substances, such as proteins (e.g., human serum albumin (HSA), low density lipoprotein (LDL) or globulins), carbohydrates (e.g., dextran, amylopectin, chitin, chitosan, inulin, cyclodextrin or hyaluronic acid) or lipids. The ligand may also be a recombinant or synthetic molecule, such as a synthetic polymer, such as a synthetic polyamino acid or polyamine. Examples of polyamino acids are polylysine (PLL), poly L-aspartic acid, poly L-glutamic acid, styrene-maleic anhydride copolymer, poly (L-lactide-co-glycolic acid) copolymer, divinyl ether-maleic anhydride copolymer, N-(2-hydroxypropyl) methacrylamide copolymer (HMPA), polyethylene glycol (PEG), polyvinyl alcohol (PVA), polyurethane, poly (2-ethyl acrylic acid), N-isopropylacrylamide polymer, or polyphosphazine. Examples of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of polyamine, or alpha helical peptide.

The ligands included in the compositions and/or methods of the present invention may comprise targeting groups, non-limiting examples of which are cell or tissue targeting agents, such as lectins, glycoproteins, lipids or proteins, such as antibodies that bind to specific cell types such as kidney cells or hepatocytes. A targeting group may be thyrotropin, melanotropin, lectin, glycoprotein, surfactant protein A, mucin carbohydrate, multivalent lactose, multivalent galactose, N-acetyl-galactosamine, N-acetyl-glucosamine multivalent mannose, multivalent fucose, glycosylated polyamino acid, multivalent galactose, transferrin, bisphosphonate, polyglutamate, polyaspartate, a lipid, cholesterol, a steroid, bile acid, folate, vitamin B 12, vitamin A, biotin or an RGD peptide or an RGD peptides mimic.

Other examples of ligands include dyes, intercalating agents (e.g. acridines), cross linkers (e.g. psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), lipophilic molecules, e.g, cholesterol, cholic acid, adamantane acetic acid, 1-pyrene butyric acid, dihydrotestosterone, 1,3-bis-O(hexadecyl)glycerol, geranyloxyhexyl group, hexadecylglycerol, borneol, menthol, 1,3-propanediol, heptadecyl group, palmitic acid, myristic acid, O3-(oleoyl)lithocholic acid, O3-(oleoyl)cholic acid, dimethoxytrityl, or phenoxazine and peptide conjugates (e.g., antennapedia peptide, Tat peptide), alkylating agents, phosphate, amino, mercapto, PEG (e.g., PEG-40K), MPEG, [MPEG]2, polyamino, alkyl, substituted alkyl, radiolabeled markers, enzymes, haptens (e.g. biotin), transport/absorption facilitators (e.g., aspirin, vitamin E, folic acid), synthetic ribonucleases (e.g., imidazole, bisimidazole, histamine, imidazole clusters, acridine-imidazole conjugates, Eu³⁺ complexes of tetraazamacrocycles), dinitrophenyl, HRP, or AP.

The ligands included in the compositions and/or methods of the present invention may be proteins, such as glycoproteins or peptides, such as molecules having a specific affinity for co-ligands, or antibodies, such as antibodies that bind to specific cell types such as cancer cells, endothelial cells, cardiomyocytes or osteocytes. Ligands useful in embodiments of compositions and/or methods of the present invention may be hormones or hormone receptors. Ligands useful in the embodiments of compositions and/or methods of the present invention may be lipids, lectins, carbohydrates, vitamins, coenzymes, multivalent lactose, multivalent galactose, N-acetyl-galactosamine, N-acetyl-glucosamine multivalent mannose, or multivalent fucose. Ligands useful in embodiments of compositions and/or methods of the present invention may be substances that increase the uptake of LPA dsRNA agent into cells, for example, by destroying the cytoskeleton of the cells (e.g., by destroying microtubules, microfilaments, and/or intermediate filaments of the cells). Non-limiting examples of such agents are taxon, vincristine, vinblastine, cytochalasin, nocodazole, japlakinolide, latrunculin A, phalloidin, swinholide A, indanocine and myoservin.

In some embodiments, ligands linked to the LPA dsRNA agent of the present invention are used as pharmacokinetic (PK) modulators. Examples of PK modulators that may be used in compositions and methods of the present invention include, but are not limited to, lipophilic agents, bile acids, steroids, phospholipid analogues, peptides, protein binders, PEG, vitamins, cholesterol, fatty acids, cholic acids, lithocholic acids, dialkylglycerides, diacylglycerides, phospholipids, sphingolipids, naproxen, ibuprofen, vitamin E, biotin, aptamers that bind to serum proteins, and the like. It is also known that oligonucleotides comprising many phosphorothioate linkages bind to serum proteins, and therefore, short oligonucleotides comprising a plurality of phosphorothioate linkages in the backbone, such as oligonucleotides of about 5 bases, 10 bases, 15 bases, or 20 bases, can also be used as ligands in compositions and/or methods of the present invention.

### LPA dsRNA agent composition

In some embodiments of the present invention, LPA dsRNA agents are in compositions. Compositions of the present invention may comprise one or more LPA dsRNA agents and optionally one or more pharmaceutically acceptable carriers, delivery agents, targeting agents, detectable labels, etc. Non-limiting examples of targeting agents available according to some embodiments of the method of the present invention are agents that direct the LPA dsRNA agents of the present invention to and/or into cells to be treated. The choice of targeting agents will depend on the following factors: the nature of the LPA-related disease or condition, and the type of the target cell. In one non-limiting example, in some embodiments of the present invention, it may be necessary to target LPA dsRNA agent to and/or into hepatocytes. It will be appreciated that in some embodiments of the method of the present invention, the therapeutic agent comprises an LPA dsRNA agent having only a delivery agent, such as a delivery agent comprising N-acetylgalactosamine (GalNAc), without any additional linking elements. For example, in some aspects of the present invention, the LPA dsRNA agent may be linked to a delivery compound comprising GalNAc and included in a composition comprising a pharmaceutically acceptable carrier and administered to a cell or subject without any detectable label or targeting agent or the like linked to the LPA dsRNA agent.

In the case where the LPA dsRNA agent of the present invention is administered together with and/or linked to one or more delivery agents, targeting agents, labeling agents, etc., one skilled in the art is able to understand and to select and use suitable agents for use in the method of the present invention. Labeling agents may be used in certain methods of the present invention to determine the location of LPA dsRNA agent in cells and tissues, and may be used to determine the location of therapeutic compositions comprising LPA dsRNA agents that have been administered in the methods of the present invention in cells, tissues or organs. Means of attaching and using labeling agents such as enzyme labels, dyes, radioactive labels, etc. are well known in the art. It will be appreciated that in some embodiments of the compositions and methods of the present invention, the labeling reagent is linked to one or both of the sense polynucleotides and the antisense polynucleotides included in the LPA dsRNA agent.

### Delivery of LPA dsRNA agent and LPA antisense polynucleotide agent

Some embodiments of the method of the present invention include delivering LPA dsRNA agent into cells. As used herein, the term "delivery" refers to promoting or affecting cellular uptake or absorption. Absorption or uptake of the LPA dsRNA agent may occur by an independent diffusion or active cellular process, or by using a delivery agent, a targeting agent, or the like that may be associated with the LPA dsRNA agent of the present invention. Delivery manners suitable for the method of the present invention include, but are not limited to, in vivo delivery, where LPA dsRNA agents are injected into tissue sites or administered systemically. In some embodiments of the present invention, the LPA dsRNA agent is linked to the delivery agent.

Non-limiting examples of methods that can be used to deliver LPA dsRNA agents to cells, tissues, and/or subjects include LPA dsRNA-GalNAc conjugates, SAMiRNA technology, LNP-based delivery methods, and nake RNA delivery. These and other delivery methods have been successfully used in the art to deliver therapeutic RNAi agents to treat various diseases and conditions, such as, but not limited to, liver diseases, acute intermittent porphyria (AIP), hemophilia, pulmonary fibrosis, and the like. Details of multiple delivery manners can be found in publications such as: Nikam, R.R. & K. R. Gore (2018) Nucleic Acid Ther, 28 (4), 209-224 Aug 2018; Springer A.D. & S.F. Dowdy (2018) Nucleic Acid Ther. Jun 1; 28(3): 109-118; Lee, K. et al., (2018) Arch Pharm Res, 41(9), 867-874; and Nair, J.K. et al., (2014) J. Am. Chem. Soc. 136:16958-16961, the contents of which are incorporated herein by reference.

Some embodiments of the present invention include the use of lipid nanoparticles (LNPs) to deliver the LPA dsRNA agent of the present invention to cells, tissues, and/or subjects. LNPs are commonly used for in vivo delivery of LPA dsRNA agents, including therapeutic LPA dsRNA agents. One advantage of using LNP or other delivery agents is that the stability of the LPA RNA agent is increased when delivered to the subject using LNP or other delivery agents. In some embodiments of the present invention, the LNP comprises a cationic LNP loaded with one or more LPA RNAi molecules of the present invention. LNPs comprising LPA RNAi molecules are administered to the subject, and LNPs and their attached LPA RNAi molecules are taken up by cells through endocytosis. Their presence leads to the release of molecules triggering RNAi, thereby mediating RNAi.

Another non-limiting example of a delivery agent that may be used in embodiments of the present invention to deliver the LPA dsRNA agent of the present invention to cells, tissues, and/or subjects is an agent comprising GalNAc that is linked to the LPA dsRNA agent of the present invention and delivers the LPA dsRNA agent to the cells, the tissues, and/or the subjects. Examples of certain other delivery agents comprising GalNAc that may be used in certain embodiments of the methods and compositions of the present invention are disclosed in PCT application WO2020191183A1. Non-limiting examples of GalNAc targeting ligands that can be used in compositions and methods of the present invention to deliver LPA dsRNA agent to cells are targeting ligand clusters. Examples of targeting ligand clusters provided herein are GalNAc ligands with phosphodiester bonds (GLO) and GalNAc ligands with phosphorothioate bonds (GLS). The term "GLX-n" may be used herein to indicate that the GalNAC-containing compound linked is any of the following compounds: GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16, each of which has a structure as shown below. In the figure below, the linking position of the GalNAc targeting ligand to the RNAi agent of the present invention is on the far right of each targeting ligand. It will be appreciated that any RNAi and dsRNA molecules of the present invention can be linked to GLS-1, GLS-2, GLS-3, GLS-4, GLS-5, GLS-6, GLS-7, GLS-8, GLS-9, GLS-10, GLS-11, GLS-12, GLS-13, GLS-14, GLS-15, GLS-16, GLO-1, GLO-2, GLO-3, GLO-4, GLO-5, GLO-6, GLO-7, GLO-8, GLO-9, GLO-10, GLO-11, GLO-12, GLO-13, GLO-14, GLO-15, and GLO-16, the following are the structures of GLO-1 to GLO-16 and GLS-1 to GLS-16.

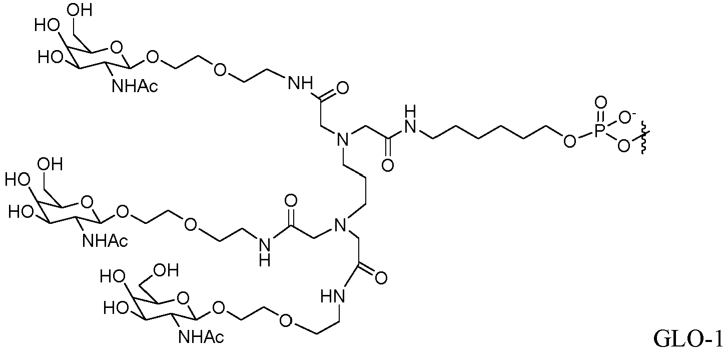

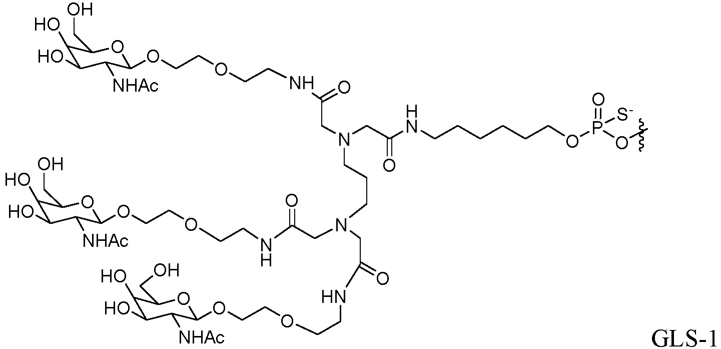

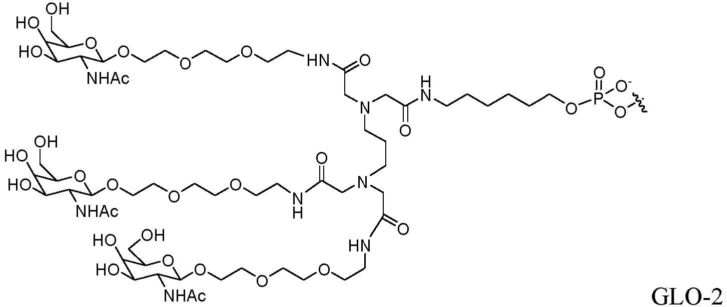

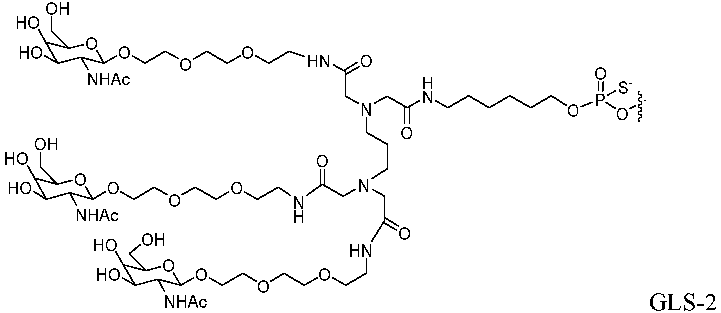

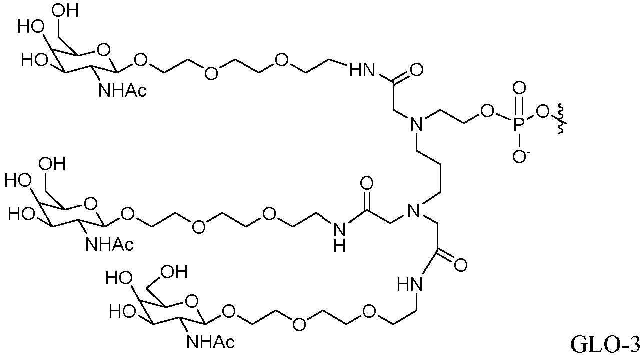

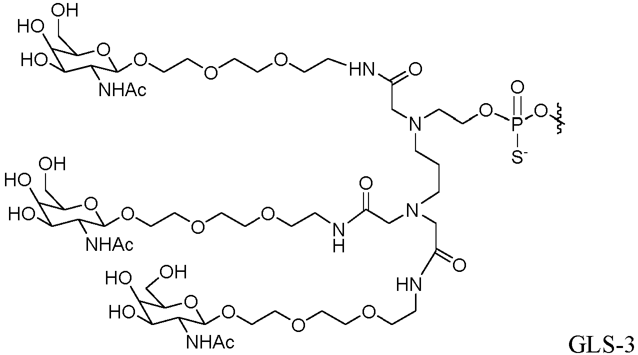

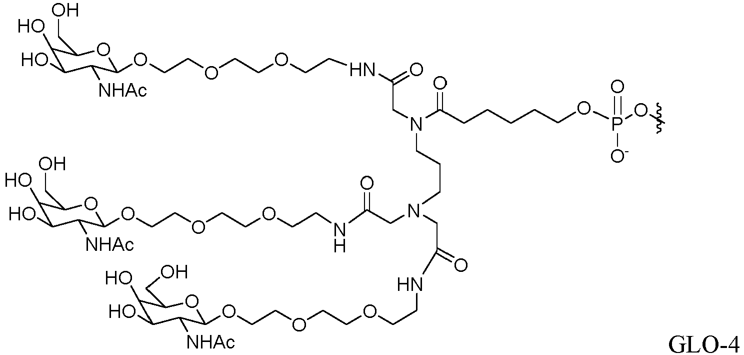

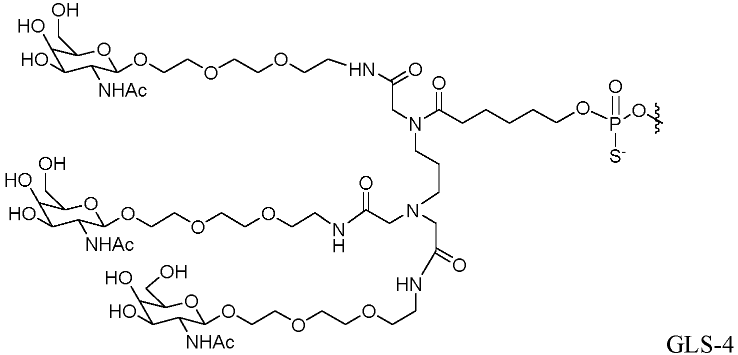

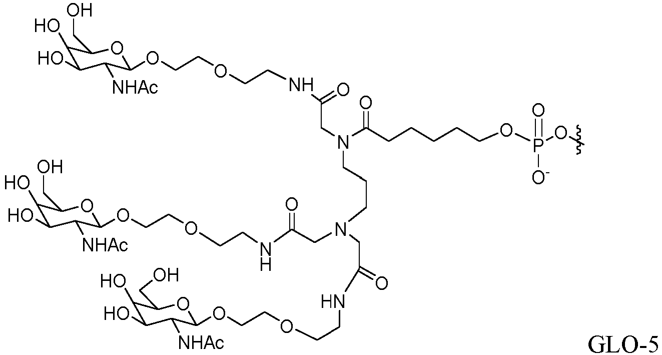

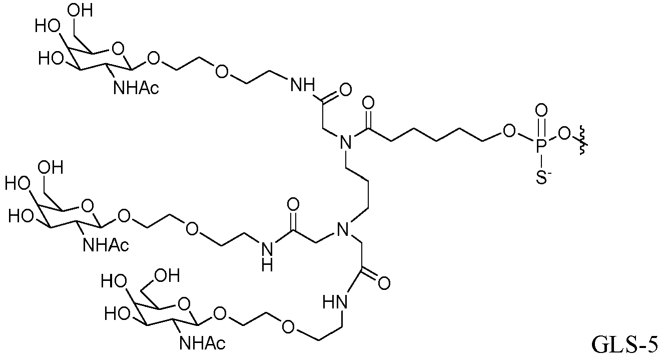

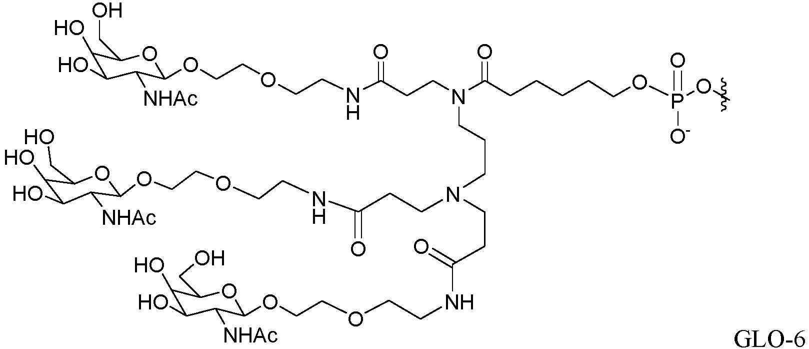

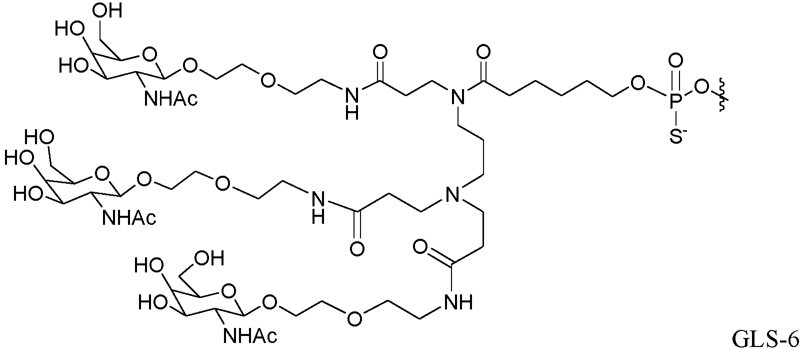

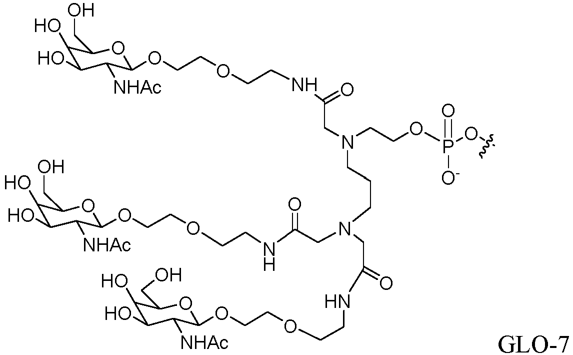

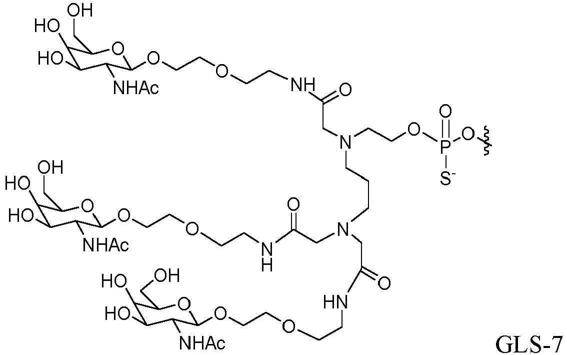

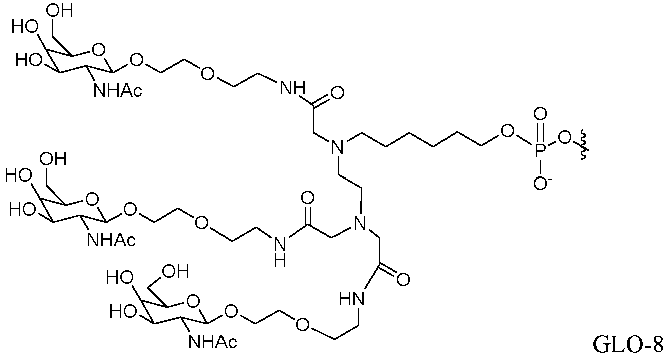

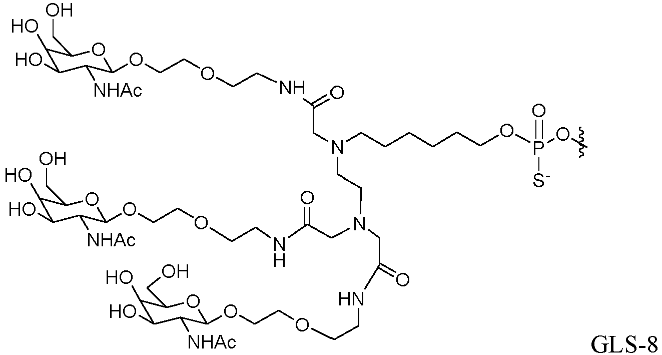

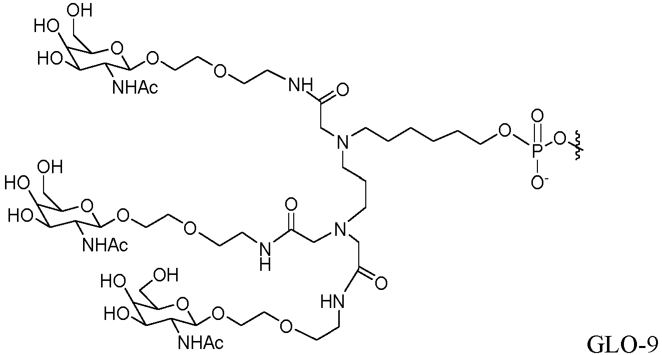

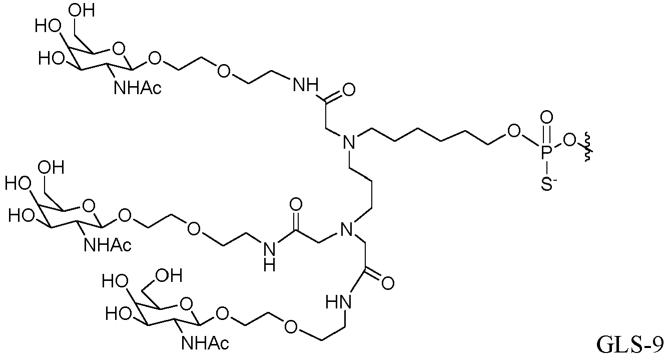

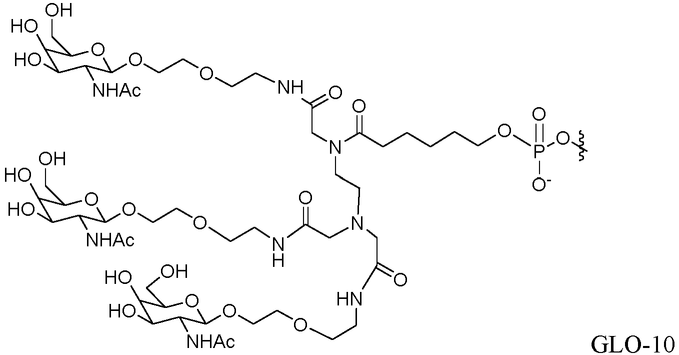

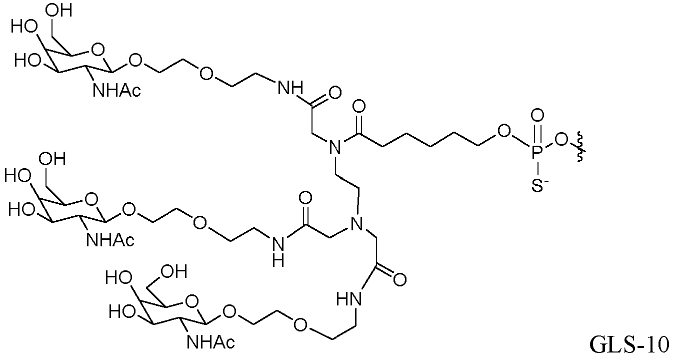

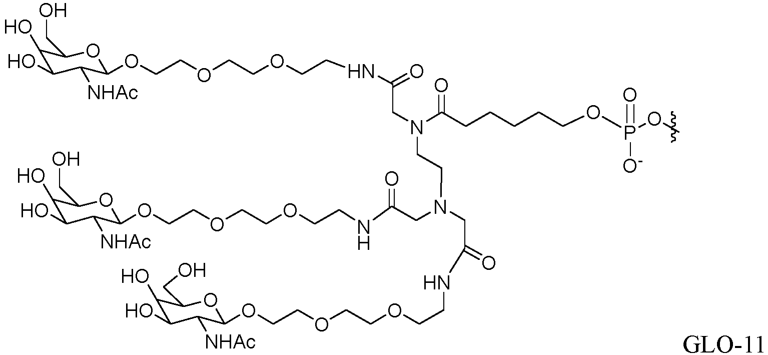

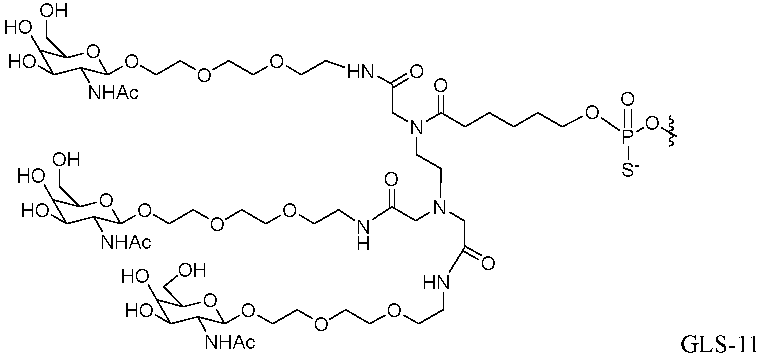

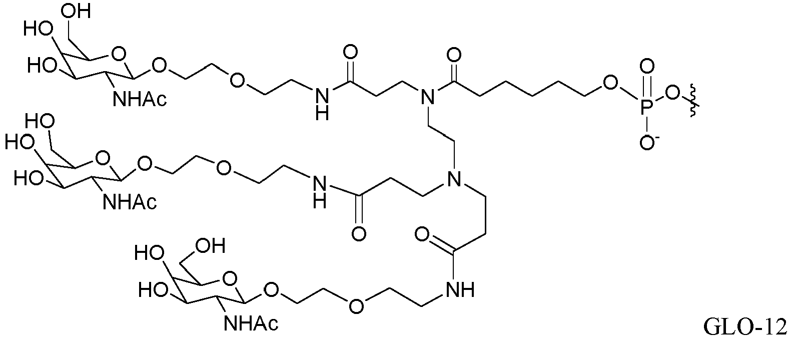

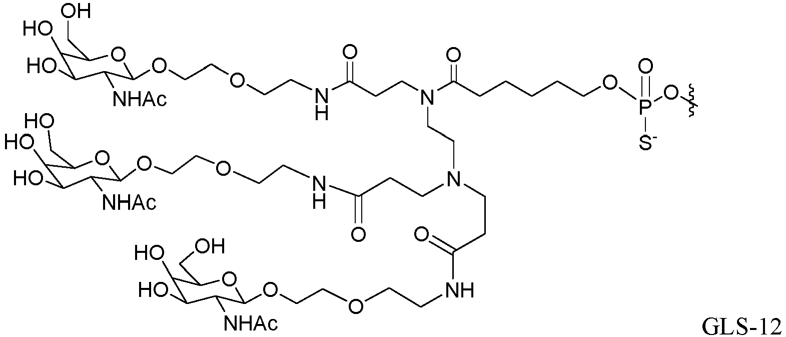

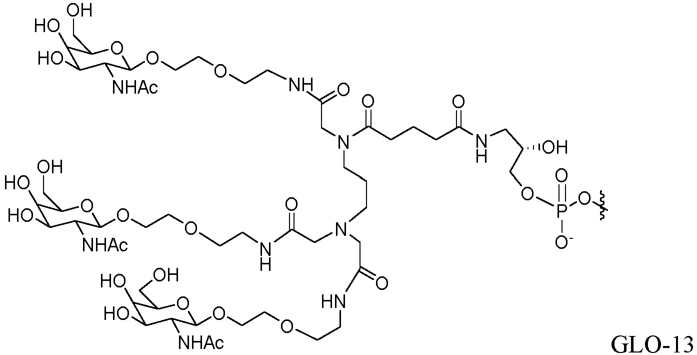

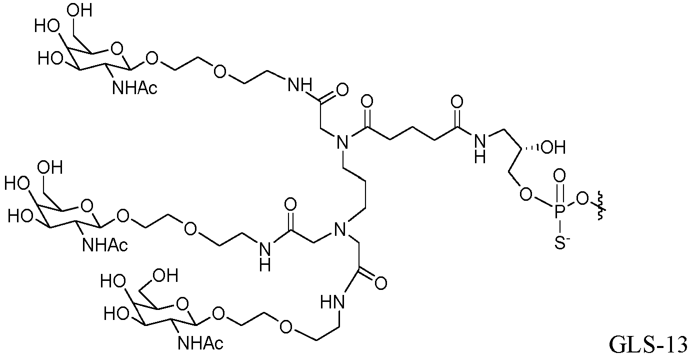

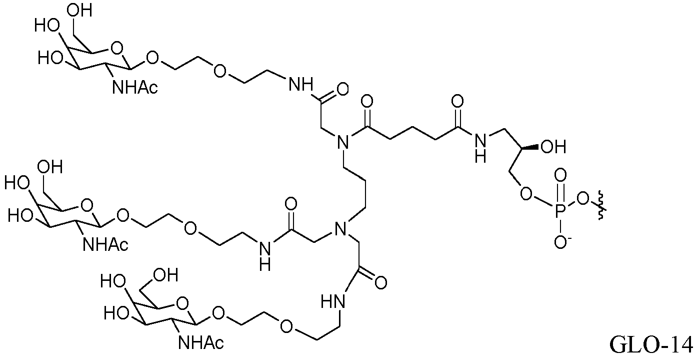

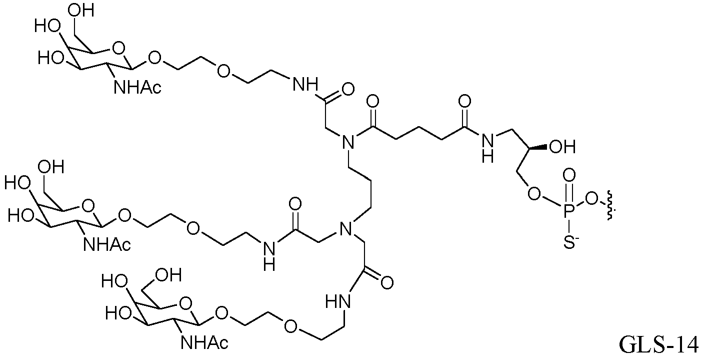

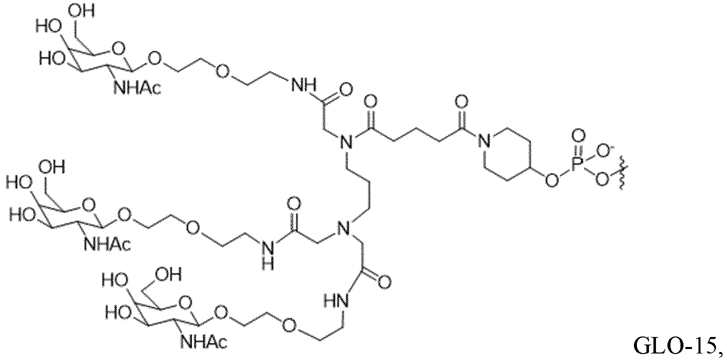

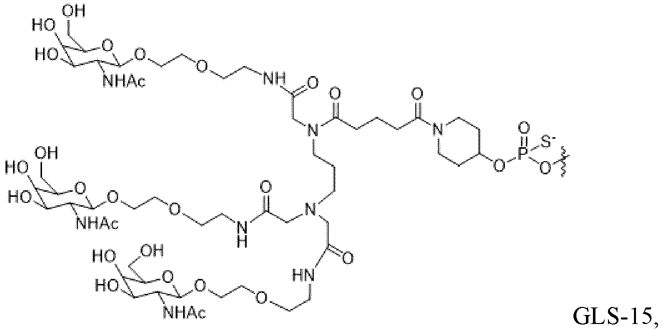

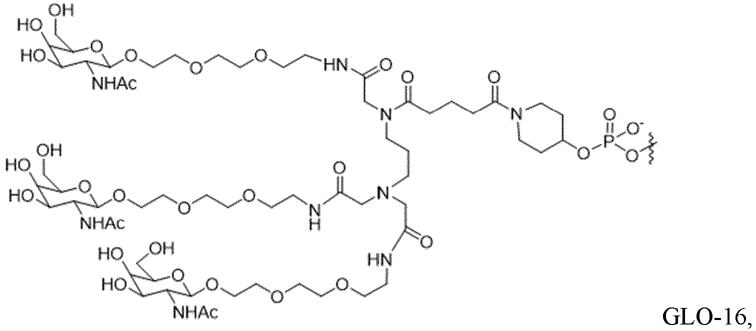

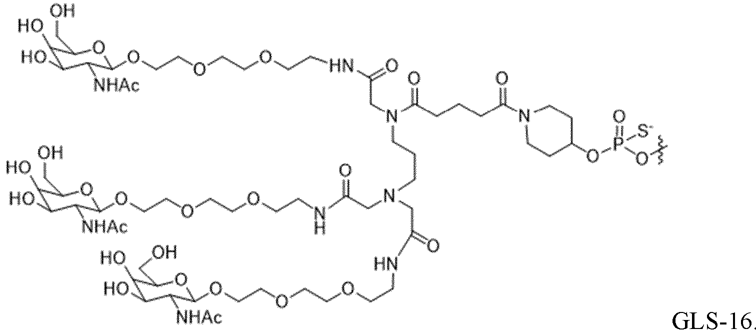

In some embodiments of the present invention, in vivo delivery may also be by beta-dextran delivery systems, such as those described in U.S. Pat. No. 5,032,401 and 5,607,677, and U.S. Publication No. 2005/0281781, the entirety of which is incorporated herein by reference. LPA RNAi agents may also be introduced into cells in vitro using methods known in the art, such as electroporation and lipid transfection. In some embodiments of the method of the present invention, LPA dsRNA is delivered without a targeting agent. These RNAs can be delivered as "naked" RNA molecules. As a non-limiting example, the LPA dsRNA of the present invention can be administered to a subject in a pharmaceutical composition comprising an RNAi agent but not a targeting agent (e.g., a GalNAc targeting compound) to treat an LPA-related disease or condition in the subject, such as a cardiovascular disease, wherein the cardiovascular disease includes Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles.

It will be appreciated that, in addition to certain delivery manners described herein, other RNAi delivery modes may be used in conjunction with embodiments of LPA RNAi agents and therapeutic methods described herein, such as, but not limited to, those described herein and those used in the art.

The LPA dsRNA agent of the present invention can be administered to the subject in an amount and manner that effectively reduces the level of the LPA polypeptide in the cell and/or the subject. In some embodiments of the method of the present invention, one or more LPA dsRNA agents are administered to cells and/or subjects to treat diseases or conditions associated with LPA expression. In some embodiments, the method of the present invention includes administering one or more LPA dsRNA agents to a subject in need thereof to alleviate diseases or conditions associated with LPA expression in the subject. The LPA dsRNA agent or the LPA antisense polynucleotide agent of the present invention may be administered to reduce LPA expression in one or more of the cells in vitro, ex vivo and in vivo.

In some embodiments of the present invention, the level of LPA polypeptides in cells is reduced by delivery (e.g., introduction) of LPA dsRNA agents or LPA antisense polynucleotide agents into the cells. Targeting agents and methods may be used to facilitate delivery of LPA dsRNA agents or LPA antisense polynucleotide agents to specific cell types, cell subtypes, organs, spatial regions, and/or subcellular regions within cells within the subject. The LPA dsRNA agent may be administered alone or in combination with one or more additional LPA dsRNA agents in certain methods of the present invention. In some embodiments, 2, 3, 4 or more independently selected LPA dsRNA agents are administered to the subject. In some embodiments of the present invention, LPA dsRNA agents are administered to a subject in combination with one or more additional therapeutic regimens for treating an LPA-related disease or condition to treat the LPA-related disease or condition. Other non-limiting examples of therapeutic regimens are administration of one or more LPA antisense polynucleotides of the present invention, administration of non-LPA dsRNA therapeutic agents, and behavioral modifications. Additional therapeutic regimens may be administered at one or more of the following times: before, simultaneously with, and after administration of the LPA dsRNA agent of the present invention. It will be appreciated that "simultaneously" as used herein means within 5 minutes of zero time, within 10 minutes of zero time, within 30 minutes of zero time, within 45 minutes of zero time, and within 60 minutes of zero time, wherein "zero time" is the time at which the LPA dsRNA agent of the present invention is administered to the subject. Non-limiting examples of non-LPA dsRNA therapeutic agents are additional therapeutic agents such as a HMg Co-A reductase inhibitor (statins), ezetimibe, a PCSK-9 inhibitor, a CTEP inhibitor, an ANGPTL3-targeting therapy, an APOC3-targeting therapy and niacin, or any combination thereof. Non-limiting examples of behavioral modifications are: dietary regimens, counseling and exercise regimens. These and other therapeutic agents and behavioral modifications are known in the art and may be used to treat an LPA disease or condition of a subject, and may also be combined with one or more LPA dsRNA agents of the present invention to be administered to the subject to treat the LPA disease or condition. The LPA dsRNA agent of the present invention administered to a cell or subject to treat an LPA-related disease or condition may act in a synergistic manner with one or more other therapeutic agents or active ingredients, thereby increasing the effectiveness of the one or more therapeutic agents or active ingredients and/or increasing the effectiveness of the LPA dsRNA agent in treating the LPA-related disease or condition.

Therapeutic method of the present invention includes administration of an LPA dsRNA agent that may be used prior to the onset of an LPA-related disease or condition and/or when an LPA-related disease or condition is present, including in the early, middle, late stages of the disease or condition and at all times before and after any of these stages. The method of the present invention can also treat a subject who has been previously treated with one or more other therapeutic agents and/or therapeutically active ingredients for an LPA-related disease or condition, where the one or more other therapeutic agents and/or therapeutically active ingredients are unsuccessful, minimally successful, and/or no longer successful in treating the LPA-related disease or condition of the subject.

### Vector-encoded dsRNA

In some embodiments of the present invention, the LPA dsRNA agent may be delivered into cells using a vector. LPA dsRNA agent transcription units may be contained in DNA or RNA vectors. The preparation and use of such transgene-encoding vectors for delivery of sequences into cells and/or subjects is well known in the art. Vectors resulting in transient expression of LPA dsRNA, for example for at least 1, 2, 3, 4, 5, 6, 7, 8, 9, 10 h or more, 1, 2, 3, 4, 5, 6, 7, 8, 9, 10 weeks or more, may be used in the method of the present invention. The length of transient expression can be determined using conventional methods based on factors such as, but not limited to, selected specific vector constructs and target cells and/or tissues. Such transgenes can be introduced as linear constructs, cyclic plasmids or viral vectors, which can be integrated or non-integrated vectors. Transgenes can also be constructed to be inherited as extrachromosomal plasmids (Gassmann, et al., Proc. Natl. Acad. Sci. USA (1995) 92: 1292).

One or more single strands of the LPA dsRNA agent can be transcribed from a promoter on the expression vector. In cases where two individual strands are to be expressed to produce, for example, dsRNA, two individual expression vectors can be co-introduced into the cell using, for example, transfection or infection. In certain embodiments, each individual strand of the LPA dsRNA agent of the present invention may be transcribed by a promoter contained on the same expression vector. In some embodiments of the present invention, the LPA dsRNA agent is expressed as a reverse repeat polynucleotide linked by a linker polynucleotide sequence such that the LPA dsRNA agent has a stem-loop structure.

Non-limiting examples of RNA expression vectors are DNA plasmids or viral vectors. Expression vectors useful in embodiments of the present invention may be compatible with eukaryotic cells. Eukaryotic cell expression vectors are conventionally used in the art and are available from many commercial sources. Delivery of the LPA dsRNA expression vector may be systemic, such as by intravenous or intramuscular administration, by administration to target cells removed from the subject and then reintroduction of the target cells into the subject, or by any other means that allows for the introduction of desired target cells.

Viral vector systems that may be included in embodiments of the methods include, but are not limited to, (a) adenovirus vectors; (b) retrovirus vectors, including but not limited to lentiviral vectors, moloney murine leukemia virus, etc.; (c) adeno-associated virus vectors; (d) herpes simplex virus vectors; (e) SV 40 vectors; (f) polyoma virus vectors; (g) papilloma virus vectors; (h) picornavirus vectors; (i) pox virus vectors such as orthopox virus vectors, such as vaccinia virus vectors or avipox virus vectors, such as canary pox or fowl pox virus vectors; (j) helper-dependent or gutless adenovirus vectors. Constructs for recombinant expression of LPA dsRNA agents may comprise regulatory elements, such as promoters, enhancers, etc., which may be selected to provide constitutive or regulatory/inducible expression. The use of viral vector systems and promoters and enhancers and the like are conventional in the art and may be used in conjunction with the methods and compositions described herein.

Some embodiments of the present invention include delivering LPA dsRNA agents into cells using viral vectors. Many adenovirus-based delivery systems are conventionally used in the art for delivery to, for example, lungs, liver, central nervous system, endothelial cells and muscles. Non-limiting examples of viral vectors that may be used in the method of the present invention are: AAV vectors, pox viruses such as vaccinia viruses, modified Ankara viruses (MVA), NYVAC, avipox such as avipox viruses or canary pox viruses.

Certain embodiments of the present invention include methods for delivering LPA dsRNA agents into cells using vectors, and such vectors may be in pharmaceutically acceptable carriers that may, but do not necessarily, comprise a sustained release matrix in which a gene delivery vector is embedded. In some embodiments, the vector for delivering the LPA dsRNA may be produced from recombinant cells, and the pharmaceutical composition of the present invention may comprise one or more cells that produce the LPA dsRNA delivery system.

### Pharmaceutical composition comprising LPA dsRNA or ssRNA agent

Certain embodiments of the present invention include the use of a pharmaceutical composition comprising an LPA dsRNA agent or an LPA antisense polynucleotide agent and a pharmaceutically acceptable carrier. The pharmaceutical composition comprising the LPA dsRNA agent or the LPA antisense polynucleotide agent may be used in the method of the present invention to reduce LPA gene expression in cells and may be used in the treatment of LPA-related diseases or conditions. Such a pharmaceutical composition may be formulated based on the delivery manner. Non-limiting examples of formulations for delivery manners are compositions formulated for subcutaneous delivery, compositions formulated for systemic administration by parenteral delivery, compositions formulated for intravenous (IV) delivery, compositions formulated for intrathecal delivery, compositions formulated for direct delivery into the brain, and the like. A pharmaceutical composition of the present invention may be administered using one or more manners to deliver an LPA dsRNA agent or an LPA antisense polynucleotide agent into a cell, and the manners is for example via surface (e.g., by a transdermal patch agent); lung, such as by inhalation or insufflation of powder or aerosol, including through a nebulizer; intra-airway, intranasal, epidermal and transdermal, oral or parenteral. Parenteral administration includes intravenous, intraarterial, subcutaneous, intraperitoneal or intramuscular injection or infusion; subepidermal, e.g. by an implantation device; or intracranial, e.g. through intraparenchymal; intrathecal or intraventricular administration. LPA dsRNA agents or LPA antisense polynucleotide agents can also be delivered directly to target tissues, for example, directly to the liver, directly to the kidney, and the like. Understandably, "delivery of LPA dsRNA agent" or "delivery of LPA antisense polynucleotide agent" into a cell includes, respectively, delivery of LPA dsRNA agent or LPA antisense polynucleotide agent, expression of LPA dsRNA agent directly in a cell and expression of LPA dsRNA agent from a coding vector delivered into a cell, or any suitable manner of making LPA dsRNA or LPA antisense polynucleotide agent appear in a cell. Preparation and use of formulations and means for delivering inhibitory RNA are well known and conventionally used in the art.

As used herein, the "pharmaceutical composition" comprises a pharmacologically effective amount of the LPA dsRNA agent or LPA antisense polynucleotide agent of the present invention and a pharmaceutically acceptable carrier. The term "pharmaceutically acceptable carrier" refers to a carrier used to administer a therapeutic agent. Such a carrier includes, but are not limited to, brine, buffered brine, glucose, water, glycerin, ethanol, and combinations thereof. The term explicitly excludes cell culture media. For drugs administered orally, pharmaceutically acceptable carriers include, but are not limited to, pharmaceutically acceptable excipients, such as inert diluents, disintegrants, binders, lubricants, sweeteners, flavorings, colorants, and preservatives. Suitable inert diluents comprise sodium carbonate and calcium carbonate, sodium phosphate and calcium phosphate, and lactose, while corn starch and alginate are suitable disintegrants. The binders may comprise starch and gelatin, while the lubricants, if present, are usually magnesium stearate, stearic acid or talc. If desired, the tablet may be coated with a material such as glyceryl monostearate or glyceryl distearate to delay absorption in the gastrointestinal tract. Reagents included in pharmaceutical formulations are further described below. The terms used herein, such as "pharmacologically effective amount", "therapeutically effective amount" and "effective amount", refer to the amount at which the LPA dsRNA agent or the LPA antisense polynucleotide agent of the present invention produces the desired pharmacological, therapeutic or prophylactic results. For example, if a given clinical treatment is considered effective when a measurable parameter associated with a disease or disorder is reduced by at least 10%, then the therapeutically effective amount of a drug for treating the disease or condition is the amount required to reduce the parameter by at least 10%. For example, therapeutically effective amounts of LPA dsRNA agent or LPA antisense polynucleotide agent may reduce LPA polypeptide levels by at least 10%. The pharmaceutical composition may comprise such a dsRNAi agent comprising, for example, the duplex shown in Table 1. In some other embodiments, such a dsRNAi agent comprises a variant of the duplex in Table 1.

### Effective amount

In some aspects, the method of the present invention comprises contacting cells with an effective amount of LPA dsRNA agent or LPA antisense polynucleotide agent to reduce LPA gene expression in the contacted cells. Some embodiments of the method of the present invention comprises administering an LPA dsRNA agent or an LPA antisense polynucleotide agent to a subject in an amount that effectively reduces LPA gene expression and treats an LPA-related disease or condition of the subject. For the purposes of reducing the expression of LPA and/or for the treatment of LPA-related diseases or conditions, the "effective amount" used is the amount necessary or sufficient to achieve the desired biological effect. For example, the effective amount of an LPA dsRNA agent or an LPA antisense polynucleotide agent for the treatment of an LPA-related disease or condition may: (i) be the amount required to slow or stop the progression of the disease or condition; (ii) reverse, reduce or eliminate one or more symptoms of a disease or condition. In some aspects of the present invention, the effective amount is the amount of an LPA dsRNA agent or an LPA antisense polynucleotide agent that, when administered to a subject requiring treatment of an LPA-related disease or condition, results in a therapeutic response of prevention and/or treatment of the disease or condition. According to some aspects of the present invention, the effective amount is the amount of an LPA dsRNA agent or an LPA antisense polynucleotide agent of the present invention that, when combined with or co-administered with another therapeutic treatment for an LPA-related disease or condition, results in a therapeutic response of prevention and/or treatment of the disease or condition. In some embodiments of the present invention, the biological effect of treating a subject with an LPA dsRNA agent or an LPA antisense polynucleotide agent of the present invention may be the improvement and/or complete elimination of symptoms caused by an LPA-related disease or condition. In some embodiments of the present invention, the biological effect is the complete elimination of an LPA-related disease or condition, as evidenced, for example, by a diagnostic test indicating that the subject does not have an LPA-related disease or condition. Non-limiting examples of detectable physiological symptoms include a reduction in lipid accumulation in the subject's liver after administration of the agent of the present invention. Other ways of assessing the state of an LPA-related disease or condition known in the art may be used to determine the effect of the agents and/or methods of the present invention on an LPA-related disease or condition.

Effective amounts of an LPA dsRNA agent or an LPA antisense polynucleotide agent that reduce LPA polypeptides to levels for treatment of LPA-related diseases or conditions are generally determined in clinical trials that establish effective doses for test population and control population in blinded studies. In some embodiments, the effective amount is an amount that results in a desired response, such as an amount that reduces the LPA-related disease or condition in cells, tissues, and/or subjects with the disease or condition. Thus, an effective amount of an LPA dsRNA agent or LPA antisense polynucleotide agent for treatment of an LPA-related disease or condition that may be treated by reducing the LPA polypeptide may be an amount that, when administered, reduces the amount of the LPA polypeptide in a subject to less than the amount that would be present in cells, tissues and/or the subject without administration of the LPA dsRNA agent or LPA antisense polynucleotide agent. In some aspects of the present invention, the levels of LPA polypeptide and/or LPA gene expression present in cells, tissues and/or subjects not contacted with to or administered with the LPA dsRNA agent or LPA antisense polynucleotide agent of the present invention are referred to as "control" amounts. In some embodiments of the inventive method, a control amount for a subject is the amount for the subject before treatment; in other words, the level for the subject before administration of the LPA agent may be the control level for the subject and used for comparison to LPA polypeptide and/or LPA gene expression level after the administration of the siRNA to the subject. In the case of treatment for an LPA-related disease or condition, the desired response may be to reduce or eliminate one or more symptoms of the disease or condition in cells, tissues, and/or the subject. The reduction or elimination can be temporary or permanent. It will be appreciated that the state of LPA-related diseases or conditions may be monitored using methods such as determining LPA polypeptides and LPA gene expression, symptom assessment, clinical test, and the like. In some aspects of the present invention, the desired response to treatment of an LPA-related disease or condition is to delay the onset of the disease or condition or even prevent the onset of the disease or condition.

The effective amount of the compound that reduces the LPA polypeptide can also be determined by evaluating the physiological effect of the administration of the LPA dsRNA agent or the LPA antisense polynucleotide agent on the cell or subject, such as the reduction of LPA-related diseases or conditions after administration. The assay and/or symptom monitoring of the subject may be used to determine the efficacy of the LPA dsRNA agent or LPA antisense polynucleotide agent of the present invention, which may be administered in the pharmaceutical compound of the present invention, and to determine whether there is a response to treatment. One non-limiting example is one or more serum lipid profile tests known in the art. Another non-limiting example is that one or more liver function tests known in the art may be used to determine the state of the LPA-related disease or condition of the subject before and after treating the subject with the LPA dsRNA agent of the present invention. In another non-limiting example, one or more cholesterol accumulation tests in the liver known in the art are used to determine the state of the LPA-related disease in the subject. In this embodiment, the disease includes cholesterol accumulation and the test is used to determine cholesterol levels in the subject before and after treating the subject with the LPA dsRNA agent of the present invention.

Some embodiments of the present invention include a method of determining the efficacy of a dsRNA agent or an LPA antisense polynucleotide agent of the present invention administered to a subject to treat an LPA-related disease or condition by evaluating and/or monitoring one or more "physiological characteristics" of the LPA-related disease or condition in the subject. Non-limiting examples of physiological characteristics of an LPA-related disease or condition are the serum LPA level of the subject, the serum lipid level of the subject, the low-density lipoprotein level of the subject, the HDL level of the subject, the LDL: HDL ratio of the subject, the triglyceride level of the subject, fat present in the liver of the subject, physical symptoms, etc. Standard methods for determining such physiological characteristics are known in the art and include, but are not limited to, blood tests, imaging studies, physical examinations, and the like.

It will be appreciated that the amount of an LPA dsRNA agent or an LPA antisense polynucleotide agent administered to the subject may be modified based at least in part on the determination results of the disease and/or condition state and/or the physiological characteristics of the subject. The therapeutic amount may be changed by, for example, increasing or decreasing the amount of an LPA dsRNA agent or an LPA antisense polynucleotide agent by changing the composition in which the LPA dsRNA agent or the LPA antisense polynucleotide agent is administered, by changing the route of administration, by changing the time of administration, or the like. An effective amount of an LPA dsRNA agent or an LPA antisense polynucleotide agent will vary with the particular condition being treated, the age and physical condition of a subject being treated, the severity of the condition, the duration of the treatment, the nature of the co-treatment (if any), the specific route of administration, and other factors within the knowledge and expertise of the health practitioner. For example, the effective amount may depend on the level of the LPA polypeptide and/or the desired level of LPA gene expression that is effective for the treatment of LPA-related diseases or conditions. A skilled person can empirically determine the effective amount of a particular LPA dsRNA agent or LPA antisense polynucleotide agent for use in the method of the present invention without excessive experimentation. In combination with the teachings provided herein, an effective prophylactic or therapeutic regimen may be planned to effectively treat a particular subject by selecting from a plurality of LPA dsRNA agents or LPA antisense polynucleotide agents of the present invention and weighing factors such as efficacy, relative bioavailability, patient weight, severity of adverse side effects, and preferred mode of administration. As used in embodiments of the present invention, the effective amount of the LPA dsRNA agent or the LPA antisense polynucleotide agent of the present invention may be the amount that produces the desired biological effect in cells when in contact with the cells.

It should be recognized that LPA gene silencing can be performed constitutively or by genome engineering in any cell expressing LPA and determined by any suitable assay. In some embodiments of the present invention, LPA gene expression is reduced by at least 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, or 100% by administration of the LPA dsRNA agent of the present invention. In some embodiments of the present invention, LPA gene expression is reduced by 5% to 10%, 5% to 25%, 10% to 50%, 10% to 75%, 25% to 75%, 25% to 100% or 50% to 100% by administration of the LPA dsRNA agent of the present invention.

### Mode of

The LPA dsRNA agent and LPA antisense polynucleotide agent are delivered in a pharmaceutical composition at a dose sufficient to inhibit LPA gene expression. In certain embodiments of the present invention, a dose of an LPA dsRNA agent or LPA antisense polynucleotide agent is 0.01 to 200.0 mg per kg of body weight of a subject per day, generally 1 to 50 mg/kg body weight, 5 to 40 mg/kg body weight, 10 to 30 mg/kg body weight, 1 to 20 mg/kg body weight, 1 to 10 mg/kg body weight, 4 to 15 mg/kg body weight per day, including endpoint values. For example, the LPA dsRNA agent or LPA antisense polynucleotide agent may be administered at a single dose of about 0.01 mg/kg, 0.05 mg/kg, 0.1 mg/kg, 0.2 mg/kg, 0.3 mg/kg, 0.4 mg/kg, 0.5 mg/kg, 1 mg/kg, 1.1 mg/kg, 1.2 mg/kg, 1.3 mg/kg, 1.4 mg/kg, 1.5 mg/kg, 1.6 mg/kg, 1.7 mg/kg, 1.8 mg/kg, 1.9 mg/kg, 2 mg/kg, 2.1 mg/kg, 2.2 mg/kg, 2.3 mg/kg, 2.4 mg/kg, 2.5 mg/kg, 2.6 mg/kg, 2.7 mg/kg, 2.8 mg/kg, 2.9 mg/kg, 3.0 mg/kg, 3.1 mg/kg, 3.2 mg/kg, 3.3 mg/kg, 3.4 mg/kg, 3.5 mg/kg, 3.6 mg/kg, 3.7 mg/kg, 3.8 mg/kg, 3.9 mg/kg, 4 mg/kg, 4.1 mg/kg, 4.2 mg/kg, 4.3 mg/kg, 4.4 mg/kg, 4.5 mg/kg, 4.6 mg/kg, 4.7 mg/kg, 4.8 mg/kg, 4.9 mg/kg, 5 mg/kg, 5.1 mg/kg, 5.2 mg/kg, 5.3 mg/kg, 5.4 mg/kg, 5.5 mg/kg, 5.6 mg/kg, 5.7 mg/kg, 5.8 mg/kg, 5.9 mg/kg, 6 mg/kg, 6.1 mg/kg, 6.2 mg/kg, 6.3 mg/kg, 6.4 mg/kg, 6.5 mg/kg, 6.6 mg/kg, 6.7 mg/kg, 6.8 mg/kg, 6.9 mg/kg, 7 mg/kg, 7.1 mg/kg, 7.2 mg/kg, 7.3 mg/kg, 7.4 mg/kg, 7.5 mg/kg, 7.6 mg/kg, 7.7 mg/kg, 7.8 mg/kg, 7.9 mg/kg, 8 mg/kg, 8.1 mg/kg, 8.2 mg/kg, 8.3 mg/kg, 8.4 mg/kg, 8.5 mg/kg, 8.6 mg/kg, 8.7 mg/kg, 8.8 mg/kg, 8.9 mg/kg, 9 mg/kg, 9.1 mg/kg, 9.2 mg/kg, 9.3 mg/kg, 9.4 mg/kg, 9.5 mg/kg, 9.6 mg/kg, 9.7 mg/kg, 9.8 mg/kg, 9.9 mg/kg, 10 mg/kg, 11 mg/kg, 12 mg/kg, 13mg/kg, 14 mg/kg, 15 mg/kg, 16 mg/kg, 17 mg/kg, 18 mg/kg, 19 mg/kg, 20 mg/kg, 21 mg/kg, 22 mg/kg, 23mg/kg, 24 mg/kg, 25 mg/kg, 26 mg/kg, 27 mg/kg, 28 mg/kg, 29 mg/kg, 30 mg/kg, 31 mg/kg, 32 mg/kg, 33mg/kg, 34 mg/kg, 35 mg/kg, 36 mg/kg, 37 mg/kg, 38 mg/kg, 39 mg/kg, 40 mg/kg, 41 mg/kg, 42 mg/kg, 43mg/kg, 44 mg/kg, 45 mg/kg, 46 mg/kg, 47 mg/kg, 48 mg/kg, 49 mg/kg to 50 mg/kg body weight.

A variety of factors may be considered in determining the delivery dose and time of the LPA dsRNA agent of the present invention. The absolute amount of LPA dsRNA agents or LPA antisense polynucleotide agents delivered will depend on a variety of factors, including co-treatment, number of doses and individual subject parameters, including age, physical condition, physical size and body weight. These factors are well known to those of ordinary skill in the art and can be solved by conventional experiments. In some embodiments, a maximum dose, that is, the highest safe dose based on reasonable medical judgment, may be used.

In some embodiments, the method of the present invention may comprise administering 1, 2, 3, 4, 5, 6, 7, 8, 9, 10 or more doses of LPA dsRNA agents or LPA antisense polynucleotide agents to the subject. In some cases, a pharmaceutical compound (e.g., comprising an LPA dsRNA agent or comprising an LPA antisense polynucleotide agent) may be administered to a subject at a certain dose at least daily, every other day, weekly, every other week, monthly, etc., and may be administered once a day or more than once a day, e.g. 2, 3, 4, 5, or more times in a 24-hour cycle. The pharmaceutical composition of the present invention may be administered once a day; or an LPA dsRNA agent or an LPA antisense polynucleotide agent may be administered in two, three, or more subdoses at appropriate intervals within a day, or even delivered using continuous infusions or by controlled release formulations. In some embodiments of the method of the present invention, the pharmaceutical composition of the present invention is administered to the subject once or more daily, once or more weekly, once or more monthly, or once or more annually.

In certain aspects, the method of the present invention comprises administering a pharmaceutical compound alone or in combination with one or more other LPA dsRNA agents or LPA antisense polynucleotide agents, and/or in combination with other drug therapies or therapeutic activities or protocols administered to a subject with an LPA-related disease or condition. The pharmaceutical compound may be administered in the form of a pharmaceutical composition. The pharmaceutical composition used in the method of the present invention may be sterile and comprises an amount of LPA dsRNA agent or LPA antisense polynucleotide agent that reduces the level of the LPA polypeptide to a level sufficient to produce the desired response in a unit of weight or volume suitable for administration to the subject. The dose of the pharmaceutical composition comprising the LPA dsRNA agent or LPA antisense polynucleotide agent to be administered to the subject may be selected according to different parameters to reduce the LPA protein level, in particular according to the mode of administration used and the state of the subject. Other factors include the duration of treatment required. If the response of the subject is insufficient at the initial dose, a higher dose may be used within the tolerance of the patient (or the dose may be effectively increased by a different, more localized delivery route).

### Treatment

As used herein, the terms "prevent" or "preventing", when used in reference to a disease, condition, or disorder that would benefit from decreased LPA gene expression, refers to a reduction in the likelihood that a subject will experience a symptom related to such a disease, condition, or disorder, such as a cardiovascular disease, including Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles. In such cases, the likelihood of occurring such a disease is reduced, for example, when an individual has one or more cardiovascular disease risk factors, but does not develop a cardiovascular disease or only develops a cardiovascular disease of less severity, it is considered effective prevention if the individual fails to develop a related disease, condition or condition, or has a reduced degree of development of symptoms associated with such a disease, condition or condition (e.g., a reduction of at least about 10% on the scale of the disease or condition clinically), or a delayed presentation of symptoms (e.g., a delay of days, weeks, months or years), relative to a population with the same risk factors and without receiving the treatment described herein.

For an LPA-related disease and condition in which a reduction in the level of the LPA polypeptide is effective in treating the disease or condition, the method and the LPA dsRNA agent of the present invention may be used for treatment to inhibit LPA expression. Examples of diseases and conditions that can be treated with the LPA dsRNA agent or LPA antisense polynucleotide agent of the present invention and the therapeutic method of the present invention include, but are not limited to: Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles. Such a disease and condition may herein be referred to as "LPA-related disease and condition" and "disease and condition caused and/or regulated by LPA".

In some aspects of the present invention, the LPA dsRNA agent or the LPA antisense polynucleotide agent of the present invention may be administered to a subject at one or more times before or after diagnosis of an LPA-related disease or condition. In some aspects of the present invention, the subject is at risk of suffering from or developing an LPA-related disease or condition. Subject at risk of developing an LPA-related disease or condition are those with an increased likelihood of developing the LPA-related disease or condition compared to controls at risk of developing the LPA-related disease or condition. In some embodiments of the present invention, the level of the risk is statistically significant compared to the control level of the risk. Subjects at risk may include, for example: those who are or will be subjects with pre-existing diseases and/or genetic abnormalities that make the subjects more susceptible to an LPA-related disease or condition than control subjects without the pre-existing diseases or genetic abnormalities; subjects with a family and/or personal history of an LPA-related disease or condition; and subjects who have previously been treated for an LPA-related disease or condition. It will be appreciated that pre-existing diseases and/or genetic abnormalities that make the subject more susceptible to an LPA-related disease or condition may be diseases or genetic abnormalities that, when present, have previously been determined to be associated with a higher likelihood of developing the LPA-related disease or condition.

It will be appreciated that the LPA dsRNA agent or LPA antisense polynucleotide agent may be administered to a subject based on the medical condition of the individual subject. For example, the healthcare provided to the subject may evaluate the LPA level measured in the sample obtained from the subject and determine that it is desirable to reduce the LPA level of the subject by administering the LPA dsRNA agent or the LPA antisense polynucleotide agent of the present invention. In one non-limiting example, a biological sample, such as a blood or serum sample, may be obtained from the subject, and the LPA level of the subject may be determined in the sample. An LPA dsRNA agent or LPA antisense polynucleotide agent is administered to a subject, and a blood or serum sample is obtained from the subject after administration, and the LPA level is measured using the sample, and the result is compared to the result determined in the sample of the subject before administration (previous). Compared to the level before administration, the subsequent reduction of LPA level in the sample of the subject indicates the efficacy of the administered LPA dsRNA agent or LPA antisense polynucleotide agent in reducing the LPA level of the subject. In one non-limiting example, the level of Lp (a) in the blood may be considered a physiological characteristic of an LPA-related condition, even if the subject has not been diagnosed with the LPA-related condition, such as that disclosed herein. The healthcare provider may monitor changes in Lp(a) levels in the blood of the subject as a measure of the efficacy of the administered LPA dsRNA agent or LPA antisense polynucleotide agent of the present invention.

Some embodiments of the method of the present invention include adjusting the treatment, and the treatment comprises administering the dsRNA agent or the LPA antisense polynucleotide agent of the present invention to a subject based at least in part on an evaluation of changes in one or more physiological characteristics of LPA-related diseases or conditions in the subject caused by the treatment. For example, in some embodiments of the present invention, the action of a dsRNA agent or an LPA antisense polynucleotide agent of the present invention administered to a subject may be determined and used to help regulate the amount of the dsRNA agent or LPA antisense polynucleotide agent of the present invention subsequently administered to the subject. In one non-limiting example, a dsRNA agent or an LPA antisense polynucleotide agent of the present invention is administered to a subject, and the Lp (a) level in the blood of the subject is measured after administration; and at least partially based on the determined level, it is determined whether a higher amount of the dsRNA agent or the LPA antisense polynucleotide agent is required to improve the physiological effect of the administered agent, such as reducing or further reducing Lp (a) level in the blood of the subject. In another non-limiting example, the dsRNA agent or LPA antisense polynucleotide agent of the present invention is administered to the subject and the level of Lp (a) in the blood of the subject is determined after administration, and a lower amount of the dsRNA agent or LPA antisense polynucleotide agent is expected to be administered to the subject based at least in part on the determined level.

Thus, some embodiments of the present invention include evaluating changes in one or more physiological characteristics caused by prior treatment of the subject to adjust the amount of the dsRNA agent or LPA antisense polynucleotide agent of the present invention subsequently administered to the subject. Some embodiments of the method of the present invention include measuring physiological characteristics of an LPA-related disease or condition 1, 2, 3, 4, 5, 6 or more times; evaluating and/or monitoring the efficacy of administered LPA dsRNA agent or LPA antisense polynucleotide agent of the present invention; and optionally adjusting one or more of the followings using the measured results: dosage, dosing regimen, and/or dosing frequency of the dsRNA agent or LPA antisense polynucleotide agent of the present invention for treating the LPA-related disease or condition in a subject. In some embodiments of the method of the present invention, the desired result of administering an effective amount of the dsRNA agent or the LPA antisense polynucleotide agent of the present invention to a subject is that the Lp(a) level in blood of the subject is reduced compared to the previous Lp(a) level in the blood determined for the subject; the Lp(a) level in the blood of the subject was within the normal range.

As used herein, the terms "treat", "therapeutic" or "treated" when used for an LPA-related disease or condition may refer to a prophylactic treatment, reducing the likelihood of developing an LPA-related disease or condition in a subject, and may also refer to a treatment to eliminate or reduce the level of an LPA-related disease or condition after the subject has developed the LPA-related disease or condition, preventing an LPA-related disease or condition from becoming more severe, and/or slowing the progression of an LPA-related disease or condition in a subject compared to the subject in the absence of therapy that reduces the level of the LPA polypeptide in the subject.

Certain embodiments of the agents, compositions, and methods of the present invention may be used to inhibit LPA gene expression. As used herein, with regard to the expression of the LPA gene, the terms "inhibit", "silence", "reduce", "down-regulate" and "knock down" refer to, for example, changing the expression of the LPA gene by one or more of the followings: the level of RNA transcribed by the gene, the level of LPA expressed, and the level of LPA polypeptide, protein or protein subunit translated from the mRNA in a cell, cell population, tissue, organ or subject is reduced when the cell, cell population, tissue, organ or subject is contacted with (e.g., treated with) the LPA dsRNA agent or the LPA antisense polynucleotide agent of the present invention, as compared to the control level of RNA transcribed by the LPA gene, the control level of LPA translated from the mRNA, respectively. In some embodiments, the control level is the level in cells, tissues, organs or subjects not contacting with (e.g. treated with) the LPA dsRNA agents or LPA antisense polynucleotide agents.

### Application method

Various routes of administration of the LPA dsRNA agent or LPA antisense polynucleotide agent may be used in the method of the present invention. The choice of a particular delivery mode will depend, at least in part, on the specific condition being treated and the dose required for therapeutic efficacy. In general, the method of the present invention can be implemented using any medically acceptable dosing mode, meaning any mode that produces an effective therapeutic level on LPA-related diseases or conditions without causing clinically unacceptable side effects. In some embodiments of the present invention, the LPA dsRNA agents or LPA antisense polynucleotide agents may be administered orally, enterally, mucosally, subcutaneously, and/or parenterally. The term "parenteral" includes subcutaneous, intravenous, intrathecal, intramuscular, intraperitoneal and intrasternal injection or infusion techniques. Other routes include, but are not limited to, nasal (e.g., through a nasogastric tube), percutaneous, vaginal, rectal, sublingual, and inhalation. The delivery route of the present invention may include intrathecal, intraventricular or intracranial. In some embodiments of the present invention, the LPA dsRNA agent or LPA antisense polynucleotide agent may be placed in a sustained release matrix and administered by placing the matrix in a subject. In some aspects of the present invention, the LPA dsRNA agent or LPA antisense polynucleotide agent may be delivered to cells of the subject using nanoparticles coated with delivery agents targeting specific cells or organelles. Various delivery manners, methods, and agents are known in the art. Non-limiting examples of delivery methods and delivery agents are provided elsewhere herein. In some aspects of the present invention, the term "delivery" with respect to LPA dsRNA agent or LPA antisense polynucleotide agent may refer to the administration of one or more "naked" LPA dsRNA agent or LPA antisense polynucleotide agent sequences to a cell or subject. In certain aspects of the present invention, "delivery" refers to delivery to a cell or subject by transfection, delivery of a cell comprising an LPA dsRNA agent or an LPA antisense polynucleotide agent to a subject, delivery of a vector encoding an LPA dsRNA agent or an LPA antisense polynucleotide agent to a cell and/or subject, etc. Delivery of an LPA dsRNA agent or an LPA antisense polynucleotide agent using a transfection mode may include administering a vector to a cell and/or a subject.

In some methods of the present invention, one or more LPA dsRNA agents or LPA antisense polynucleotide agents may be administered in formulation form or in pharmaceutically acceptable solutions, which may generally contain pharmaceutically acceptable concentrations of salts, buffers, preservatives, compatible carriers, adjuvants, and optionally other therapeutic ingredients. In some embodiments of the present invention, the LPA dsRNA agent or LPA antisense polynucleotide agent may be formulated with another therapeutic agent for simultaneous administration. According to the method of the present invention, the LPA dsRNA agent or LPA antisense polynucleotide agent may be administered in the form of a pharmaceutical composition. Typically, the pharmaceutical composition comprises the LPA dsRNA agent or LPA antisense polynucleotide agent and optionally a pharmaceutically acceptable carrier. The pharmaceutically acceptable carrier is well known to those of ordinary skill in the art. As used herein, the pharmaceutically acceptable vector refers to a nontoxic material that does not interfere with the effectiveness of the biological activity of the active ingredient (e.g., the ability of LPA dsRNA agent or LPA antisense polynucleotide agent to inhibit LPA gene expression in cells or subjects). Various methods of administering and delivering the dsRNA agent or LPA antisense polynucleotide agent for therapeutic use are known in the art and may be used in the methods of the present invention.

Pharmaceutically acceptable carriers include diluents, fillers, salts, buffers, stabilizers, solubilizers and other materials known in the art. Exemplary pharmaceutically acceptable carriers are described in U.S. Pat. No. 5,211,657, while other carriers are known to those skilled in the art. Such formulations may generally contain salts, buffers, preservatives, compatible carriers and optionally other therapeutic agents. The salts should be pharmaceutically acceptable for use in medicine, but non-pharmaceutically acceptable salts can be conveniently used to prepare their pharmaceutically acceptable salts and are not excluded from the scope of the present invention. Such pharmacologically and pharmaceutically acceptable salts include, but are not limited to, salts prepared from hydrochloric acid, hydrobromic acid, sulfuric acid, nitric acid, phosphoric acid, maleic acid, acetic acid, salicylic acid, citric acid, formic acid, malonic acid, succinic acid, and the like. Further, pharmaceutically acceptable salts may be prepared as alkali metal salts or alkaline earth metal salts, such as sodium salts, potassium salts or calcium salts.

Some embodiments of the method of the present invention include administrating one or more LPA dsRNA agents or LPA antisense polynucleotide agents directly to tissues. In some embodiments, the tissue to which the compound is administered is a tissue in which LPA-related diseases or conditions are present or are likely to develop, non-limiting examples of the tissue are the liver or kidney. Direct tissue administration can be achieved by direct injection or by other means. Many orally delivered compounds naturally enter and pass through the liver and kidneys, and some embodiments of the therapeutic method of the present invention include the oral administration of one or more LPA dsRNA agents to the subject. LPA dsRNA agents or LPA antisense polynucleotide agents, alone or in combination with other therapeutic agents, may be administered once, or may be administered multiple times. If administered multiple times, the LPA dsRNA agent or LPA antisense polynucleotide agent can be administered via different routes. For example, although not intended to be limiting, the first (or first few) administration may be performed subcutaneously, and one or more additional administrations may be oral and/or systemic.

For embodiments of the present invention in which it is desirable to administer the LPA dsRNA agent or LPA antisense polynucleotide agent systemically, the LPA dsRNA agent or LPA antisense polynucleotide agent may be formulated for parenteral administration by injection, for example by bolus or continuous infusion. Injectable formulations may be present in unit dosage forms, such as ampoules or multi-dose containers, with or without preservatives. LPA dsRNA agent formulations (also referred to as pharmaceutical compositions) may be in the form of suspensions, solutions, or emulsions in oily or aqueous carriers, and may contain excipients, such as suspending agents, stabilizers, and/or dispersants.

Formulations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, alcohol/aqueous solutions, emulsions or suspensions, including saline and buffer media. Parenteral carriers include sodium chloride solution, Ringer's glucose solution, glucose and sodium chloride solution, lactate Ringer's solution or fixed oil. Intravenous excipients include fluid and nutritional supplements, electrolyte supplements (such as those based on Ringer's glucose solution), and the like. Preservatives and other additives may also be present, such as antimicrobial agents, antioxidants, chelating agents, inert gases, and the like. Other forms of administration, such as intravenous administration, will result in lower doses. If the response of the subject is insufficient at the initial dose, a higher dose can be used within the tolerance of the patient (or the dose may be effectively increased by a different, more localized delivery route). Multiple doses per day may be used as needed to achieve appropriate systemic or local levels of one or more LPA dsRNA agents or LPA antisense polynucleotide agents and to achieve appropriate reduction in LPA protein levels.

In other embodiments, the method of the present invention comprises the use of delivery carriers, such as biocompatible microparticles, nanoparticles, or implants suitable for implantation into recipients such as subjects. Exemplary biodegradable implants that may be used according to this method are described in PCT publication WO 95/24929 (incorporated herein by reference), which describes a biocompatible, biodegradable polymer matrix for incorporating biomacromolecules.

Both non-biodegradable and biodegradable polymer matrices may be used in the method of the present invention to deliver one or more LPA dsRNA agents or LPA antisense polynucleotide agents to the subject. In some embodiments, the matrix may be biodegradable. The matrix polymer may be a natural or synthetic polymer. The polymer may be selected based on the desired period of release, typically on the order of a few hours to a year or more. Usually, releases over a period of time between a few hours and three to twelve months can be used. The polymer is optionally in the form of a hydrogel, which can absorb up to about 90% of its weight of water, and is also optionally cross-linked with multivalent ions or other polymers.

In general, the LPA dsRNA agent or LPA antisense polynucleotide agent may be delivered in some embodiments of the present invention using biodegradable implants by diffusion or by degradation of a polymer matrix. Exemplary synthetic polymers for this use are well known in the art. Using methods known in the art, biodegradable polymers and non-biodegradable polymers can be used to deliver the LPA dsRNA agent or LPA antisense polynucleotide agent. Bioadhesive polymers such as bioerodible hydrogels (H. S. Sawhney, C. P. Pathak and J. A. Hubell in Macromolecules, 1993, 26, 581-587) can also be used to deliver the LPA dsRNA agent or LPA antisense polynucleotide agent to treat LPA-related diseases or conditions. Other suitable delivery systems may include timed release, delayed release, or sustained release delivery systems. Such systems avoid repeated administration of LPA dsRNA agent or LPA antisense polynucleotide agent, thereby improving convenience for subjects and healthcare professionals. Many types of release delivery systems are available and are known to those of ordinary skill in the art. See, for example, U.S. Pat. No. 5,075,109, 4,452,775, 4,675,189, 5,736,152, 3,854,480, 5,133,974, and 5,407,686. In addition, pump-based hardware delivery systems can be used, some of which are also suitable for implantation.

The use of long-term sustained release implants may be adapted for the prophylactic treatment of subjects and subjects at risk of developing recurrent LPA-related diseases or conditions. As used herein, long-term release refers to the construction and arrangement of implants to deliver a therapeutic level of an LPA dsRNA agent or an LPA antisense polynucleotide agent for at least up to 10 days, 20 days, 30 days, 60 days, 90 days, six months, one year or more. Long-term sustained release implants are well known to those of ordinary skill in the art and include some of the release systems described above.

Therapeutic formulations of LPA dsRNA agent or LPA antisense polynucleotide agent may be prepared for storage by mixing molecules or compounds with the desired purity with an optional pharmaceutically acceptable carrier, excipient or stabilizer [Remington's Pharmaceutical Sciences 21st edition, (2006)] in the form of a lyophilized formulation or an aqueous solution. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butanol or benzyl alcohol; parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt forming counter-ions such as sodium; metal complexes (e.g., Zn-protein complexes); and/or nonionic surfactants such as TWEEN^{®}, PLURONICS^{®} or polyethylene glycol (PEG).

### Cells, subjects and controls

The methods of the present invention may be used in conjunction with cells, tissues, organs, and/or subjects. In some aspects of the present invention, the subjects are humans or vertebrate mammals, including but not limited to dogs, cats, horses, cows, goats, mice, rats and primates, such as monkeys. Therefore, the present invention can be used to treat LPA-related diseases or conditions of both human and non-human subjects. In some aspects of the present invention, a subject may be a farm animal, a zoo animal, a domesticated animal, or a non-domesticated animal, and the method of the present invention may be used in veterinary prophylactic and therapeutic regimens. In some embodiments of the present invention, the subject is a person and the method of the present invention can be used in prophylactic and therapeutic regimens for human.

Non-limiting examples of subjects to which the present invention may be applied are subjects diagnosed with, suspected of having, or at risk of developing a disease or condition associated with LPA expression higher than expected expression, also referred to as an "elevated LPA expression level". Non-limiting examples of the disease and condition associated with higher than expected level of LPA expression are described elsewhere herein. The method of the present invention can be applied to subjects who have been diagnosed with the disease or condition at the time of treatment, subjects associated with LPA expression higher than expected expression, or subjects who are considered to be at risk of suffering from or developing a disease or condition associated with LPA expression higher than expected expression. In some aspects of the present invention, a disease or condition associated with a LPA expression level higher than expected level is an acute disease or condition; in some aspects of the present invention, a disease or condition associated with a LPA expression level higher than expected level is a chronic disease or condition.

In one non-limiting example, the LPA dsRNA agent of the present invention is administered to a subject diagnosed with a cardiovascular disease, wherein the cardiovascular disease includes Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles. The method of the present invention can be applied to subjects who have been diagnosed with the disease or condition at the time of treatment, or who are considered to be at risk of suffering from or developing the disease or condition.

In another non-limiting example, the LPA dsRNA agent of the present invention is administered to treat a disease or disorder that is caused by or associated with renin-angiotensin-aldosterone system (RAAS) activation, or whose symptoms or progression responds to RAAS inactivation. The term "LPA-related disease" includes a disease, disorder, or condition that benefits from reduced LPA expression. These diseases are usually associated with high blood pressure. Non-limiting examples of LPA-related diseases include cardiovascular diseases, including Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles.

Cells to which the method of the present invention can be applied include in vitro, in vivo and ex vivo cells. Cells may be in the subject, in culture and/or suspension, or in any other suitable state or condition. Cells to which the method of the present invention may be applied may be liver cells, hepatocytes, heart cells, pancreatic cells, cardiovascular cells, kidney cells, or other types of vertebrate cells, including human and non-human mammalian cells. In some aspects of the present invention, cells to which the method of the present invention may be applied are healthy normal cells, which are not known to be diseased cells. In some embodiments of the present invention, the methods and compositions of the present invention are applied to cells such as liver cells, hepatocytes, heart cells, pancreatic cells, cardiovascular cells, and/or kidney cells. In certain aspects of the present invention, the control cells are normal cells, but it should be understood that cells with a disease or condition may also be used as control cells in particular cases, such as in the case of comparing the results of treated cells with a disease or condition to the results of untreated cells with the disease or condition, and the like.

According to the method of the present invention, LPA polypeptide levels can be determined and compared to LPA polypeptide control levels. The control may be a predetermined value, which may take various forms. It can be a single cutoff, such as median or average. It can be established based on comparison groups, for example in groups with normal levels of LPA polypeptide and groups with increased levels of LPA polypeptide activity. Another non-limiting example of the comparison group may be a population with one or more symptoms or diagnoses of an LPA-related disease or condition versus a population without the one or more symptoms or diagnoses of the disease or condition; a group of subjects to whom the siRNA treatment of the present invention has been administered versus a group of subjects to whom the siRNA treatment of the present invention has not been administered. Typically, controls can be based on apparently healthy normal individuals or apparently healthy cells in the appropriate age group. It will be appreciated that, in addition to predetermined values, a control according to the present invention may be a material sample tested in parallel with the experimental material. Examples include samples from control populations or control samples produced by manufacturing for parallel test with experimental samples. In some embodiments of the present invention, the control may include cells or subjects that are not contacted or treated with the LPA dsRNA agent of the present invention, in which case the control level of the LPA polypeptide may be compared to the level of the LPA polypeptide in cells or subjects in contact with an LPA dsRNA agent or an LPA antisense polynucleotide agent of the present invention.

In some embodiments of the present invention, the control level may be an LPA polypeptide level determined for the subject, wherein the LPA polypeptide level determined for the same subject at different times is compared to the control level. In one non-limiting example, levels of LPA are determined in biological samples obtained from subjects who have never been received the LPA treatment according to the present invention. In some embodiments, the biological sample is a serum sample. LPA polypeptide levels measured in samples obtained from the subject may serve as baseline or control values for the subject. After one or more LPA dsRNA agents are administered to the subject according to the therapeutic method of the present invention, one or more additional serum samples may be obtained from the subject, and the LPA polypeptide levels in the subsequent one or more samples may be compared to the subject's control/baseline levels. Such comparisons can be used to assess onset, progression, or regression of LPA-related diseases or conditions in subjects. For example, the level of the LPA polypeptide in the baseline sample obtained from the subject is higher than the level obtained from the same subject after the LPA dsRNA agent or the LPA antisense polynucleotide agent of the present invention is administered to the subject, indicating regression of the LPA-related disease or condition and the efficacy of the administered LPA dsRNA agent of the present invention in treating the LPA-related disease or condition.

In certain aspects of the present invention, one or more values of the LPA polypeptide levels determined for a subject may be used as a control value and used to later compare LPA polypeptide levels in the same subject, thereby allowing for the evaluation of changes from the "baseline" LPA polypeptide level in the subject. Thus, where the initial level is used as the control level of the subject, the initial LPA polypeptide level may be used to display and/or determine the level of the LPA polypeptide in the subject that the methods and compounds of the present invention can reduce in the subj ect.

Using the method of the present invention, the LPA dsRNA agent and/or the LPA antisense polynucleotide agent of the present invention can be administered to the subject. Such dsRNAi agents include, for example, the duplexes shown in Table 1. In some other embodiments, such dsRNAi agents include duplex variants, such as those shown in Table 1. The efficacy of the administration and treatment of the present invention may be evaluated as follows: after administration and treatment, the level of the LPA polypeptide in the serum sample obtained from the subject is reduced by at least 0.5%, 1%, 5%, 10%, 20%, 30%, 40%, 50%, 60%, 70%, 80%, 90%, 95% or more compared to the level of the LPA polypeptide before administration in the serum sample obtained from the subject at a previous time point, or compared to the level of a non-contact control (e.g., the level of the LPA polypeptide in the control serum sample). It will be appreciated that the levels of LPA polypeptides are all associated with the levels of LPA gene expression. Some embodiments of the method of the present invention include administering the LPA dsRNA and/or the LPA antisense agent of the present invention to the subject in an amount that effectively inhibits the expression of the LPA gene, thereby reducing the level of the LPA polypeptide in the subject.

Some embodiments of the present invention include determining the presence, absence and/or amount (herein also referred to as level) of the LPA polypeptide from one or more biological samples obtained from one or more subjects. This determination can be used to evaluate the efficacy of the therapeutic method of the present invention. For example, the methods and compositions of the present invention may be used to determine the level of the LPA polypeptide in a biological sample obtained from a subject previously treated with the LPA dsRNA agent and/or the LPA antisense agent of the present invention. After administration and treatment, the level of the LPA polypeptide in the serum sample obtained from the subject is reduced by at least 0.5%, 1%, 5%, 10%, 20%, 30%, 40%, 50%, 60%, 70%, 80%, 90%, 95% or more compared to the level of the LPA polypeptide before administration in the serum sample obtained from the subject at a previous time point, or compared to the level of a non-contact control (e.g., the level of the LPA polypeptide in the control serum sample), the efficacy level of the treatment administered to the subject is indicated.

In some embodiments of the present invention, the physiological characteristics of the LPA-related disease or condition determined for the subject may serve as a control result, and the determination results of the physiological characteristics of the same subject at different times are compared to the control result. In one non-limiting example, the Lp(a) level in blood (and/or other physiological characteristics of an LPA disease or condition) is measured from a subj ect that has never been received the LPA treatment according to the present invention, and may be used as a baseline or control value for the subject. After one or more LPA dsRNA agents are administered to the subject according to the therapeutic method of the present invention, Lp(a) levels in the blood are measured and compared to the control/baseline levels of the subject, respectively. Such comparisons can be used to assess onset, progression, or regression of LPA-related diseases or conditions in subjects. For example, if the baseline LPA level obtained from the subject is higher than the LPA level measured from the same subject after the LPA dsRNA agent or the LPA antisense polynucleotide agent of the present invention is administered to the subject, indicating regression of the LPA-related disease or condition and the efficacy of the administered LPA dsRNA agent of the present invention in treating the LPA-related disease or condition.

In some aspects of the present invention, the value of one or more physiological characteristics of an LPA-related disease or condition determined for a subject may be used as a control value for later comparisons of physiological characteristics of the same subject, thereby allowing for the evaluation of changes from the "baseline" physiological characteristics of the subject. Thus, an initial physiological characteristic in an individual may be obtained, and measurement of the initial physiological characteristic is used as a control of the subject, and to show and/or determine the effect that the method and compound of the present invention may be used to reduce the level of the LPA polypeptide in the individual. Using the method of the present invention, the LPA dsRNA agent and/or the LPA antisense polynucleotide agent of the present invention can be administered to the subject in an effective amount for the treatment of an LPA disease or condition. The efficacy of administration and treatment of the present invention may be evaluated by determining changes in one or more physiological characteristics of an LPA disease or condition. In one non-limiting example, the Lp (a) level in blood of a subject is reduced until the Lp (a) levels in blood of the subject were within the normal range, as compared to Lp (a) level in blood obtained from the subject at a previous time point, or compared to LPA level in a non-contact control.

Some embodiments of the present invention include determining the presence, absence, and/or change of physiological characteristics of an LPA-related disease or condition using methods such as, but not limited to: (1) measuring Lp(a) levels in the blood of a subject; (2) evaluating the physiological characteristics of one or more biological samples obtained from one or more subjects; (3) or performing a physical examination on the subject. This determination can be used to evaluate the efficacy of the therapeutic method of the present invention.

### Kit

A kit comprising one or more LPA dsRNA agents and/or LPA antisense polynucleotide agents and instructions for their use in the method of the present invention is also within the scope of the present invention. The kit of the present invention may comprise one or more of an LPA dsRNA agent, an LPA sense polynucleotide agent, and an LPA antisense polynucleotide agent that can be used to treat a LPA-related disease or condition. A kit comprising one or more LPA dsRNA agents, LPA sense polynucleotide agents and LPA antisense polynucleotide agents may be prepared for use in the therapeutic methods of the present invention. The components of the kit of the present invention can be packaged in aqueous medium or freeze-dried form. The kit of the present invention may comprise a support that is partitioned to enclosedly receive one or more container devices or a series of container devices (e.g., test tubes, vials, flasks, bottles, syringes, etc.) therein. A first container device or a series of container devices may comprise one or more compounds, such as LPA dsRNA agent and/or LPA sense or antisense polynucleotide agent. The second container device or series of container devices may comprise a targeting agent, a labelling agent, a delivery agent, etc., which may be included as part of the LPA dsRNA agent and/or the LPA antisense polynucleotide administered in an embodiment of the therapeutic method of the present invention.

The kit of the present invention may also contain instructions. Instructions are usually in writing and will provide guidance for carrying out the treatment embodied by the kit and making decisions based on that treatment.

The following examples are provided to illustrate specific examples of the practice of the present invention and are not intended to limit the scope of the present invention. It is evident to those of ordinary skill in the art that the present invention can be applied to a variety of compositions and methods.

### Example

### Example 1. Synthesis of RNAi agent

The LPA RNAi agent duplex shown in Tables 2-3 above was synthesized according to the following general procedure:
Sense and antisense strand sequences of siRNA were synthesized on an oligonucleotide synthesizer using a well-established solid-phase synthesis method based on phosphoramidite chemistry. Growth of the oligonucleotide chain is achieved through a 4-step cycle: de-protection, condensation, capping, and an oxidation or sulfurization step for the addition of each nucleotide. Synthesis was performed on a solid support made of controllable porous glass (CPG, 1000 Å). Monomer phosphoramidite was purchased from commercial sources. phosphoramidites having GalNAc ligand clusters (GLPA1 and GLPA2 as non-limiting examples) were synthesized according to the procedure of Examples 2-3 herein. For siRNAs used for in vitro screening (Table 2), synthesis was performed at the 2 µmol scale; for siRNAs used for in vivo test (Tables 3, 4 to 5), synthesis scale was 5 µmol or greater. In the case where the GalNAc ligand (GLO-0 as a non-limiting example) was linked to the 3'-end of the sense strand, a CPG solid support with the GalNAc ligand attached was used. In the case where the GalNAc ligand (GLS-1 or GLS-2 as a non-limiting example) was linked to the 5'-end of the sense strand, GalNAc phosphoramidite (GLPA1 or GLPA2 as a non-limiting example) was used for the final coupling reaction. Trichloroacetic acid (TCA) in 3% dichloromethane was used to deprotect the protective group, i.e., 4,4'-dimethoxytriphenylmethyl (DMT). 5-ethylthio-1H-tetrazole was used as an activator. I₂ in THF/Py/H₂O and phenylacetyl disulfide (PADS) in pyridine/MeCN were used for oxidation and sulfurization reactions, respectively. After the final solid-phase synthesis step, the solid support-bound oligomer was cleaved by treatment with a 1: 1 volume of 40 wt% aqueous methylamine solution and 28% ammonium hydroxide solution and the protective group was removed. The crude mixture was concentrated to synthesize siRNAs for in vitro screening. The remaining solids were dissolved in 1.0 M NaOAc and ice-cold EtOH was added to precipitate a single-stranded product as a sodium salt, which could be used for annealing without further purification. To synthesize siRNAs for in vivo testing, the crude single-stranded product was further purified by ion-pair reversed-phase HPLC (IP-RP-HPLC). Purified single-stranded oligonucleotide products from IP-RP-HPLC were converted to sodium salts by dissolving them in 1.0 M NaOAc and precipitating them by adding ice-cold EtOH. Annealing of sense and antisense oligonucleotides by equimolar complementation was performed in water to form a double-stranded siRNA product, which was lyophilized to provide a fluffy white solid.

### Example 2. Preparation of intermediate-A and intermediate-B

Intermediate-A was synthesized by treating commercially available galactosamine pentaacetate with trimethylsilyl trifluoromethanesulfonate (TMSOTf) in dichloromethane (DCM), as shown in scheme 1 below. Then glycosylation was performed with Cbz-protected 2-(2-aminoethoxy)ethan-1-ol to give compound II. The Cbz protective group was removed by hydrogenation to provide intermediate-A as a trifluoroacetate (TFA) salt. Intermediate-B was synthesized based on the same scheme, except for the use of Cbz-protected 2-(2-(2-aminoethoxy)ethoxy)ethan-1-ol as feedstock.

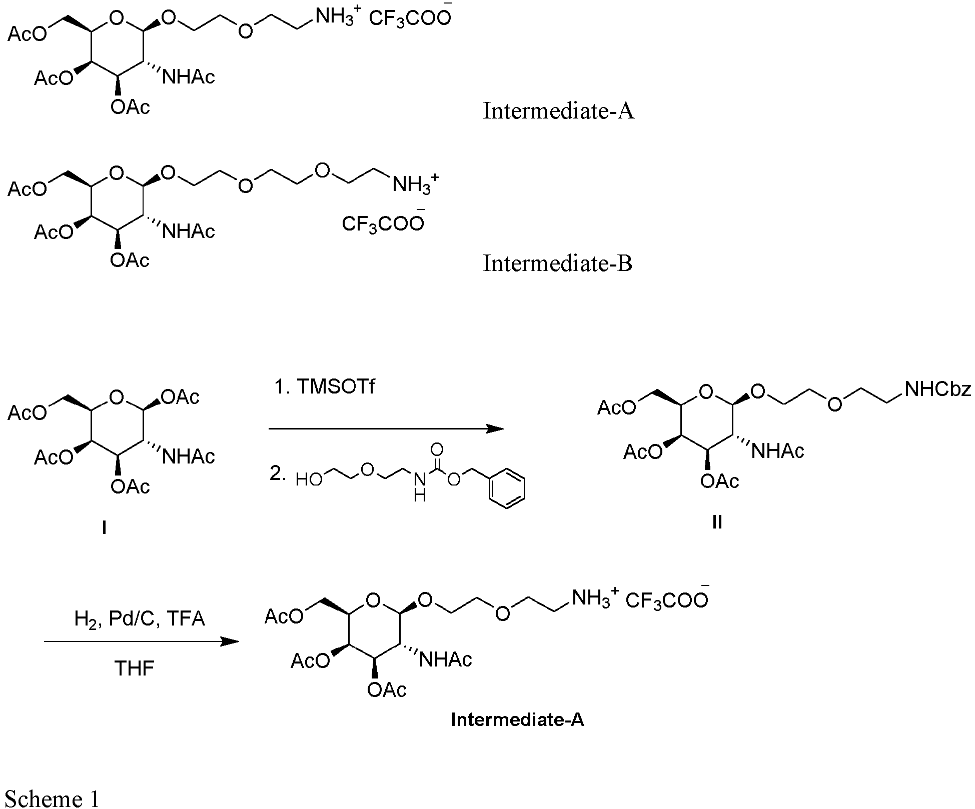

TMSOTf (17.1 g, 77.2 mmol) was added to the solution of compound I (20.0 g, 51.4 mmol) in 100 mL of 1,2-dichloroethane (DCE). The resulting reaction liquid was stirred at 60 °C for 2 h and then stirred at 25 °C for 1 h; Cbz-protected 2-(2-aminoethoxy)ethan-1-ol (13.5 g, 56.5 mmol) was dried over 4 Å powdered molecular sieves (10 g) in DCE (100 mL) and added dropwise to the above reaction liquid at 0°C under N₂ atmosphere. The resulting reaction mixture was stirred at 25 °C for 16 hours under N₂ atmosphere. The reaction mixture was filtered and washed with saturated NaHCO₃ (200 mL), water (200 mL) and saturated saline (200 mL). The organic layer was dried over anhydrous Na₂SO₄, filtered and concentrated under reduced pressure to give the crude product, which was triturated with 2-methyltetrahydrofuran/heptane (5/3, v/v, 1.80 L) for 2 h. The resulting mixture was filtered and dried to give compound II (15.0 g, yield 50.3%) as a white solid.

10% Pd/C (1.50 g) was carefully added to a dry and argon-purged hydrogenation bottle, and then 10 mL of tetrahydrofuran (THF) was added, followed by a solution of compound II (15.0 g, 26.4 mmol) in THF (300 mL) and TFA (trifluoroacetic acid, 3.00 g, 26.4 mmol). The resulting mixture was degassed and purged with H₂ three times and stirred at 25 °C under H₂ atmosphere (45 psi) for 3 h. Thin layer chromatography (TLC, solvent: DCM:MeOH = 10:1) indicated that compound II was completely consumed. The reaction mixture was filtered and concentrated under reduced pressure. The residue was dissolved in anhydrous DCM (500 mL) and concentrated. The process was repeated 3 times to give intermediate-A as a foamy white solid (14.0 g, yield 96.5%).¹H NMR (400 MHz DMSO-*d₆*): δ ppm 7.90 (d, *J* = 9.29 Hz, 1 H), 7.78 (br s, 3 H), 5.23 (d, *J* = 3.26 Hz, 1 H), 4.98 (dd, *J* = 11.29, 3.26 Hz, 1 H), 4.56 (d, *J* = 8.53 Hz, 1 H), 3.98 - 4.07 (m, 3 H), 3.79 - 3.93 (m, 2 H), 3.55 - 3.66 (m, 5 H), 2.98 (br d, *J* = 4.77 Hz, 2 H), 2.11 (s, 3 H), 2.00 (s, 3 H), 1.90 (s, 3 H), 1.76 (s, 3 H)_{∘}

Intermediate-B was synthesized using a similar procedure to that used for the synthesis of Intermediate-A. ¹H NMR (400 MHz DMSO-*d₆*): δ ppm 7.90 (br d, *J =* 9.03 Hz, 4 H), 5.21 (d, *J =* 3.51 Hz, 1 H), 4.97 (dd, *J* = 11.1 Hz, 1 H), 4.54 (d, *J* = 8.53 Hz, 1 H), 3.98 - 4.06 (m, 3 H), 3.88 (dt, *J* = 10.9 Hz, 1 H), 3.76 - 3.83 (m, 1 H), 3.49 - 3.61 (m, 9 H), 2.97 (br s, 2 H), 2.10 (s, 3 H), 1.99 (s, 3 H), 1.88 (s, 3 H), 1.78 (s, 3 H). Mass spectrometry calculated C₂₀H₃₄N₂O₁₁: 478.22; measured : 479.3 (M+H⁺)_{∘}

### Example 3. Synthesis of GalNAc ligand cluster phosphoramidites GLPA1, GLPA2, and GLPA15.

GLPA1 and GLPA2 were prepared according to the following scheme 2. Starting with benzyl-protected propane-1,3-diamine, alkylation was performed using tert-butyl 2-bromoacetate to give triester compound I. The benzyl protective group is removed by hydrogenation to give secondary amine compound II. The amide was coupled with 6-hydroxycaproic acid to give compound III. The tert-butyl protective group was then removed upon treatment with HCl in dioxane to form triacid compound IV Amide coupling between triacid compound IV and intermediate-A or intermediate-B was performed to provide compound Va or Vb. Phosphoramidite GLPA1 or GLPA2 was synthesized by phosphitylation of compound Va or Vb with 2-cyanoethyl N,N-diisopropylchlorophosphoramidite and a catalytic amount of 1H-tetrazole.

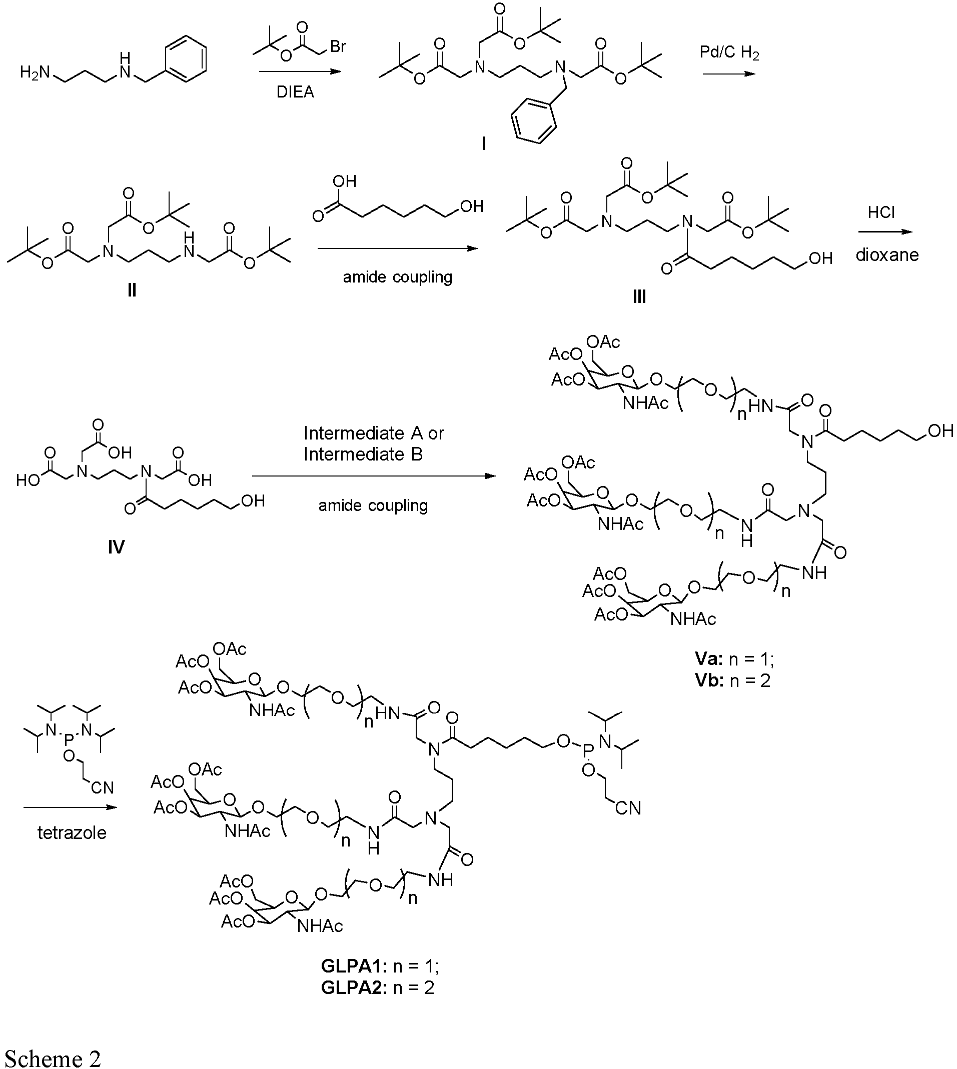

Tert-butyl 2-bromoacetate (23.7 g, 121 mmol) was added to a solution of N-benzyl-1,3-propanediamine (5.00 g, 30.4 mmol) in dimethylformamide (DMF, 100 mL); then diisopropylethylamine (DIEA, 23.61 g, 182 mmol) was added dropwise. The resulting reaction mixture was stirred at 25-30 °C for 16 h. LCMS showed that N-benzyl-1,3-propanediamine was completely consumed. The reaction mixture was diluted with H₂O (500 mL) and extracted with EtOAc (500 mL × 2). The combined organics were washed with saturated brine (1 L), dried over anhydrous Na₂SO₄, filtered, and concentrated under reduced pressure to give a crude product which was purified by silica gel column chromatography (gradient: petroleum ether:ethyl acetate 20:1 to 5:1). A compound I was obtained (12.1 g, yield 78.4%) as a colorless oil. ¹H NMR (400 MHz, CDCl₃): δ ppm 7.26 - 7.40 (m, 5 H), 3.79 (s, 2 H), 3.43 (s, 4 H), 3.21 (s, 2 H), 2.72 (dt, *J* = 16.9, 7.34 Hz, 4 H), 1.70 (quin, *J* = 7.2 Hz, 2 H), 1.44 - 1.50 (m, 27 H).

The dried hydrogenation bottle was purged 3 times with argon. Pd/C (200 mg, 10%) was added, followed by MeOH (5 mL), followed by a solution of compound I (1.00 g, 1.97 mmol) in MeOH (5 mL). The reaction mixture was degassed in vacuum and refilled with H₂. This process was repeated 3 times. The mixture was stirred at 25 °C for 12 h under H₂ atmosphere (15 psi). LCMS showed that compound I was completely consumed. The reaction mixture was filtered under reduced pressure under N₂ atmosphere. The filtrate was concentrated under reduced pressure to give compound II (655 mg, yield 79.7%) as a yellow oil, which could be used in the next step without further purification.¹H NMR (400 MHz, CDCl₃): δ ppm 3.44 (s, 4 H), 3.31 (s, 2 H), 2.78 (t, *J* = 7.1 Hz, 2 H), 2.68 (t, *J* = 6.9 Hz, 2 H), 1.88 (br s, 1 H), 1.69 (quin, *J* = 7.03 Hz, 2 H), 1.44 - 1.50 (s, 27 H).

A mixture of compound II (655 mg, 1.57 mmol), 6-hydroxyhexanoic acid (249 mg, 1.89 mmol), DIEA (1.02 g, 7.86 mmol), 1-ethyl-3-(3-dimethylaminopropyl)carbodiimide hydrochloride (EDCI, 904 mg, 4.72 mmol), and 1-hydroxybenzotriazole (HOBt, 637 mg, 4.72 mmol) in DMF (6 mL) was degassed and purged with N₂ three times; then stirred at 25 °C under N₂ atmosphere for 3 h. The LCMS indicated the desired product. The reaction mixture was diluted with H₂O (10mL) and extracted with EtOAc 20 mL (10 mL × 2). The organics were combined, washed with saturated brine (20 mL), dried with anhydrous Na₂SO₄, filtered and concentrated to obtain a crude product which was purified by silica gel column chromatography (gradient: petroleum ether:ethyl acetate from 5:1 to 1:1) to give compound III (650 mg, yield 77.8%) as a yellow oil. ¹H NMR (400 MHz, CDCl₃): δ ppm 3.90 - 3.95 (s, 2 H), 3.63 (t, *J* = 6.40 Hz, 2 H), 3.38 - 3.45 (m, 6 H), 2.72 (t, *J* = 6.65 Hz, 2 H), 2.40 (t, *J =* 7.28 Hz, 2 H), 1.55 - 1.75 (m, 8 H), 1.44 (s, 27 H). Mass spectrometry calculated C₂₇H₅₀N₂O₈: 530.36; measured : 531.3 (M+H⁺)_{∘}

A mixture of compound III (5.5 g, 10.3 mmol) in HCl/ dioxane (2 M, 55 mL) was stirred at 25 °C for 3 h. LCMS showed complete consumption of compound III. The reaction mixture was filtered, washed with EtOAc (50 mL), and dried under reduced pressure to give a crude product. The crude product was dissolved in CH₃CN (50 mL) and the volatiles were removed in vacuum. The process was repeated 3 times to give compound IV (2.05 g, 54.5% yield) as a white solid. ¹H NMR (400 MHz, D₂O): δ ppm 4.21 (s, 1 H), 4.07 (d, *J* = 4.5 Hz, 4 H), 3.99 (s, 1 H), 3.45 - 3.52 (m, 3 H), 3.42 (t, *J* = 6.5 Hz, 1 H), 3.32 - 3.38 (m, 1 H), 3.24 - 3.31 (m, 1 H), 2.37 (t, *J* = 7.4 Hz, 1 H), 2.24 (t, *J* = 7.4 Hz, 1 H), 1.99 (dt, *J* = 15.5, 7.53 Hz, 1 H), 1.85 - 1.94 (m, 1 H), 1.85 - 1.94 (m, 1 H), 1.39 - 1.56 (m, 4 H), 1.19 - 1.31 (m, 2H)_{∘}

A mixture of compound IV (500 mg, 1.05 mmol), intermediate-A (2.02 g, 3.67 mmol), DIEA (813 mg, 6.30 mmol), EDCI (704 mg, 3.67 mmol), and HOBt (496 mg, 3.67 mmol) in DMF (10 ml) was degassed and purged with N₂ three times, and then the mixture was stirred at 25 °C under N₂ atmosphere for 3 h. The LCMS indicated the desired product. The reaction mixture was quenched by adding H₂O (10mL) and extracted with DCM (10 mL × 2). The combined organics were extracted with 10% citric acid (20 mL). The aqueous phase was neutralized with saturated NaHCO₃ solution and re-extracted with DCM (10 mL × 2). The organics were dried over sodium sulfate, filtered and concentrated under reduced pressure to give compound Va (570 mg, 0.281 mmol, 26.8% yield) as a white solid. ¹H NMR: (400 MHz, CDCl₃) ppm δ 7.84 - 8.12 (m, 3 H), 6.85 - 7.15 (m, 2 H), 6.66 - 6.81 (m, 1 H), 5.36 (br d, *J =* 2.7 Hz, 3 H), 5.11 - 5.27 (m, 3 H), 4.63 - 4.85 (m, 3 H), 3.90 - 4.25 (m, 18 H), 3.37 - 3.75 (m, 28 H), 3.15 - 3.28 (m, 4 H), 2.64 (br d, *J* = 6.53 Hz, 2 H), 2.30 - 2.46 (m, 2 H), 2.13 - 2.18 (m, 9 H), 2.05 (s, 9 H), 1.94 - 2.03 (m, 18 H), 1.68 (br s, 2 H), 1.45 (br s, 2 H), 1.12 (br t, *J* = 7.0 Hz, 2 H)_{∘}

Tetrazole diisopropylammonium (30.3 mg, 0.177 mmol) was added to a solution of compound Va (260 mg, 0.161 mmol) in anhydrous DCM (5 mL); then 3-bis(diisopropylamino)phosphoryloxypropionitrile (194 mg, 0.645 mmol) was added dropwise at ambient temperature under N₂. The reaction mixture was stirred at 20 to 25 °C for 2 h. LCMS indicated that compound Va was completely consumed. After cooling to -20 °C, the reaction mixture was added to a stirred brine/saturated NaHCO₃ (1: 1, 5 mL) solution at 0 °C. After stirring for 1 minute, DCM (5 mL) was added. Stratification occured. The organic layer was washed with brine/saturated NaHCO₃ aqueous solution (1:1, 5 mL), dried over Na₂SO₄, filtered and concentrated to a volume of approximately 1 mL. The residual solution was added dropwise to 20 mL of methyl tert-butyl ether (MTBE) under stirring. This resulted in the precipitation of a white solid. The mixture was centrifuged and the solid was collected. The solid was redissolved in 1 mL of DCM and precipitated by addition of MTBE (20 mL). The solid was again separated by centrifugation. The collected solid was dissolved in anhydrous CH₃CN. The volatile was removed. This process was repeated twice more to give the GalNAc ligand phosphoramidite compound GLPA1 (153 mg, 84.4 µmol) as a white solid.¹H NMR (400 MHz, CDCl₃): ppm δ 7.71 - 8.06 (m, 2 H), 6.60 - 7.06 (m, 3 H), 5.37 (br d, J = 3.0 Hz, 3 H), 5.18 - 5.32 (m, 3 H), 4.70 - 4.86 (m, 3 H), 3.92 - 4.25 (m, 18 H), 3.42 - 3.85 (m, 30 H), 3.25 (m, 4 H), 2.59 - 2.75 (m, 4 H), 2.27 - 2.44 (m, 2 H), 2.15 - 2.20 (s, 9 H) 2.07 (s, 9 H), 1.96 - 2.03 (m, 18 H), 1.65 (br s, 4 H), 1.44 (br d, J = 7.28 Hz, 2 H), 1.14 - 1.24 (m, 12 H).³¹P NMR (CDCl₃): ppm δ 147.15.

The GalNAc ligand phosphoramidite compound GLPA2 was synthesized using the same procedure, except for using the intermediate-B.¹H NMR (400 MHz, CDCl₃): ppm δ 7.94 - 8.18 (m, 1 H), 7.69 (br s, 1 H), 6.66 - 7.10 (m, 3 H), 5.35 (d, *J* = 3.5 Hz, 3 H), 5.07 - 5.25 (m, 3 H), 4.76 - 4.86 (m, 3 H), 4.01 - 4.31 (m, 10 H), 3.91 - 4.01 (m, 8 H), 3.74 - 3.86 (m, 4 H), 3.52 - 3.71 (m, 30 H), 3.42 - 3.50 (m, 6 H), 3.15 - 3.25 (m, 4 H), 2.52 - 2.70 (m, 4 H), 2.22 - 2.45 (m, 2 H), 2.15 - 2.22 (s, 9 H), 2.06 (s, 9 H), 1.95 - 2.03 (m, 18 H), 1.77 (br s, 2 H), 1.58 - 1.66 (m, 4 H), 1.40 (m, 2 H), 1.08 - 1.24 (m, 12 H). ³¹P NMR (CDCl₃): ppm δ 147.12.

GLPA15 was prepared according to scheme 3 below.

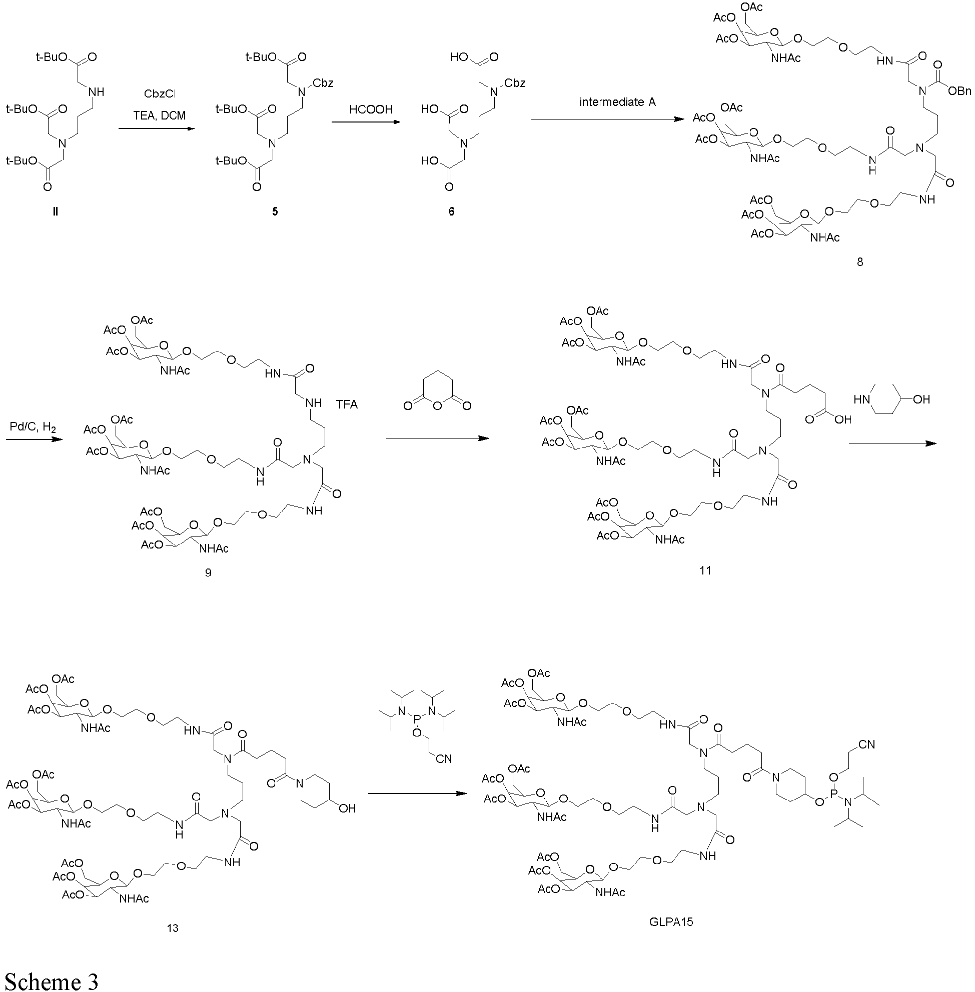

To a solution of intermediate compound II (275 g, 660 mmol, 1.00 eq.) in dichloromethane (2.75 L) was added triethylamine (133 g, 1.32 mol, 2.00 eq.), followed by the dropwise addition of Cbz-Cl (169 g, 990 mmol, 1.50 eq.). The reaction liquid was stirred at 25 °C for 2 h, and LCMS showed that compound II was completely converted. The reaction liquid was washed with a saturated NaHCO₃ solution (800 mL) and saturated sodium chloride aqueous solution (500 mL) in sequence, and the organic phase was dried over anhydrous Na₂SO₄. After being filtered to remove the desiccant, the filtrate was concentrated to dry. The residue was subjected to column chromatography (SiO₂, PE/EA=100/1 to 5/1) to give compound **5** (290 g, 527 mmol, yield 75.7%) as a colorless oil.¹H NMR (400 MHz in DMSO-d₆): δ ppm 7.23 - 7.40 (m, 5 H), 5.00 - 5.12 (m, 2 H), 3.86 - 3.95 (m, 2 H), 3.23 - 3.39 (m, 6 H), 2.55 - 2.67 (m, 2 H), 1.56 - 1.64 (m, 2 H), 1.31 - 1.46 (m, 27 H). MS (ESI) [M+H]⁺ m/z: **551.6_{∘}**

HCOOH (2.9 L) was added to compound 5 (145 g, 263 mmol, 1.00 eq), and the solution was stirred at 60 °C for 12 h. LCMS showed that compound 5 was completely converted. 1.5 L of toluene and 1.5 L of acetonitrile were added to the reaction liquid and the mixture was concentrated under reduced pressure to about 500 mL. Then toluene/acetonitrile (1:1, about 750 mL) was added and the mixture was concentrated to about 500 mL. Then acetonitrile (about 1000 mL) was added and the mixture was concentrated to dryness. The crude product was crushed with 700 mL of acetonitrile at 60 °C for 2 h and filtered. The solid was collected and dried to obtain white solid compound 6 (105 g, quantitative).¹H NMR (400 MHz inDMSO-d₆): δ ppm 7.26 - 7.40 (m, 5 H), 5.02 - 5.10 (m, 2 H), 3.89 - 4.00 (m, 2 H), 3.36 - 3.45 (m, 4 H), 3.24 - 3.34 (m, 2 H), 2.59 - 2.72 (m, 2 H), 1.40 (s, 2 H). MS (ESI) [M+H]⁺m/z: **383.0_{∘}**

TBTU (327 g, 1.02 mol, 3.90 eq.) and triethylamine (212 g, 2.09 mol, 8.00 eq.) were added to a solution of compound **6** (100 g, 261 mmol.) and intermediate-A(502 g, 915. mmol, 3.50 eq.) in DMF (1.0 L), and the reaction was carried out at 25 °C for 1 h. LCMS showed that the conversion of compound **6** was complete. The reaction liquid was added to 4000 mL of water and extracted with methyl tert-butyl ether (2000 mL, in two portions) to remove impurities, and the remaining aqueous phase was extracted with dichloromethane (3000 mL, in two portions). The dichloromethane phase was washed successively with 10% aqueous citric acid solution (2000 mL, in two portions), saturated NaHCO₃ (2.0 L, in two portions), and saturated brine (2.0 L), and dried over anhydrous Na₂SO₄. The filtrate was filtered and concentrated under reduced pressure to give compound 8 (260 g, 159 mmol, yield 60.9%) as a white solid.¹H NMR (400 MHz in DMSO-d₆): δ ppm 7.99 - 8.08 (m, 2 H), 7.93 (br d, *J*=5.50 Hz, 1 H), 7.79 - 7.86 (m, 3 H), 7.26 - 7.39 (m, 5 H), 5.22 (d, *J*=3.13 Hz, 3 H), 4.95 - 5.08 (m, 5 H), 4.54 (br d, *J*=8.38 Hz, 3 H), 4.03 (s, 9 H), 3.81 - 3.93 (m, 5 H), 3.76 (br d, *J*=4.88 Hz, 3 H), 3.44 - 3.62 (m, 10 H), 3.34 - 3.43 (m, 6 H), 3.24 (br d, *J*=6.13 Hz, 7 H), 3.02 - 3.09 (m, 4 H), 2.40 - 2.47 (m, 2 H), 2.10 (s, 9 H), 1.99 (s, 9 H), 1.89 (s, 9 H), 1.77 (s, 9 H), 1.57 - 1.68 (m, 2 H). MS (ESI) [M+H]⁺m/z: **816.4_{∘}**

A 2 L hydrogenation autoclave was inertized with argon and dry Pd/C (9 g) was carefully added. MeOH (50 mL) was added to wet the Pd/C, and then a solution of compound **8** (90 g, 55.1 mmol, 1.00 eq.) and trifluoroacetic acid (6.29 g, 55.1 mmol, 1.00 eq.) in MeOH (850 mL) was slowly added under argon atmosphere. The mixture was degassed/subjected to H₂ replacement three times to a hydrogen atmosphere and stirred at 25 °C for 10 h. LCMS showed that compound **8** was completely converted. Pd/C was removed by filtration, and the filtrate was concentrated under reduced pressure to give compound **9** (80 g, yield 90.2%).. ¹H NMR (400 MHz in DMSO-d₆): δ ppm 9.12 (br s, 2 H), 8.50 (br t, *J*=5.19 Hz, 1 H), 8.10 (br t, *J*=5.50 Hz, 2 H), 7.85 - 7.91 (m, 3 H), 5.22 (d, *J*=3.25 Hz, 3 H), 4.95 - 5.01 (m, 3 H), 4.52 - 4.58 (m, 3 H), 4.03 (s, 9 H), 3.84 - 3.93 (m, 3 H), 3.75 - 3.83 (m, 3 H), 3.39 - 3.61 (m, 16 H), 3.23 - 3.32 (m, 6 H), 3.15 - 3.18 (m, 3 H), 2.97 - 3.05 (m, 2 H), 2.54 - 2.61 (m, 2 H), 2.10 (s, 9 H), 2.00 (s, 9 H), 1.89 (s, 9 H), 1.77 - 1.80 (m, 9 H), 1.70 - 1.76 (m, 2 H). MS (ESI) [M+H]⁺m/z: **749.3_{∘}**

Triethylamine (67.8 g, 672 mmol, 4.00 eq) was added to a solution of compound **9** (270 g, 168 mmol, 1.00 eq.) and glutaric anhydride (28.6 g, 252 mmol, 1.50 eq) in dichloromethane (2.7 L). The solution was stirred at 25 °C for 1 h. LCMS showed that compound **9** was completely converted to compound **11.** 4-hydroxypiperidine (42.4 g, 420 mmol, 2.50 eq.) and TBTU (107 g, 335 mmol, 2.00 eq.) were added to the reaction liquid, and stirring was continued at 25 °C for 1 h. LCMS showed that the conversion of compound 11 was complete. The reaction was quenched by slowly adding saturated NH₄Cl (3.0 L), the layers were separated, and the aqueous phase was extracted with dichloromethane (2 × 1000 mL) and combined with the previous organic phase. The combined organic phases were washed with a 1:1 mixture of saturated NaHCO₃ (aq) and saturated brine (3.0 L), dried over anhydrous Na₂SO₄, filtered, and concentrated under reduced pressure. The crude product was dissolved in 1.5 L of dichloromethane and the obtained solution was added dropwise to methyl tert-butyl ether (7.5 L). A translucent white precipitate gradually formed during the addition. The precipitate was filtered in vacuum, and the solid was collected and dried in vacuum to give compound 13 (207 g, yield 72.8%) as a white solid. ¹H NMR (400 MHz in DMSO-d₆): δ ppm 8.05 (br d, *J* = 2.00 Hz, 2 H), 7.82 (br d, *J*=7.38 Hz, 3 H), 5.21 (br s, 3 H), 4.98 (br d, *J*=10.26 Hz, 3 H), 4.72 (br s, 1 H), 4.54 (br d, *J*=7.88 Hz, 3 H), 4.03 (br s, 9 H), 3.74 - 3.94 (m, 9 H), 3.45 - 3.71 (m, 12 H), 3.40 (br s, 6 H), 3.24 (br s, 7 H), 3.07 (br d, *J*=14.13 Hz, 5 H), 2.91 - 3.01 (m, 1 H), 2.24 - 2.44 (m, 5 H), 2.20 (br s, 1 H), 2.10 (s, 9H), 1.96 - 2.04 (m, 9H), 1.89 (br s, 9 H), 1.74 - 1.81 (m, 9 H), 1.51 - 1.73 (m, 6 H), 1.07 - 1.36 (m, 3 H). MS (ESI) [M+H]⁺m/z: 848.0_{∘}

3-bis(diisopropylamino)phosphonyloxypropionitrile (53.3 g, 177 mmol, 1.50 eq.) was added to a solution of compound **13** (200 g, 118 mmol, 1.00 eq.) and tetrazole diisopropylammonium (8.08 g, 47.2 mmol, 0.40 eq) in dichloromethane (2.0 L), and the reaction liquid was stirred at 40 °C for 2 h. LCMS showed that the conversion of compound 13 was complete. The reaction liquid was washed with a 1:1 mixture of saturated NaHCO₃ and saturated sodium chloride aqueous solution (2.0 L), dried over anhydrous Na₂SO₄, and the filtrate was concentrated and the resulting crude product was dissolved in dichloromethane (1.2 L) and the obtained solution was added dropwise to stirred methyl tert-butyl ether (6.0 L). The suspension was filtered, the filter cake was rinsed with methyl tert-butyl ether, the solid was collected and dried in vacuum, and the product was dissolved in dichloromethane (1.0 L) and concentrated to dryness, and the operation was repeated 4 times to remove the residual tert-butyl ether to give GLPA15 (164 g, yield 73.3%). ¹H NMR (400 MHz in DMSO-d6): δ ppm 8.05 (br d, J = 6.50 Hz, 2 H), 7.81 (br d, J=9.01 Hz, 3 H), 5.22 (d, J=3.25 Hz, 3 H), 4.98 (dd, J=11.26, 3.25 Hz, 3 H), 4.55 (br d, J=8.50 Hz, 3 H), 4.03 (s, 9 H), 3.64 - 3.97 (m, 12 H), 3.55 - 3.63 (m, 6 H), 3.50 (br s, 5 H), 3.40 (br d, J=6.13 Hz, 6 H), 3.17 - 3.30 (m, 9 H), 3.07 (br d, J=14.26 Hz, 4 H), 2.76 (t, J=5.82 Hz, 2 H), 2.18 - 2.47 (m, 6 H), 2.10 (s, 9 H), 1.99 (s, 9 H), 1.89 (s, 9 H), 1.78 (s, 9 H), 1.52 - 1.74 (m, 6 H), 1.12 - 1.19 (m, 12 H). 31P NMR (DMSO-d6): ppm δ 145.25. MS (ESI) [M+H]+ m/z: 1895.7_{∘}

In certain studies, methods are provided for linking a targeting group comprising GalNAc (herein also referred to as GalNAc delivery compound) to the 5'-end of a sense strand, comprising using GalNAc phosphoramidite (GLPA1) in the last coupling step of solid phase synthesis and using a synthesis process, such as a synthesis process that is used during oligonucleotide strand extension (i.e., adding nucleotides at the 5'-end of the sense strand) to link GLPA1 to the 5'-end of the sense strand.

In some studies, methods for linking GalNAc-containing targeting groups to the 3'-end of the sense strand include using a solid support (CPG) comprising GLO-n. In some studies, the method for linking a targeting group comprising GalNAc to the 3'-end of a sense strand includes: linking the GalNAc targeting group to the CPG solid support via an ester bond and using the resulting CPG with the linked GalNAc targeting group when the sense strand is synthesized, which results in the GalNAc targeting group being linked to the 3'-end of the sense strand. Other GalNAc phosphoramidite compounds (GLPAn) can also be obtained by using a reasonable corresponding intermediate and using a method similar to that described herein or known in the art, and can be linked to a suitable position of the siRNA duplex as a targeting group.

### Example 4. Synthesis of isomannitol phosphoramidite (Compound 2)

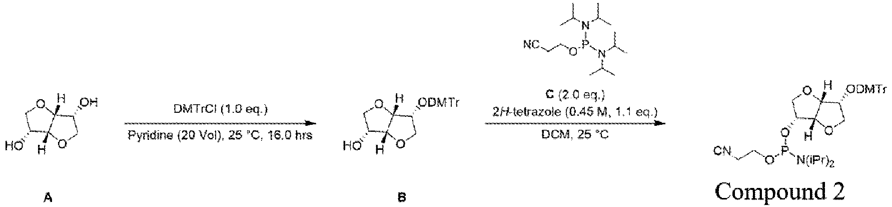

4,4'-dimethoxytriphenylmethane chloride (DMTrCl, 232 g, 684 mmol, 1.0 eq.) in pyridine (400 ml) was added to a solution of compound A (isomannitol, 100 g, 684 mmol, 1.0 eq.) in pyridine (600 ml), and the mixture was stirred at 25 °C for 16 h. LC-MS showed that compound A was completely consumed and a major peak with the desired mass was detected. The resulting reaction mixture was diluted with water (500 mL), extracted with dichloromethane (500 mL*2), the combined organic phases were washed with brine (500 mL), dried over Na₂SO₄ and concentrated in vacuum to obtain a residue. The residue was purified by column chromatography (DCM/MeOH = 100/1 to 50/1, 0.1% Et₃N) to give compound B (150 g, yield 48.9%) as a yellow solid. ¹H NMR: EC4783-404-P1B1_C (400 MHz, DMSO-*d₆*)
*δ* ppm 7.46 (br d, *J*=7.63 Hz, 2 H) 7.28 - 7.37 (m, 6 H) 7.19 - 7.25 (m, 1 H) 6.90 (br d, *J*=7.88 Hz, 4 H) 4.70 (d, *J*=6.50 Hz, 1 H) 3.99 - 4.09 (m, 6 H) 3.88 - 3.96 (m, 2 H) 3.83 (br dd, *J*=7.82, 6.94 Hz, 1 H) 3.74 (s, 6 H) 3.41 (br t, *J*=8.13 Hz, 1 H) 3.05 (t, *J*=8.44 Hz, 1 H) 2.85 (br t, *J*=7.50 Hz, 1 H).

2H-tetrazole (0.45 M, 436 mL, 1.1 eq) was added dropwise to a solution of compound B (80.0 g, 178 mmol, 1.0 eq) in dichloromethane (5.0 mL) at 25 °C under N₂ atmosphere, and then a solution of compound C (2-cyanoethyldiisopropylchlorophosphoramidite, 80.6 g, 267 mmol, 85.0 mL, 1.5 eq) in dichloromethane (200 mL) was added dropwise; the reaction mixture was stirred at 25 °C for 1.0 h; LC-MS showed that compound B was completely consumed and a major peak with the desired mass was detected. The resulting reaction mixture was cooled to -20 °C, poured into ice-cold NaHCO₃ (500 mL), extracted with dichloromethane (500 mL * 3), and the combined organic layers were washed with NaHCO₃/brine = 1: 1 (300 mL/300 mL), dried over Na₂SO₄, and concentrated in vacuum (35 °C) to obtain a residue (100 mL). The residue was purified by column chromatography (Al₂O₃, DCM/MeOH=100/1 to 50/1, 0.1% Et₃N) to give the the isomannitol phosphoramidite compound 2 (77 g, 119 mmol, yield: 66.5%) as a white solid.
¹H NMR: EC4783-423-P1B1_C (400 MHz, DMSO-*d₆*)
*δ* ppm 7.22 (br d, J=7.50 Hz, 2 H) 7.05 - 7.14 (m, 6 H) 6.96 - 7.02 (m, 1 H) 6.67 (br dd, J=8.82, 1.81 Hz, 4 H) 3.95 - 4.07 (m, 2 H) 3.73 - 3.83 (m, 1 H) 3.62 - 3.72 (m, 2 H) 3.48 - 3.53 (m, 6 H) 3.27 - 3.37 (m, 3 H) 3.11 (s, 6 H) 2.82 (td, J=8.54, 2.31 Hz, 1 H) 2.47 - 2.63 (m, 3 H) 2.28 (br d, J=1.63 Hz, 3 H) 0.82 - 1.00 (m, 13 H).

### Example 5. Preparation of solid support containing isomannitol monomer

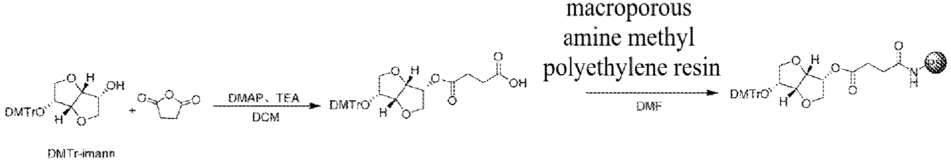

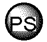

represents the macroporous amine methyl polyethylene resin support moiety.

A 50 L glass kettle was placed under the protection of nitrogen, dichloromethane (19.50 kg) was added to the glass kettle, and the stirring was started. The temperature was controlled at 20-30 °C, DMTr-imann (1.47 kg) was added to the glass kettle, triethylamine (1.50 kg), 4-dimethylaminopyridine (0.164 kg) and succinic anhydride (1.34 kg) were added to the reaction kettle. The system was kept warm at 20 - 30 °C for 18 h and then sampled, and the reaction was terminated. The saturated sodium bicarbonate solution (22.50 kg) was added to the reaction system, stirred for 10-20 min and then stood until layered, the organic phase in the lower layer was transferred, and the aqueous phase in the upper layer was extracted with dichloromethane twice; the organic phases were combined, and dried over anhydrous sodium sulfate, the filtrate was filtrated, and then transferred for rotary evaporation and concentrated to no fraction, forming a gray to quasi-white solid 1.83 kg.

N,N-dimethylformamide (23.50 kg) was added to a 100 L glass kettle and stirred. The temperature was controlled at 20-30 °C. Under nitrogen protection, the product from the previous step, O-benzotriazole-tetramethyluronium hexafluorophosphate (0.33 kg) and N,N-diisopropylethylamine (0.13 kg) were added to the 100 L glass kettle through a solid feeding funnel. After the addition was completed, the mixture was stirred for 10-30 min and then discharged into a 50 L galvanized bucket for standby use. Macroporous amino-methyl resin (3.25 kg) (available from Tianjin Nankai Hecheng Technology Co., Ltd., batch number HA2X1209, loading capacity 0.48 mmol/g) was added to the above-mentioned 100 L solid phase synthesis kettle through a solid feeding funnel, the temperature was controlled at 20-30 °C, N,N-dimethylformamide (21.00 kg + 21.00 kg) and the reaction liquid to be used in the galvanized bucket in previous step were added to the solid phase synthesis kettle. The system was reacted under temperature-controlled conditions, monitored until a solid load ≥ 250 umol/g, and the load detection method was UV The system was subjected to pressure filtration under nitrogen, and the filter cake was rinsed three times with N,N-dimethylformamide (26.00 kg + 26.10 kg + 26.00 kg), and the filter cake remained in the kettle. CAP. A (4.40 kg + 4.42 kg + 4.30 kg) and CAP.B (4.40 kg + 4.40 kg + 4.47 kg) were added into 80 L glass kettle and stirred for 3 - 8 min. This operation was repeated 3 times for capping. Acetonitrile (18.00 kg + 18.00 kg + 18.00 kg + 17.50 kg + 17.50 kg) was added to the solid-phase synthesis kettle. Nitrogen was bubbled for 10-30 min before pressure filtration. This operation was repeated four times. The filter cake was purged with nitrogen in the solid-phase synthesis kettle for 2-4 h and then transferred to a 50 L pressure filter tank. The temperature was controlled at 15-30 °C and drying was continued. The yellow to white solid product after drying was weighted: 3.516 kg.

Isosorbide residues (imann) may be added to the 5'-end or 3'-end of the oligonucleotide strand by a process well known to those skilled in the art, such as the reverse abasic process (invab), and a targeting group was further added.

### Example 6. In vitro screening of LPA siRNA duplexes using Huh7 cells and dual fluorescent reporter gene vector

Huh7 cells were adjusted to the appropriate density and then seeded into 96-well plates. According to the manufacturer's recommendations, at the same time as inoculation, the dual fluorescent reporter gene vector psciCHECK2 containing the target gene was co-transfected with siRNA into Huh7 cells using Lipofectamine RNAiMax (Invitrogen-13778-150). Cells were transfected with test siRNAs or control siRNAs. SiRNAs were tested in triplicate at two concentrations (0.1 nM and 1.0 nM), and 48 h after transfection, Dual-Glo ^{®} Luciferase Assay Reagent was added to detect fluorescence values. The ratio of Renilla luminescence to firefly luminescence was calculated and normalized based on the ratio of control siRNA-treated samples to calculate knockdown efficiency. As a result, as shown in Table 4, the duplex AV# used is derived from the sequence corresponding to that shown in Table 2.

**Table 4 provides experimental results from in vitro studies of inhibition of LPA expression using a plurality of LPA RNAi agents.**

| **Compound ID** | **% inhibition** | | | | | | **Mean ± SD** | |
|---|---|---|---|---|---|---|---|---|
| | **1nM** | | | **0.1nM** | | | **1nM** | **0.1nM** |
| AV00122 | 46.88 | 39.56 | 29.62 | -10.77 | -1.49 | -18.62 | 38.69±8.66 | -10.29±8.57 |
| AV00123 | 42.34 | 26.55 | 36.79 | -7.68 | -31.59 | -10.12 | 35.23±8.01 | -16.46±13.16 |
| AV00124 | 62.09 | 60.30 | 65.01 | 23.45 | 32.98 | 27.50 | 62.47±2.38 | 27.98±4.78 |
| AV00125 | 25.96 | 19.54 | 13.62 | 10.87 | 5.64 | 8.35 | 19.71±6.17 | 8.28±2.62 |
| AV00126 | 57.79 | 49.37 | 49.63 | 24.96 | 28.22 | 13.57 | 52.26±4.79 | 22.25±7.69 |
| AV00127 | 60.31 | 51.67 | 54.57 | 22.52 | 12.95 | 13.87 | 55.52±4.4 | 16.44±5.28 |
| AV00128 | 14.45 | 19.71 | 8.00 | 14.39 | 2.08 | -5.46 | 14.05±5.87 | 3.67±10.02 |
| AV00129 | 37.12 | 38.30 | 20.18 | 26.01 | 12.46 | -1.20 | 31.87±10.14 | 12.42±13.61 |
| AV00130 | 5.18 | 5.32 | 10.82 | 21.04 | 18.38 | 13.33 | 7.11±3.22 | 17.58±3.92 |
| AV00131 | 30.07 | 31.44 | 38.14 | 8.51 | 25.97 | 23.80 | 33.22±4.32 | 19.43±9.51 |
| AV00132 | 26.46 | 16.87 | 24.53 | 26.67 | -5.93 | 18.47 | 22.62±5.07 | 13.07±16.96 |
| AV00133 | 30.64 | 13.58 | 30.46 | 1.27 | 16.57 | 14.84 | 24.9±9.8 | 10.89±8.38 |
| AV00134 | -1.69 | 23.47 | 3.75 | -10.06 | -11.46 | 2.31 | 8.51±13.24 | -6.4±7.58 |
| AV00135 | -0.25 | 15.11 | 11.55 | -0.35 | -1.05 | -10.59 | 8.8±8.04 | -4±5.72 |
| AV00136 | -5.77 | 2.13 | 5.80 | 10.89 | 14.96 | 2.82 | 0.72±5.91 | 9.56±6.18 |
| AV00137 | 19.35 | 21.06 | 29.49 | 15.89 | 13.70 | 10.16 | 23.3±5.43 | 13.25±2.89 |
| AV00138 | 48.10 | 46.49 | 44.67 | 19.50 | 13.68 | 19.53 | 46.42±1.71 | 17.57±3.37 |
| AV00139 | 61.67 | 63.90 | 49.21 | 26.21 | 27.72 | 25.37 | 58.26±7.92 | 26.44±1.19 |
| AV00140 | 34.63 | 29.60 | 28.49 | 16.87 | 25.81 | 23.73 | 30.91±3.27 | 22.14±4.68 |
| AV00141 | 37.26 | 40.08 | 34.19 | 24.02 | 10.49 | 7.39 | 37.17±2.95 | 13.96±8.84 |
| AV00142 | 31.56 | 38.44 | 47.32 | 32.86 | 30.04 | 7.52 | 39.11±7.9 | 23.47±13.89 |
| AV00143 | 16.55 | 5.61 | 25.08 | 2.56 | 1.09 | 14.66 | 15.75±9.76 | 6.1±7.44 |
| AV00144 | 34.87 | 36.47 | 22.09 | -1.37 | 23.23 | -2.57 | 31.14±7.88 | 6.43±14.56 |
| AV00145 | 12.58 | 20.84 | 23.93 | 6.89 | 17.73 | 18.90 | 19.12±5.87 | 14.51±6.62 |
| AV00146 | 17.23 | 2.18 | 35.70 | 9.75 | -14.56 | 9.86 | 18.37±16.79 | 1.68±14.07 |
| AV00147 | 37.07 | 29.40 | 30.89 | -12.86 | 12.78 | 3.28 | 32.46±4.07 | 1.07±12.96 |
| AV00148 | 18.91 | 24.27 | 20.66 | 15.39 | -0.29 | 10.06 | 21.28±2.74 | 8.38±7.97 |
| AV00149 | 34.67 | 35.51 | 42.00 | 17.63 | 14.16 | 0.91 | 37.39±4.01 | 10.9±8.83 |
| AV00150 | 23.64 | 32.49 | 39.08 | 8.26 | 27.22 | 4.10 | 31.74±7.75 | 13.2±12.32 |
| AV00151 | 29.63 | 18.23 | 33.40 | -11.53 | 4.85 | 3.28 | 27.08±7.9 | -1.14±9.04 |
| AV00152 | 7.14 | 8.73 | 11.52 | -10.32 | 7.00 | 19.93 | 9.13±2.22 | 5.54±15.18 |
| AV00153 | 15.18 | 17.10 | 11.01 | -2.68 | 18.17 | 8.92 | 14.43±3.11 | 8.14±10.45 |
| AV00154 | 3.76 | 8.40 | -2.82 | 3.55 | 11.84 | 23.76 | 3.11±5.64 | 13.05±10.16 |
| AV00155 | 40.39 | 35.99 | 24.94 | 20.29 | 4.72 | 8.22 | 33.78±7.96 | 11.08±8.17 |
| AV00156 | 48.03 | 53.87 | 44.46 | 19.65 | 4.24 | 0.54 | 48.79±4.75 | 8.14±10.14 |
| AV00157 | 5.83 | 0.73 | 14.54 | 8.81 | 0.28 | -5.20 | 7.03±6.98 | 1.29±7.06 |
| AV00158 | -25.20 | -13.67 | -10.50 | -19.74 | -27.38 | -8.06 | -16.46±7.74 | -18.39±9.73 |
| AV00159 | -0.49 | -1.23 | 3.57 | 12.19 | 7.24 | -8.92 | 0.62±2.59 | 3.5±11.04 |
| AV00160 | -14.61 | -0.51 | -16.26 | -13.95 | 4.74 | -36.43 | -10.46±8.65 | -15.21±20.61 |
| AV00161 | 37.16 | 23.76 | 20.23 | 0.03 | 3.95 | 4.53 | 27.05±8.94 | 2.84±2.45 |
| AV00162 | 15.17 | 30.06 | 26.53 | 1.97 | 4.32 | 9.94 | 23.92±7.78 | 5.41±4.1 |
| AV00163 | 26.51 | 6.32 | 4.45 | 3.68 | -6.42 | -0.56 | 12.43±12.23 | -1.1±5.08 |
| AV00164 | 12.92 | 3.15 | 0.52 | 4.68 | -0.72 | -5.31 | 5.53±6.54 | -0.45±5 |
| AV00165 | 8.82 | 27.01 | -8.40 | 6.07 | -1.25 | -4.54 | 9.14±17.71 | 0.09±5.43 |
| AV00166 | -0.92 | -11.91 | 4.33 | -1.21 | 5.41 | 7.70 | -2.83±8.29 | 3.97±4.63 |
| AV00167 | 38.80 | 26.25 | 13.39 | 16.98 | 9.31 | 1.68 | 26.14±12.7 | 9.32±7.65 |
| AV00168 | 50.66 | 44.21 | 42.59 | -3.68 | 18.09 | 24.86 | 45.82±4.27 | 13.09±14.91 |
| AV00169 | -4.71 | 1.84 | -6.06 | -7.67 | 0.35 | -1.47 | -2.97±4.23 | -2.93±4.2 |
| AV00170 | -26.60 | -24.07 | -18.79 | -25.70 | 8.71 | 12.90 | -23.15±3.98 | -1.36±21.18 |
| AV00171 | -9.86 | 0.81 | 8.35 | -18.50 | -8.10 | -28.33 | -0.23±9.15 | -18.31±10.11 |
| AV00172 | 21.83 | 29.14 | 21.85 | 14.74 | 8.44 | -2.30 | 24.28±4.21 | 6.96±8.62 |
| AV00173 | 18.80 | 11.14 | 9.85 | 0.53 | 4.28 | 2.49 | 13.26±4.84 | 2.44±1.87 |
| AV00174 | 20.20 | 3.18 | 27.08 | 0.74 | 11.08 | -3.33 | 16.82±12.3 | 2.83±7.43 |
| AV00175 | 17.95 | 12.15 | 13.36 | 12.15 | 17.57 | -0.95 | 14.49±3.06 | 9.59±9.52 |
| AV00176 | 44.25 | 48.81 | 43.11 | 7.78 | 25.40 | 20.96 | 45.39±3.02 | 18.05±9.16 |
| AV00177 | 23.93 | 36.34 | 27.92 | 23.12 | -5.39 | 9.75 | 29.4±6.34 | 9.16±14.26 |
| AV00178 | 0.00 | 6.42 | -9.82 | 9.86 | 18.29 | 4.30 | -1.13±8.18 | 10.82±7.04 |
| AV00179 | 10.09 | 0.83 | 15.90 | 17.67 | 0.27 | -3.09 | 8.94±7.6 | 4.95±11.15 |
| AV00180 | 23.19 | 31.99 | 32.14 | 1.35 | -1.15 | 10.37 | 29.11±5.12 | 3.52±6.06 |
| AV00181 | -2.78 | -11.51 | -6.05 | 3.14 | 3.68 | 17.42 | -6.78±4.41 | 8.08±8.1 |
| AV00182 | -42.00 | -13.22 | -16.52 | -18.26 | -6.23 | -5.03 | -23.91±15.75 | -9.84±7.32 |
| AV00183 | -6.80 | 7.22 | -13.19 | -5.60 | -3.55 | -10.33 | -4.26±10.44 | -6.49±3.48 |
| AV00184 | -19.87 | -3.42 | 2.48 | -11.82 | 5.71 | -5.44 | -6.93±11.58 | -3.85±8.87 |
| AV00185 | 7.87 | 12.20 | 6.68 | 7.79 | 20.31 | 8.12 | 8.92±2.91 | 12.07±7.14 |
| AV00186 | 16.29 | 14.22 | 8.71 | 4.34 | 1.60 | 19.64 | 13.07±3.92 | 8.53±9.72 |
| AV00187 | 27.62 | 23.09 | 32.62 | -0.06 | -7.08 | -2.73 | 27.78±4.77 | -3.29±3.55 |
| AV00188 | 25.32 | 11.82 | 9.96 | 6.89 | 2.45 | 5.45 | 15.7±8.39 | 4.93±2.26 |
| AV00189 | 36.90 | 25.16 | 27.89 | 9.72 | 26.25 | 13.84 | 29.98±6.14 | 16.6±8.61 |
| AV00190 | -19.33 | -3.61 | 17.31 | 2.40 | 13.23 | 6.97 | -1.88±18.38 | 7.53±5.44 |
| AV00191 | -8.17 | 2.85 | -14.56 | 7.21 | 6.42 | 4.45 | -6.63±8.81 | 6.03±1.42 |
| AV00192 | -15.69 | 7.96 | 7.26 | 5.66 | 16.15 | 10.03 | -0.16±13.46 | 10.61±5.27 |
| AV00193 | -4.92 | 15.62 | -2.27 | 2.99 | -3.76 | 3.73 | 2.81±11.17 | 0.98±4.13 |
| AV00194 | -33.87 | -19.43 | -28.60 | -43.88 | -20.84 | -7.36 | -27.3±7.31 | -24.03±18.47 |
| AV00195 | 0.20 | 18.94 | 13.75 | -12.53 | -16.30 | -4.18 | 10.96±9.68 | -11±6.21 |
| AV00196 | 13.36 | 2.83 | 9.10 | -11.87 | 1.83 | -11.47 | 8.43±5.3 | -7.17±7.8 |
| AV00197 | 16.31 | 10.80 | 17.97 | 15.72 | 3.62 | -12.32 | 15.03±3.75 | 2.34±14.07 |
| AV00198 | 9.66 | -9.58 | 13.15 | 11.57 | 25.56 | 9.67 | 4.41±12.24 | 15.6±8.67 |
| AV00199 | 1.16 | 4.54 | 7.07 | 18.16 | -2.74 | 13.05 | 4.26±2.97 | 9.49±10.9 |
| AV00200 | 0.38 | 17.21 | 5.05 | 11.62 | -19.20 | -21.34 | 7.55±8.69 | -9.64±18.44 |
| AV00201 | 13.46 | 3.72 | -1.04 | -2.48 | -6.79 | 13.82 | 5.38±7.39 | 1.51±10.87 |
| AV00202 | 17.81 | 27.79 | 23.96 | 24.33 | 16.06 | 14.70 | 23.19±5.04 | 18.36±5.22 |
| AV00203 | 23.36 | 24.72 | 14.70 | 12.28 | 0.46 | 11.09 | 20.93±5.44 | 7.94±6.51 |
| AV00204 | 9.50 | 27.29 | 11.48 | -0.63 | 11.79 | -7.73 | 16.09±9.75 | 1.14±9.88 |
| AV00205 | 5.30 | 6.46 | 11.40 | -3.32 | 4.30 | -15.98 | 7.72±3.24 | -5±10.24 |

### Example 7. In vivo test of LPA siRNA duplex

To evaluate the in vivo activity of LPA siRNAs, mice infected with AAV encoding human LPA and luciferase gene were used (4 mice per group). Female C57BL/6J mice were infected 14 days before siRNA administration by intravenous injection of a stock of 2 × 10 ^ 11 viral particle of adeno-associated virus 8 (AAV8) vector encoding human LPA and luciferase gene. On day 0, mice were subcutaneously injected with a single dose of 6 mg/kg of LPA siRNA agent or PBS. Blood samples were collected on day 0, before siRNA administration and at the end of day 7. Luciferase activity was measured. The percentage of knockdown was calculated by comparing the luciferase activity of blood samples from the siRNA-treated group before administration and the luciferase activity of blood samples collected at the end of day 7 and performing normalization based on changes in luciferase activity in serum samples from the PBS-treated group. As a result, as shown in Table 5, the duplex AD# used is derived from the sequence corresponding to that shown in Table 3.

**Table 5 provides the experimental results from in vivo studies of inhibitory effect on LPA expression using a plurality of LPA_ RNAi agents at a single dose of 6 mpk. On day 7, the remaining luciferase activity relative to day 0 was normalized to the change in the PBS-treated group (mean ± SD)**

| Duplex AD# | On day 7, the luciferase activity relative to PBS (mean ± SD) |
|---|---|
| AD00433 | 0.71±0.23 |
| AD00434 | 0.66±0.33 |
| AD00438 | 0.69±0.22 |
| AD00436 | 0.22±0.07 |
| AD00441 | 0.54±0.08 |

### Example 8. In vivo test of LPA siRNA duplex

To evaluate the in vivo activity of LPA siRNAs, mice infected with AAV encoding human LPA and luciferase gene were used (4 mice per group). Female C57BL/6J mice were infected 14 days before siRNA administration by intravenous injection of a stock of 2 × 10 ^ 11 viral particle of adeno-associated virus 8 (AAV8) vector encoding human LPA and luciferase gene. On day 0, mice were subcutaneously injected with a single dose of 6 mg/kg of LPA siRNA agent or PBS. Blood samples were collected before siRNA administration on day 0, at the end of day 7 and day 14. Luciferase activity was measured and the percentage of knockdown was calculated by comparing the luciferase activity of blood samples from the siRNA-treated group before administration and the luciferase activity of blood samples collected on day 7 and day 14 and performing normalization based on changes in luciferase activity in serum samples from the PBS-treated group. The knockdown (retention) percentage of human LPA mRNA levels (determined by qPCR) in the liver of mice on day 14 between the siRNA-treated group and the PBS-treated group was compared, and the results are shown in Table 6. The duplex AD# used was derived from the sequences corresponding to those shown in Table 3.

**Table 6 provides the experimental results from in vivo studies of inhibition of LPA expression using a plurality of LPA_RNAi agents at a single dose of 6 mpk.**

| Duplex AD# | On day 7, the luciferase activity relative to PBS (mean ± SD) | On day 14, the luciferase activity relative to PBS (mean ± SD) | The percentage of human LPA mRNA retained in mouse liver was detected by qPCR (mean ± SD) |
|---|---|---|---|
| AD00474 | 0.36±0.05 | 0.31±0.18 | 0.36±0.24 |
| AD00475 | 0.53±0.56 | 0.36±0.27 | 0.14±0.1 |
| AD00476 | 0.73±0.46 | 0.38±0.1 | 0.17±0.08 |
| AD00477 | 1.11±0.3 | 0.64±0.14 | 0.82±0.14 |
| AD00478 | 0.73±0.39 | 0.61±0.17 | 0.38±0.31 |
| AD00479 | 0.48±0.26 | 0.48±0.07 | 0.18±0.11 |
| AD00480 | 0.31±0.18 | 0.22±0.14 | 0.14±0.12 |
| AD00481 | 0.93±0.35 | 0.72±0.32 | 0.57±0.12 |
| AD00482 | 0.47±0.37 | 0.4±0.33 | 0.15±0.08 |
| AD00474-1 | 0.48±0.1 | 0.3±0.1 | 0.33±0.4 |
| AD00478-1 | 0.48±0.09 | 0.32±0.05 | 0.32±0.1 |
| AD00482-1 | 0.4±0.1 | 0.34±0.07 | 0.18±0.13 |
| AD00483 | 0.49±0.16 | 0.6±0.21 | 0.65±0.4 |
| AD00484 | 0.83±0.11 | 1.08±0.35 | 0.77±0.32 |
| AD00485 | 0.41±0.09 | 0.54±0.14 | 0.49±0.16 |
| AD00486 | 0.4±0.19 | 0.69±0.09 | NA |
| AD00487 | 0.44±0.11 | 0.83±0.18 | NA |
| AD00484-1 | 0.51±0.16 | 0.64±0.35 | 0.45±0.49 |
| AD00342 | 0.6±0.18 | 0.58±0.13 | 0.59±0.21 |

| | | | |
|---|---|---|---|
| NA means not detected. | | | |

### Example 9. In vivo test of LPA siRNA duplex

To evaluate the in vivo activity of LPA siRNAs, mice infected with AAV encoding human LPA and luciferase gene were used (4 mice per group). Female C57BL/6J mice were infected 7 days before siRNA administration by intravenous injection of a stock of 2 × 10 ^ 11 viral particle of adeno-associated virus 8 (AAV8) vector encoding human LPA and luciferase gene. On day 0, mice were subcutaneously injected with a single dose of 3 mg/kg, 6 mg/kg and 10 mg/kg of LPA siRNA agent or PBS, respectively. Mice were sacrificed at termination. The knockdown (retention) percentage of human LPA mRNA levels (determined by qPCR) in the liver of mice on day 14 between the siRNA-treated group and the PBS-treated group was compared, and the results are shown in Table 7. The duplex AD# used was derived from the sequences corresponding to those shown in Table 3.

**Table 7 provides the experimental results from in vivo studies of inhibition of LPA expression using a plurality of LPA_RNAi agents at a single dose of 3 mpk, 6 mpk, and 10 mpk, respectively.**

| Duplex AD# | The percentage of human LPA mRNA retained in mouse liver was detected by qPCR (mean ± SD) | | Dose (mg/kg) |
|---|---|---|---|
| | Mean | SD | |
| AD00436 | 0.31 | 0.07 | 3 |
| | 0.29 | 0.07 | 6 |
| | 0.29 | 0.20 | 10 |
| AD00337 | 0.59 | 0.23 | 3 |
| | 0.72 | 0.39 | 6 |
| | 0.46 | 0.23 | 10 |

### Example 10. In vivo test of LPA siRNA duplex

To evaluate the in vivo activity of LPA siRNAs, mice infected with AAV encoding human LPA and luciferase gene were used (4 mice per group). Female C57BL/6J mice were infected 7 days before siRNA administration by intravenous injection of a stock of 2 × 10 ^ 11 viral particle of adeno-associated virus 8 (AAV8) vector encoding human LPA and luciferase gene. On day 0, mice were subcutaneously injected with a single dose of 2 mg/kg or 6 mg/kg of LPA siRNA agent or PBS. Blood samples were collected on day 0, before siRNA administration and at the end of day 7, 14 and 21, respectively. Luciferase activity was measured. The percentage of knockdown was calculated by comparing the luciferase activity of blood samples from the siRNA-treated group before administration and the luciferase activity of blood samples collected at the end of day 7, 14 and 21 and performing normalization based on changes in luciferase activity in serum samples from the PBS-treated group. As a result, as shown in Table 8, the duplex AD# used is derived from the sequence corresponding to that shown in Table 3.

**Table 8 provides the experimental results from in vivo studies of inhibition of LPA expression using a plurality of LPA_ RNAi agents at a single dose of 2 and 6 mpk, and on days 7, 14 and 21, the remaining luciferase activity relative to day 0 was normalized to the change in the PBS-treated group (mean ± SD).**

| Duplex AD# | The luciferase activity relative to PBS (mean ± SD) | | | Dose (mg/kg) |
|---|---|---|---|---|
| | Day 7 | Day 14 | Day 21 | |
| PBS | 1.00± 0.38 | 1.00± 0.23 | 1.00± 0.37 | |
| AD00436 | 0.53± 0.36 | 0.45± 0.09 | 0.40± 0.29 | 2 |
| AD00436 | 0.34± 0.07 | 0.30± 0.06 | 0.31± 0.11 | 6 |
| AD00474 | 0.98± 0.66 | 0.52± 0.20 | 0.66± 0.39 | 2 |
| AD00474 | 0.36± 0.16 | 0.19± 0.10 | 0.21± 0.07 | 6 |
| AD00475 | 0.48± 0.21 | 0.34± 0.17 | 0.34± 0.21 | 2 |
| AD00475 | 0.47± 0.20 | 0.15± 0.03 | 0.18± 0.12 | 6 |
| AD00476 | 0.75± 0.33 | 0.38± 0.13 | 0.52± 0.26 | 2 |
| AD00476 | 0.65± 0.04 | 0.36± 0.14 | 0.29± 0.16 | 6 |
| AD00479 | 0.53± 0.34 | 0.41± 0.19 | 0.53± 0.25 | 2 |
| AD00479 | 0.41± 0.17 | 0.34± 0.08 | 0.31± 0.17 | 6 |
| AD00480 | 0.35± 0.15 | 0.23± 0.02 | 0.24± 0.13 | 2 |
| AD00480 | 0.28± 0.07 | 0.15± 0.05 | 0.24± 0.08 | 6 |
| AD00482 | 0.71± 0.37 | 0.50± 0.13 | 0.56± 0.27 | 2 |
| AD00482 | 0.41± 0.33 | 0.27± 0.08 | 0.31± 0.08 | 6 |
| AD00474-1 | 0.42± 0.11 | 0.35± 0.11 | 0.29± 0.04 | 6 |
| AD00478-1 | 0.54± 0.15 | 0.28± 0.08 | 0.37± 0.01 | 6 |
| AD00482-1 | 0.24± 0.15 | 0.28± 0.14 | 0.15± 0.04 | 6 |
| AD00337 | 0.41± 0.19 | 0.62± 0.21 | 0.74± 0.16 | 2 |
| AD00337 | 0.47± 0.21 | 0.45± 0.33 | 0.37± 0.19 | 6 |
| AD00150 | 0.83± 0.35 | 0.74± 0.26 | 0.58± 0.10 | 2 |
| AD00150 | 0.41± 0.20 | 0.25± 0.09 | 0.28± 0.24 | 6 |

### Example 11. In vivo test of LPA siRNA duplex

To evaluate the in vivo activity of LPA siRNA, male cynomolgus monkeys (13-22 years old, weighing 7-9 kg) were recruited in this study, with 3 animals in each group. Each animal was subcutaneously injected with 2 mg/kg of the test article. The test articles used corresponded to the compounds shown in Table 3 (AD00377-1, AD00436-1, AD00480, AD00480-1, AD00480-2, and AD00474-2).

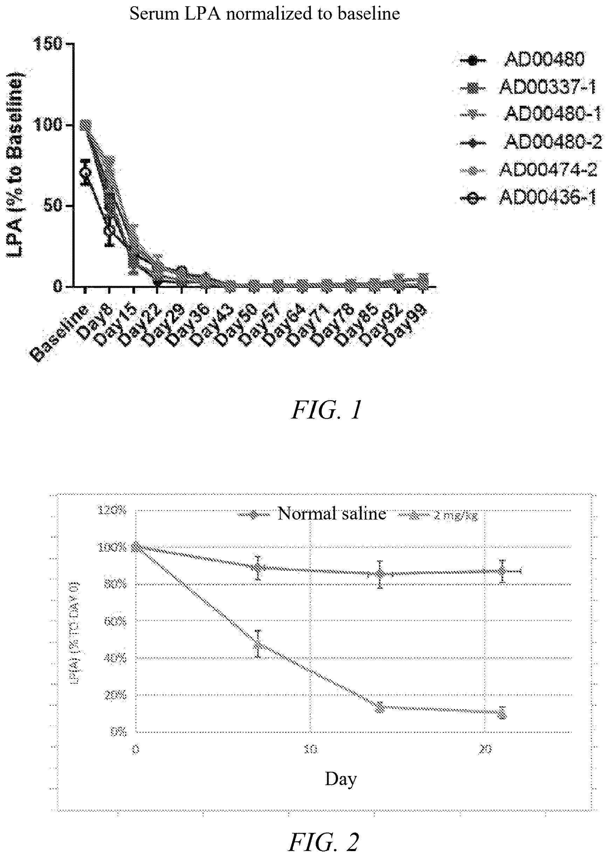

After fasting overnight, blood collection was performed on days -14 (before administration), -7 (before administration), 1 (before administration) and days 8, 15, 22, 29, 43, 50, 57, 64, 71, 78, 85, 92 and 99 after administration. The collected blood samples were left to clot at room temperature for at least 30 min and then centrifuged at 3500 rpm for 10 min at 4 °C. The collected serum (approximately 1.0 mL) was transferred to two pre-labeled polypropylene screw-capped vials (0.5 ml/vial, one for ELISA assay and the other spare) and stored in a refrigerator at -80 °C until testing. LPA remaining percentage (normalized to the mean of day -14 (before administration), -7 (before administration), and 1 (before administration) , pre-administration of siRNA) is shown in Fig. 1.

### Example 12. In vivo test of LPA siRNA duplex

To evaluate the in vivo activity of LPA siRNA, male cynomolgus monkeys (2-6 years old, weighing 2-6 kg) were recruited in this study, with 4 animals in each group. Each animal was subcutaneously injected with saline or 2 mg/kg of the AD00480-8 test product. The test product AD00480-8 used corresponds to the compound shown in Table 3. After fasting overnight, blood collection was performed on days -14 (before administration), 0 (before administration) and days 7, 14 and 21 after administration. The collected blood samples were left to clot at room temperature for at least 30 min and then centrifuged at 3500 rpm for 10 min at 4 °C. The collected serum (approximately 1.0 mL) was transferred to two pre-labeled polypropylene screw-capped vials (0.5 ml/vial, one for ELISA assay and the other spare) and stored in a refrigerator at -80 °C until testing. LPA remaining percentage (normalized to the mean of day -14 (before administration), and 0 (before administration), pre-administration of siRNA) is shown in Fig. 2.

### Example 13. In vitro screening of LPA siRNA duplexes using Huh7 cells and dual fluorescent reporter gene vector

Huh7 cells were adjusted to the appropriate density and then seeded into 96-well plates. According to the manufacturer's recommendations, at the same time as inoculation, the dual fluorescent reporter gene vector psciCHECK2 containing the target gene was co-transfected with siRNA into Huh7 cells using Lipofectamine RNAiMax (Invitrogen-13778-150). Cells were transfected with test siRNAs or control siRNAs. SiRNAs were tested in triplicate at two concentrations (0.1 nM and 1.0 nM), and 48 h after transfection, Dual-Glo ^{®} Luciferase Assay Reagent was added to detect fluorescence values. The ratio of Renilla luminescence to firefly luminescence was calculated and normalized based on the ratio of control siRNA-treated samples to calculate knockdown efficiency. As a result, as shown in Table 9, the duplex AV# used is derived from the sequence corresponding to that shown in Table 2.

**Table 9 provides experimental results from in vitro studies of inhibition of LPA expression using a plurality of LPA RNAi agents.**

| Compound | **% inhibition** (MEAN ± SD) | | Compound | **% inhibition** (MEAN ± SD) | |
|---|---|---|---|---|---|
| | 1nM | 0.1nM | | 1nM | 0.1nM |
| AV01867 | 46.80±4.94 | 20.67±6.22 | AV01921 | 43.98±3.11 | 10.24±2.51 |
| AV01868 | 49.97±3.00 | 21.34±5.62 | AV01922 | 48.03±3.11 | 16.51±1.01 |
| AV01873 | 22.50±6.76 | 6.04±3.83 | AV01923 | 50.53±4.02 | 30.41±4.87 |
| AV01874 | 20.31±1.71 | 1.22±8.37 | AV01924 | 57.72±3.14 | 34.72±1.99 |
| AV01875 | 27.87±1.62 | -5.41±4.13 | AV01925 | 49.37±3.12 | 28.89±5.77 |
| AV01876 | 25.57±6.31 | -7.12±10.15 | AV01926 | 57.83±4.08 | 38.92±1.85 |
| AV01877 | 32.85±3.12 | 7.02±1.04 | AV01927 | 32.41±9.25 | 16.81±3.52 |
| AV01878 | 37.56±0.72 | 2.90±8.20 | AV01928 | 58.81±3.31 | 40.57±2.02 |
| AV01879 | 38.63±0.47 | 14.23±4.04 | AV01929 | 58.05±4.18 | 33.99±5.29 |
| AV01880 | 36.24±5.54 | 9.07±11.68 | AV01930 | 58.89±3.50 | 35.67±3.53 |
| AV01881 | 45.01±3.67 | 19.69±5.00 | AV01931 | 49.37±2.20 | 19.50±3.95 |
| AV01882 | 35.40±2.43 | 7.05±4.97 | AV01932 | 30.69±0.79 | 4.38±5.60 |
| AV01883 | 44.89±0.46 | 13.21±3.78 | AV01934 | 34.14±3.07 | 13.58±4.37 |
| AV01884 | 26.17±1.54 | 12.79±1.48 | AV01935 | 44.01±2.35 | 16.32±4.15 |
| AV01885 | 25.52±7.87 | 10.49±2.89 | AV01936 | 50.96±4.79 | 24.30±9.44 |
| AV01892 | 53.83±2.87 | 25.26±5.37 | AV01937 | 48.58±2.39 | 14.95±3.03 |
| AV01893 | 36.28±2.27 | 6.31±2.85 | AV01938 | 32.80±16.53 | 4.91±7.45 |
| AV01894 | 35.36±3.10 | 11.69±5.37 | AV01939 | 53.10±3.18 | 27.01±2.43 |
| AV01895 | 45.05±3.74 | 23.56±4.75 | AV01940 | 50.61±0.41 | 25.92±2.96 |
| AV01896 | 40.16±7.76 | 17.86±5.24 | AV01941 | 60.07±2.85 | 35.89±2.87 |
| AV01897 | 45.63±7.21 | 19.64±4.51 | AV01942 | 53.04±2.48 | 25.26±6.30 |
| AV01898 | 49.62±9.10 | 16.53±7.32 | AV01943 | 54.83±2.70 | 29.55±3.91 |
| AV01899 | 57.70±3.74 | 35.66±2.89 | AV01944 | 53.74±3.75 | 23.32±1.22 |
| AV01900 | 53.18±2.20 | 30.51±3.16 | AV01945 | 56.37±2.30 | 25.57±1.53 |
| AV01901 | 44.34±6.20 | 13.45±5.55 | AV01946 | 54.40±1.99 | 27.22±1.92 |
| AV01904 | 35.60±7.26 | 2.08±11.15 | AV01947 | 46.31±3.73 | 10.99±5.81 |
| AV01905 | 40.65±4.84 | 15.01±4.32 | AV01948 | 34.03±5.43 | 13.50±5.30 |
| AV01906 | 44.74±0.90 | 13.56±2.16 | AV01949 | 29.76±2.51 | 7.70±1.98 |
| AV01907 | 34.73±0.26 | 14.73±1.92 | AV01952 | 49.08±1.47 | 27.95±4.70 |
| AV01908 | 49.62±7.01 | 31.33±6.37 | AV01953 | 46.02±3.99 | 21.19±4.05 |
| AV01909 | 47.80±0.70 | 21.96±2.27 | AV01954 | 48.61±1.50 | 25.36±2.97 |
| AV01910 | 51.88±0.67 | 20.22±6.09 | AV01955 | 53.20±2.87 | 30.14±2.11 |
| AV01911 | 35.42±2.80 | 11.40±6.02 | AV01956 | 53.62±4.74 | 35.02±4.48 |
| AV01912 | 49.53±7.27 | 20.62±3.24 | AV01957 | 54.16±3.13 | 13.92±6.54 |
| AV01913 | 47.11±0.39 | 17.63±1.31 | AV01958 | 57.25±3.76 | 28.45±5.01 |
| AV01914 | 45.76±1.63 | 22.16±4.50 | AV01959 | 57.76±2.66 | 35.48±2.07 |
| AV01915 | 48.93±1.28 | 23.82±3.60 | AV00124 | 60.09±1.74 | 33.15±2.78 |
| AV01916 | 32.73±3.88 | -3.38±3.58 | AV00142 | 56.94±1.21 | 27.73±1.55 |
| AV01917 | 23.54±4.29 | -0.21±6.61 | | | |

### Equivalent

Although several embodiments of the present invention have been described and illustrated herein, it is readily understood by those of ordinary skill in the art that a variety of other means and/or structures for performing the functions described herein and/or obtaining results and/or one or more advantages, and each of these variations and/or modifications, are considered to be within the scope of the present invention. More generally, it will be readily understood by those skilled in the art that all parameters, sizes, materials and configurations described herein are exemplary and that the actual parameters, sizes, materials and/or configurations will depend on the particular application in which the teachings of the present invention are used. Those skilled in the art will recognize or be able to determine many equivalents of the particular embodiments of the present invention described herein using only conventional experiments. It should therefore be understood that the foregoing embodiments are presented by way of example only and fall within the scope of the appended claims and their equivalents, and that the present invention may be implemented in a manner different from that specifically described and claimed for protection. The present invention is directed to each of the individual features, systems, articles, materials and/or methods described herein. Further, any combination of two or more such features, systems, articles, materials and/or methods, if such features, systems, articles, materials and/or methods are not contradictory to each other, is also included within the scope of the present invention.

All definitions as defined and used herein shall be understood as referring to dictionary definitions, definitions in files incorporated by reference, and/or the ordinary meanings of defined terms.

Where quantitative limitations are not used in the specification and claims, they are to be understood as "at least one" unless expressly stated to the contrary.

The phrases "and/or" as used in the specification and claims are to be understood as denoting "one or both" of the elements so combined, i.e. such elements appear in combination in some cases and separately in others. In addition to elements specifically identified by "and/or", other elements may optionally exist, whether related or not to those specifically identified elements, unless the contrary is clearly stated.

All references, patents and patent applications and publications cited or referenced in the present application are incorporated herein by reference in their entirety.

## Claims

1. A double-stranded ribonucleic acid (dsRNA) agent that inhibits LPA (Apo(a)) expression, wherein the dsRNA agent comprises a sense strand and an antisense strand, and optionally comprises a targeting ligand; a region complementary to a LPA RNA transcript is comprised at nucleotide positions 2 to 18 in the antisense strand, wherein the complementary region comprises at least 15 continuous nucleotides that differ by 0, 1, 2 or 3 nucleotides from one of the antisense sequences listed in one of Tables 1-3.

2. The dsRNA agent of claim 1, wherein the region complementary to the LPA RNA transcript comprises at least 15, 16, 17, 18, or 19 continuous nucleotides that differ by no more than 3 nucleotides from one of the antisense sequences listed in one of Tables 1-3.

3. The dsRNA agent of claim 1 or 2, wherein the antisense strand of the dsRNA is at least substantially complementary to any target region in the mRNA of the human LPA gene and is provided in any one of Tables 1-3.

4. The dsRNA agent of claim 3, wherein the antisense strand of the dsRNA is completely complementary to any target region in the mRNA of the human LPA gene and is provided in any one of Tables 1-3.

5. The dsRNA agent of claim 1, wherein the dsRNA agent comprises the sense strand sequence of any one of Tables 1-3, wherein the sense strand sequence is at least substantially complementary to the antisense strand sequence in the dsRNA agent.

6. The dsRNA agent of claim 1, wherein the dsRNA agent comprises the sense strand sequence of any one of Tables 1-3, wherein the sense strand sequence is completely complementary to the antisense strand sequence in the dsRNA agent.

7. The dsRNA agent of claim 1, wherein the dsRNA agent comprises the antisense strand sequence listed in any one of Tables 1-3.

8. The dsRNA agent of claim 1, wherein the dsRNA agent comprises a sequence listed as a duplex sequence in any one of Tables 1-3.

9. The dsRNA agent of claim 1, wherein the dsRNA agent comprises at least one modified nucleotide.

10. The dsRNA agent of claim 1, wherein all or substantially all nucleotides in the antisense strand are modified nucleotides.

11. The dsRNA agent of claim 5 or 6, wherein the at least one modified nucleotide comprises: a 2'-O-methyl nucleotide, a 2'-fluoro nucleotide, a 2'-deoxynucleotide, a 2',3'-seco nucleotide mimic, a locked nucleotide, an unlocked nucleic acid (UNA) nucleotide, a glycol nucleic acid nucleotide (GNA), a 2'-F-arabinose nucleotide, a 2'-methoxyethyl nucleotide, an abasic nucleotide, a ribitol, a reverse nucleotide, a reverse abasic nucleotide, a reverse 2'-OMe nucleotide, a reverse 2'-deoxynucleotide, a 2'-amino modified nucleotide, a 2'-alkyl modified nucleotide, a morpholino nucleotide and a 3'-OMe nucleotide, a nucleotide comprising 5'-phosphorothioate group, or a terminal nucleotide linked to a cholesterol derivative or a dodecanoic acid bisdecylamide group, a 2'-amino modified nucleotide, a phosphoramidate, or a non-natural base comprising nucleotide.

12. The dsRNA agent of claim 9 or 10, wherein an E-vinylphosphonate nucleotide is comprised at the 5'-end of the guide strand.

13. The dsRNA agent of claim 1, wherein the dsRNA agent comprises at least one phosphorothioate internucleoside linkage.

14. The dsRNA agent of claim 1, wherein the sense strand comprises at least one phosphorothioate internucleoside linkage.

15. The dsRNA agent of claim 1, wherein the antisense strand comprises at least one phosphorothioate internucleoside linkage.

16. The dsRNA agent of claim 1, wherein the sense strand comprises 1, 2, 3, 4, 5, or 6 phosphorothioate internucleoside linkage.

17. The dsRNA agent of claim 1, wherein the antisense strand comprises 1, 2, 3, 4, 5, or 6 phosphorothioate internucleoside linkage.

18. The dsRNA agent of claim 1, wherein all or substantially all nucleotides of the sense strand and the antisense strand are modified nucleotides.

19. The dsRNA agent of claim 1, wherein the modified sense strand is a modified sense strand sequence listed in one of Tables 2-3.

20. The dsRNA agent of claim 1, wherein the modified antisense strand is a modified antisense strand sequence listed in one of Tables 2-3.

21. The dsRNA agent of claim 1, wherein the sense strand is complementary or substantially complementary to the antisense strand and the complementary region is 16 to 23 nucleotides in length.

22. The dsRNA agent of claim 21, wherein the complementary region is 19 to 21 nucleotides in length.

23. The dsRNA agent of claim 1, wherein each strand is no more than 30 nucleotides in length.

24. The dsRNA agent of claim 1, wherein each strand is no more than 25 nucleotides in length.

25. The dsRNA agent of claim 1, wherein each strand is no more than 23 nucleotides in length.

26. The dsRNA agent of claim 1, wherein the dsRNA agent comprises at least one modified nucleotide and further comprises one or more targeting groups or linking groups.

27. The dsRNA agent of claim 26, wherein the one or more targeting groups or linking groups are conjugated to the sense strand.

28. The dsRNA agent of claim 26 or 27, wherein the targeting groups or linking groups comprise N-acetyl-galactosamine (GalNAc).

29. The dsRNA agent of claim 26 or 27, wherein the targeting groups have structures of: 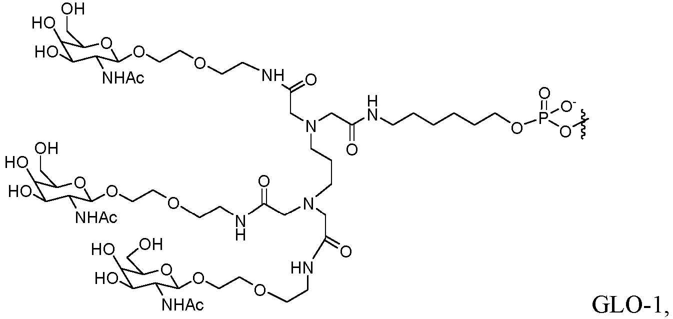 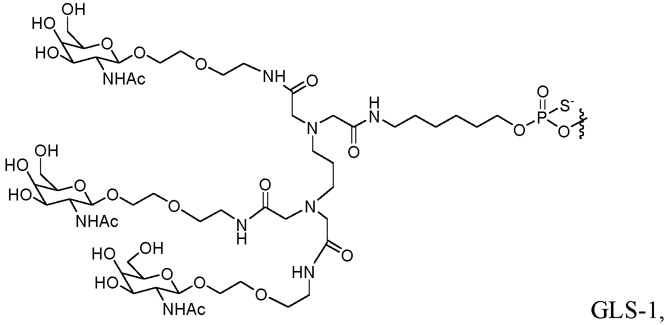 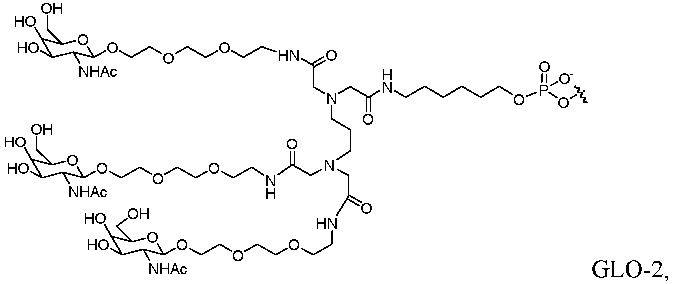 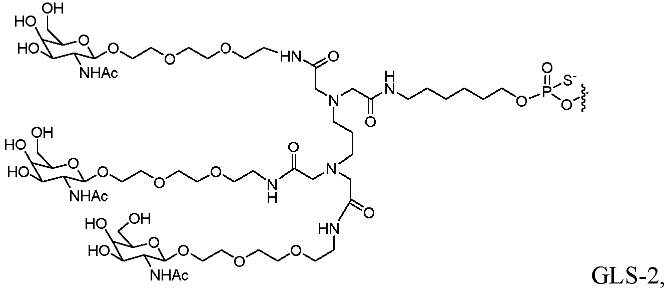 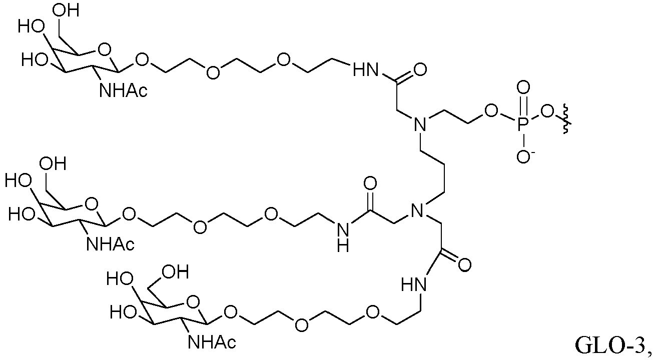 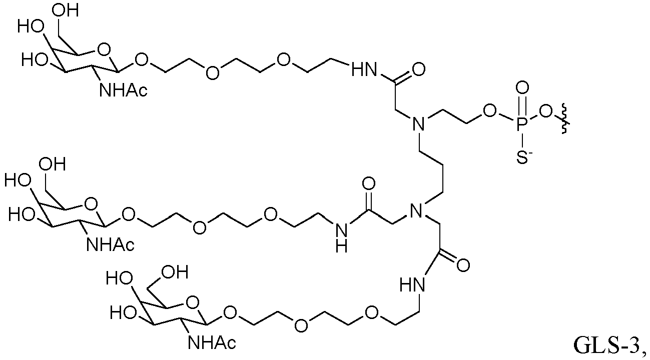 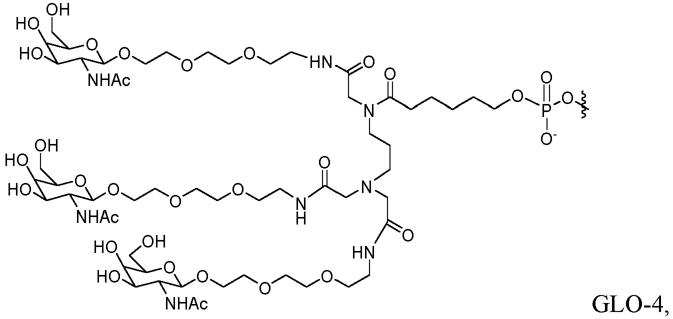 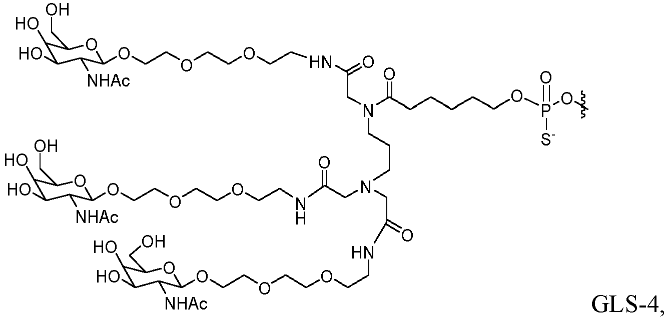 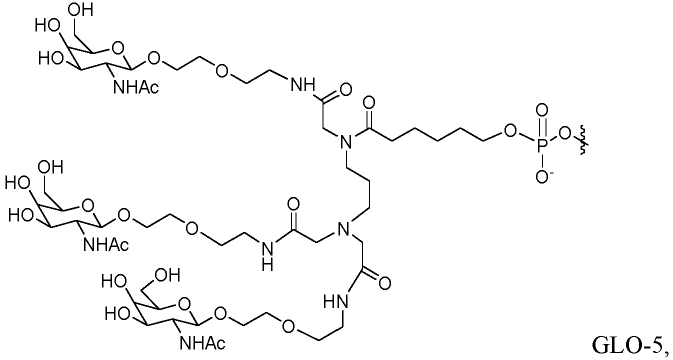 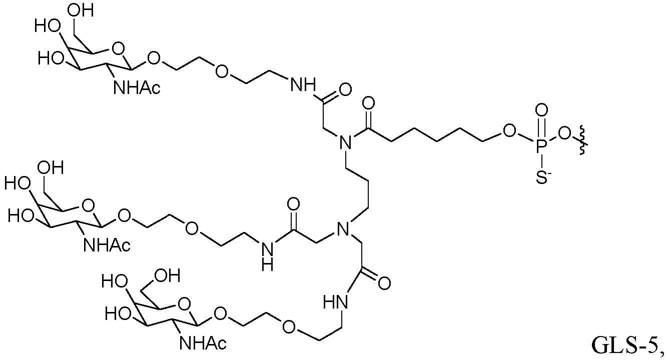 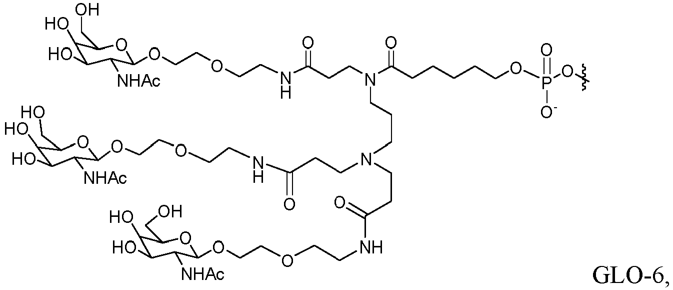 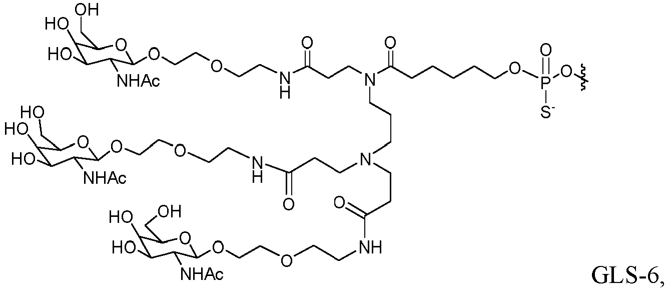 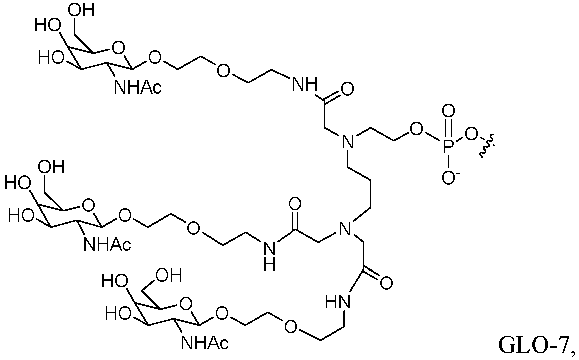 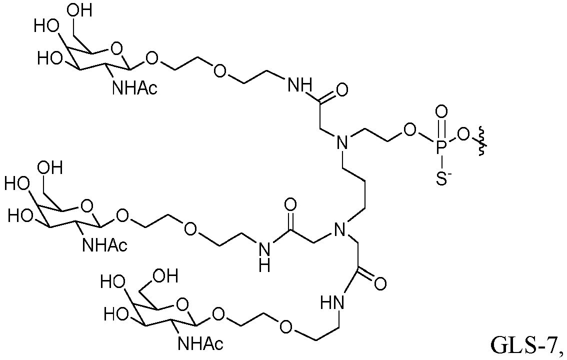 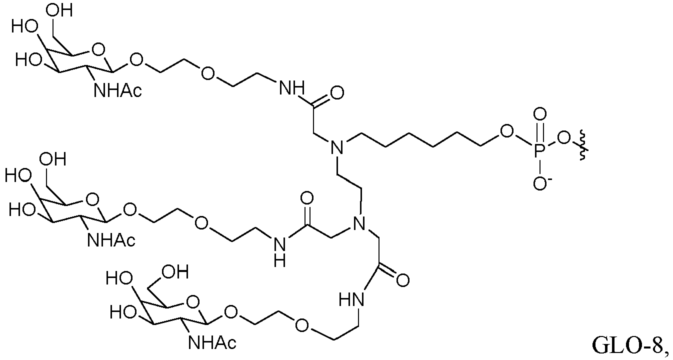 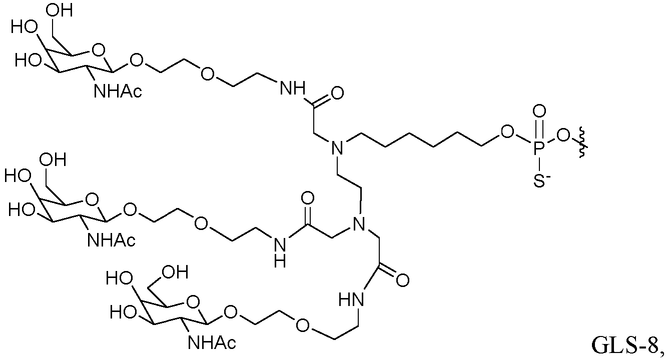 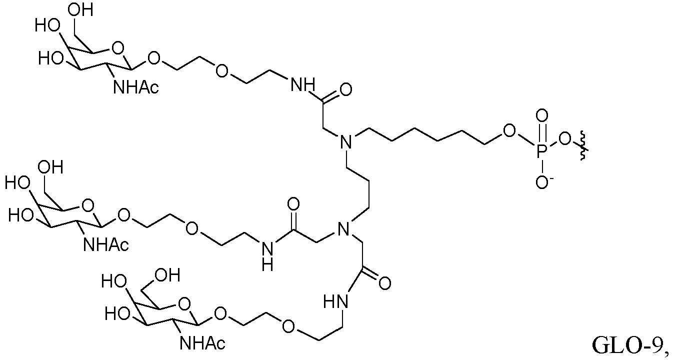 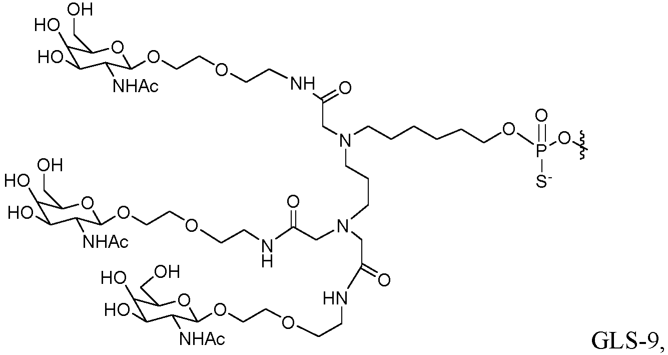 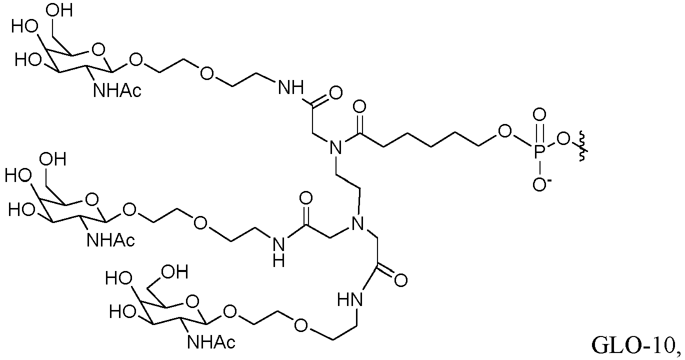 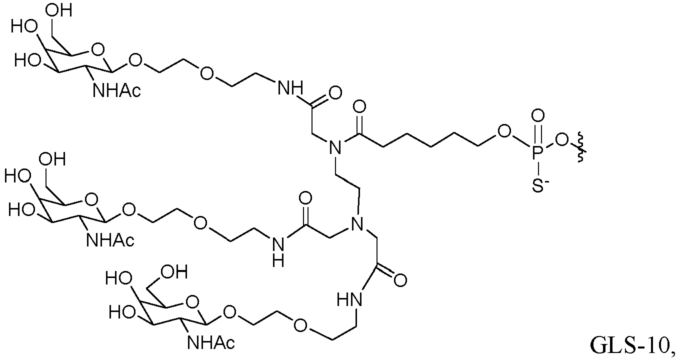 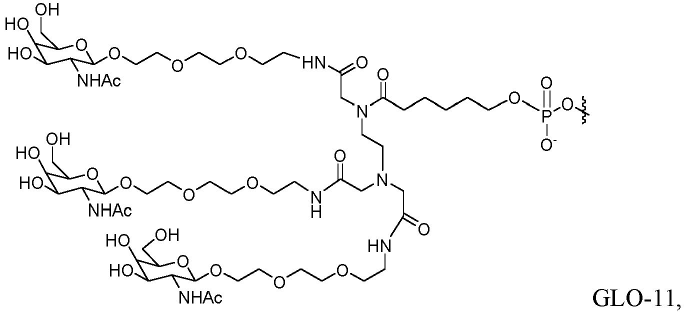 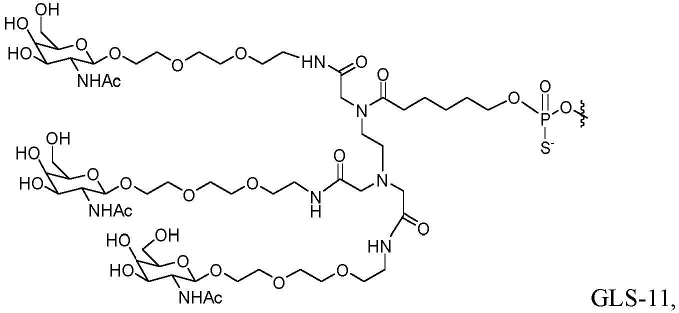 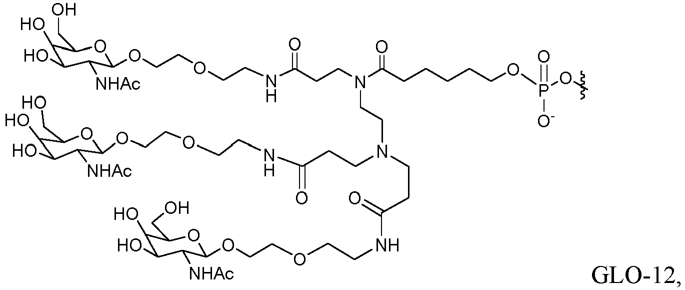 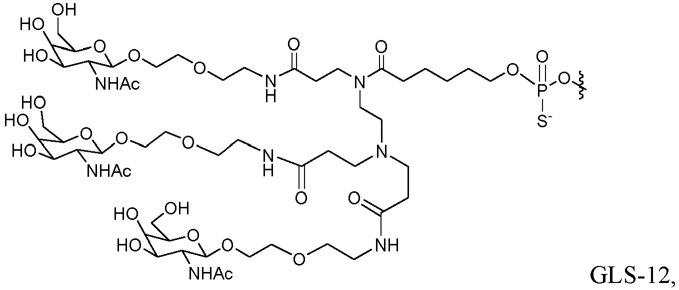 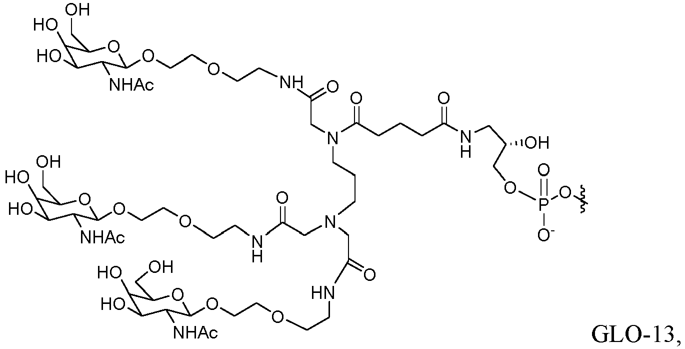 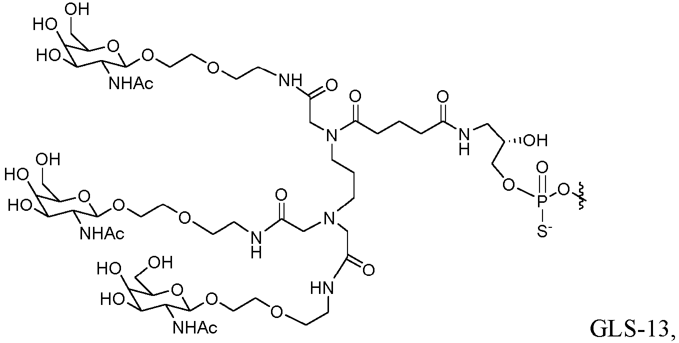 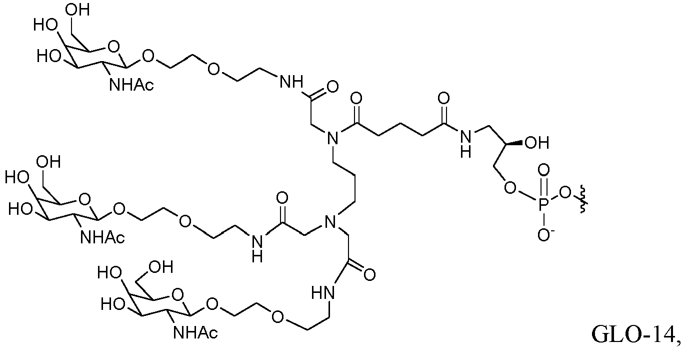 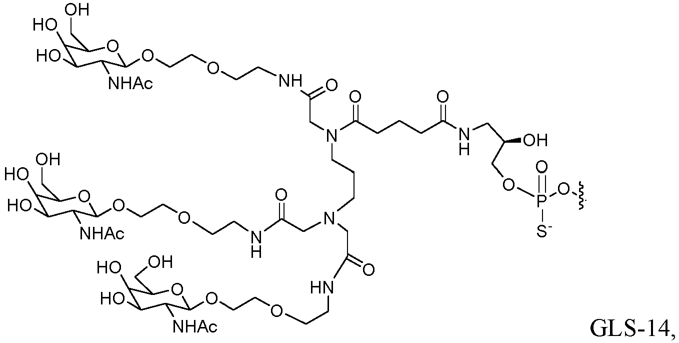 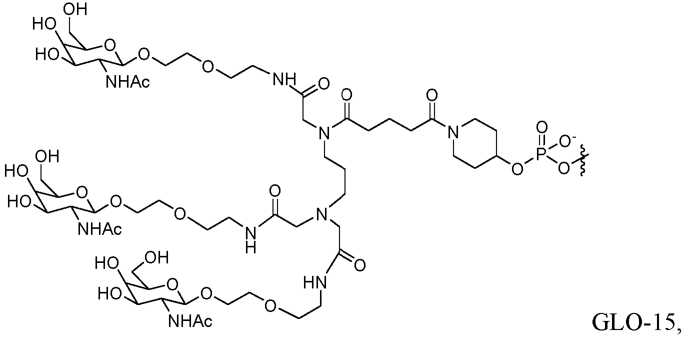 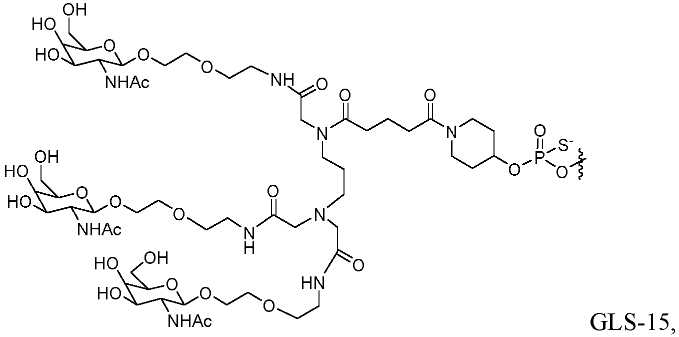 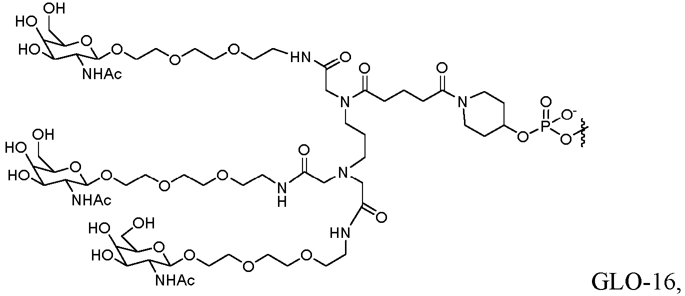 or

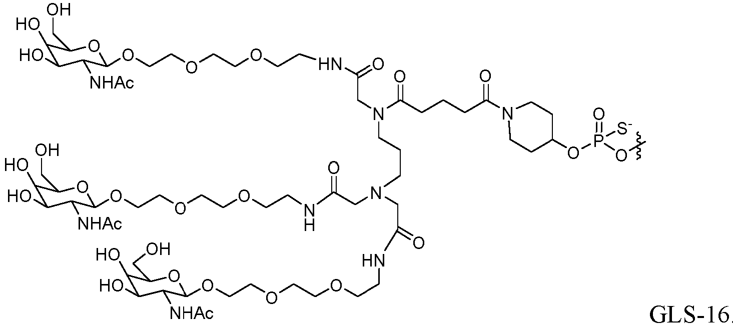

30. The dsRNA agent of claim 1, wherein the dsRNA agent comprises a targeting group conjugated to the 5'-end of the sense strand.

31. The dsRNA agent of claim 1, wherein the dsRNA agent comprises a targeting group conjugated to the 3'-end of the sense strand.

32. The dsRNA agent of claim 1, wherein the antisense strand comprises one reverse abasic residue at the 3'-end.

33. The dsRNA agent of claim 1, wherein the sense strand comprises one or two reverse abasic residues at the 3'-end or/and 5'-end; or the sense strand comprises one or two isomannitol residues at the 3'-end or/and 5'-end.

34. The dsRNA agent of claim 1, wherein the dsRNA agent has two blunt ends.

35. The dsRNA agent of claim 1, wherein at least one strand comprises a 3' overhang of at least 1 nucleotide.

36. The dsRNA agent of claim 1, wherein at least one strand comprises a 3' overhang of at least 2 nucleotides.

37. A composition comprising the dsRNA agent of any one of claims 1 to 36.

38. The composition of claim 37, further comprising a pharmaceutically acceptable carrier.

39. The composition of claim 38, further comprising one or more additional therapeutic agents.

40. The composition of claim 39, wherein the composition is packaged in a kit, a container, a packaging, a dispenser, a pre-filled syringe, or a vial.

41. The composition of claim 37, wherein the composition is formulated for subcutaneous administration or is formulated for intravenous (IV) administration.

42. A cell comprising the dsRNA agent of any one of claims 1 to 36.

43. The cell of claim 42, wherein the cell is a mammalian cell, optionally a human cell.

44. A method for inhibiting LPA gene expression in a cell, comprising:
(i) preparing a cell comprising an effective amount of the double-stranded ribonucleic acid (dsRNA) agent of any one of claims 1 to 36 or the composition of any one of claims 37 to 41.

45. The method of claim 44, further comprising:
(ii) maintaining the cell prepared in (i) of claim 44 for a time sufficient to effect the degradation of an mRNA transcript of an LPA gene, thereby inhibiting the expression of the LPA gene in the cell.

46. The method of claim 44, wherein the cell is in the body of the subject and the dsRNA agent is subcutaneously administered to the subject.

47. The method of claim 44, wherein the cell is in the body of the subject and the dsRNA agent is administered to the subject via IV administration.

48. The method of claim 46 or 47, further comprising evaluating the inhibitory effect on the LPA gene after administration of the dsRNA agent to the subject, wherein a means for the evaluation comprises:
(i) identifying one or more physiological characteristics of an LPA-related disease or condition in the subject, and
(ii) comparing the identified physiological characteristics with baseline physiological characteristics before the treatment of the LPA-related disease or condition and/or control physiological characteristics of the LPA-related disease or condition,
wherein the comparison indicates one or more of the presence or absence of the inhibition of the LPA gene expression in the subject.

49. The method of claim 48, wherein the identified physiological characteristic is an Lp(a) level in the blood.

50. The method of claim 49, wherein a reduction in the Lp(a) level in the blood of the subject indicates a reduction in the LPA gene expression in the subject.

51. A method for inhibiting LPA gene expression in a subject, comprising administering to the subject an effective amount of the double-stranded ribonucleic acid (dsRNA) agent of any one of claims 1 to 36 or the composition of any one of claims 37 to 41.

52. The method of claim 51, wherein the dsRNA agent is subcutaneously administered to the subj ect.

53. The method of claim 51, wherein the dsRNA agent is administered to the subject via IV administration.

54. The method according to any one of claims 51 to 53, further comprising evaluating the inhibitory effect on the LPA gene after administration of the dsRNA agent to the subject, wherein a means for the evaluation comprises:
(i) identifying one or more physiological characteristics of an LPA-related disease or condition in the subject, and
(ii) comparing the identified physiological characteristics with baseline physiological characteristics before the treatment of the LPA-related disease or condition and/or control physiological characteristics of the LPA-related disease or condition,
wherein the comparison indicates one or more of the presence or absence of the inhibition of the LPA gene expression in the subject.

55. The method of claim 54, wherein the identified physiological characteristic is an Lp(a) level in the blood.

56. The method of claim 55, wherein a reduction in the Lp(a) level in the blood of the subject indicates a reduction in the LPA gene expression in the subject.

57. A method for treating and preventing a disease or condition related to an LPA protein, comprising administering to a subject an effective amount of the double-stranded ribonucleic acid (dsRNA) agent of any one of claims 1 to 36 or the composition of any one of claims 37 to 41 to inhibit the LPA gene expression.

58. The method of claim 57, wherein the disease or condition is a cardiovascular disease, wherein the cardiovascular disease includes: Berger's disease, peripheral arterial disease, coronary artery disease, metabolic syndrome, acute coronary syndrome, aortic stenosis, aortic regurgitation, aortic dissection, retinal artery occlusion, cerebrovascular diseases, mesenteric ischemia, superior mesenteric artery occlusion, renal artery stenosis, stable/unstable angina, acute coronary syndrome, heterozygous or homozygous familial hypercholesterolemia, hyperapolipoprotein beta lipoproteinemia, cerebrovascular atherosclerosis, cerebrovascular diseases and venous thrombosis, stroke, atherosclerosis, thrombosis, coronary heart diseases or aortic stenosis and/or any other diseases or pathologies related to elevated levels of Lp(a)-containing particles.

59. The method of claim 57, further comprising administering an additional therapeutic regimen to the subject.

60. The method of claim 59, wherein the additional therapeutic regimen comprises administration of one or more LPA antisense polynucleotides of the present invention to the subject, administration of a non-LPA dsRNA therapeutic agent to the subject, and behavior modifications in the subject.

61. The method of claim 60, wherein the non-LPA dsRNA therapeutic agent is one or more additional therapeutic agents such as a HMg Co-A reductase inhibitor (statins), ezetimibe, a PCSK-9 inhibitor, a CTEP inhibitor, an ANGPTL3-targeting therapy, an AGT-targeting therapy, an APOC3-targeting therapy and niacin, or any combination thereof.

62. The method of claim 57, wherein the dsRNA agent is subcutaneously administered to the subj ect.

63. The method of claim 57, wherein the dsRNA agent is administered to the subject via IV administration.

64. The method of any one of claims 57 to 63, further comprising determining the efficacy of the administered double-stranded ribonucleic acid (dsRNA) agent in the subject.

65. The method of claim 64, wherein a means of determining the efficacy of the treatment in the subject comprises:
(i) identifying one or more physiological characteristics of an LPA-related disease or condition in the subject, and
(ii) comparing the identified physiological characteristics with baseline physiological characteristics before the treatment of the LPA-related disease or condition,
wherein the comparison indicates one or more of the presence or absence of the efficacy of, and efficacy level of administering the double-stranded ribonucleic acid (dsRNA) agent to the subject.

66. The method of claim 65, wherein the identified physiological characteristic is an Lp(a) level in the blood.

67. The method of claim 65, wherein a reduction in the Lp(a) level in the blood of the subject indicates the presence of the efficacy of administering the double-stranded ribonucleic acid (dsRNA) agent to the subject.

68. A method for reducing an LPA protein level in a subject compared to a baseline level of the LPA protein in the subject before treatment, comprising administering to the subject an effective amount of the double-stranded ribonucleic acid (dsRNA) agent of any one of claims 1 to 36 or the composition of any one of claims 37 to 41 to reduce the LPA gene expression level.

69. The method of claim 68, wherein the dsRNA agent is administered subcutaneously to the subject or administered via IV to the subject.

70. A method for changing a physiological characteristic of an LPA-related disease or condition in a subject compared to a baseline physiological characteristic of the LPA-related disease or condition in the subject, comprising administering to the subject an effective amount of the double-stranded ribonucleic acid (dsRNA) agent of any one of claims 1 to 36 or the composition of any one of claims 37 to 41 to change the physiological characteristic of the LPA-related disease or condition in the subject.

71. The method of claim 70, wherein the dsRNA agent is administered subcutaneously to the subject or administered via IV to the subject.

72. The method of claim 70, wherein the physiological characteristic is an Lp(a) level in the blood.
